# EUROPÄISCHE PATENTSCHRIFT

(11) **EP 1 204 638 B1**
(45) Veröffentlichungstag und Bekanntmachung des Hinweises auf die Patenterteilung: **23.11.2005**
(21) Anmeldenummer: 00949371.9
(22) Anmeldetag: 18.07.2000
(51) Int. Cl.: C07D 207/40, C07D 209/54, C07D 307/94, C07D 307/60, C07C 49/753, C07D 487/04, C07D 491/10, A01N 43/34, A01N 43/08, A01N 43/10, A01N 31/00

(54) **BIPHENYLSUBSTITUIERTE CYCLISCHE KETOENOLE ALS SCHÄDLINGSBEKÄMPFUNGSMITTEL**
BIPHENYL-SUBSTITUTED CYCLIC KETO ENOLS AS PESTICIDES
CETO-ENOLS CYCLIQUES A SUBSTITUTION BIPHENYLE COMME PRODUITS ANTIPARASITES

(30) Priorität: 30.07.1999 DE 19935963
(43) Veröffentlichungstag der Anmeldung: 15.05.2002
(73) Patentinhaber: Bayer CropScience AG, 40789 Monheim (DE)
(72) Erfinder: FISCHER, Reiner, D-40789 Monheim (DE); GRAFF, Alan, D-51061 Köln (DE); BRETSCHNEIDER, Thomas, D-53797 Lohmar (DE); ERDELEN, Christoph, D-42799 Leichlingen (DE); DREWES, Mark, Wilhelm, D-40764 Langenfeld (DE); FEUCHT, Dieter, D-40789 Monheim (DE)
(86) Internationale Anmeldenummer: PCT/EP2000/006852
(87) Internationale Veröffentlichungsnummer: WO 2001/009092

(56) Entgegenhaltungen:
- WO-A-98/05638
- WO-A-99/16748
- WO-A-99/43649
- WO-A-99/48869
- WO-A-99/55673

## Beschreibung

Die vorliegende Erfindung betrifft neue biphenylsubstituierte cyclische Ketoenole, mehrere Verfahren zu ihrer Herstellung und ihre Verwendung in der Landwirtschaft, Tiergesundheit und im Haushalts- und Hygienebereich als Schädlingsbekämpfungsmittel und Herbizide.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599 und EP-415211) sowie substituierte monocyclische 3-Aryl-pyrrolidin-2,4-dion-Derivate (EP-A-377 893 und EP-442 077).

Weiterhin bekannt sind polycyclische 3-Arylpyrrolidin-2,4-dion-Derivate (EP-442 073) sowie 1H-Arylpyrrolidin-dion-Derivate (EP-456 063, EP-521 334, EP-. 596 298, EP-613 884, EP-613 885, WO 94/01 997, WO 95/26 954, WO 95/20 572, EP-A-0 668 267, WO 96/25 395, WO 96/35 664, WO 97/01 535, WO 97/02 243 und WO 97/36 868, WO 98/05 638, WO 98/06721, WO 98/25928, WO 99/16748, WO 99/24437).

Es ist bekannt, daß bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-dihydrofuranon-Derivate mit herbiziden, akariziden und insektiziden Eigenschaften aus EP-A-528 156, EP-A-0 647 637, WO 95/26 345, WO 96/20 196, WO 96/25 395, WO 96/35 664, WO 97/01 535, WO 97/02 243 und WO 97/36 868, WO 98/05 638, WO 99/16748, WO 98/25928 bekannt. Auch 3-Aryl-Δ³-dihydrothiphen-on-Derivate sind bekannt (WO 95/26 345, 96/25 395, WO 97/01 535, WO 97/02 243, WO 97/36 868, WO 98/05 638, WO 98/25928).

Aus der Literatur sind ferner bestimmte 3H-Pyrazol-3-on-Derivate, wie beispielsweise 1,2-Diethyl-1,2-dihydro-5-hydroxy-4-phenyl-3H-pyrazol-3-on oder {[5-Oxo-1,2-diphenyl-4-(p-sulfophenyl)-3-pyrazolin-3-yl]-oxy}-dinatriumsalz oder p-(3-Hydroxy-5-oxo-1,2-diphenyl-3-pyrazolin-4-yl)-benzolsulfonsäure bekannt (vgl. J. Heterocycl. Chem., 25(5), 1301-1305, 1988 oder J. Heterocycl. Chem., 25(5), 1307-1310, 1988 oder Zh. Obshch. Khim., 34(7), 2397-2402, 1964). Eine biologische Wirkung dieser Verbindungen wird aber nicht beschrieben.

Weiterhin ist bekannt, daß das Trinatriumsalz der 4,4',4"-(5-Hydroxy-3-oxo-1H-pyrazol-1,2,4(3H)-triyl)-tris-benzolsulfonsäure pharmakologische Eigenschaften besitzt (vgl. Farmakol. Toksikol. (Moscow), 38(2), 180-186, 1976). Seine Verwendung im Pflanzenschutz ist aber nicht bekannt.

Außerdem sind in EP-508 126 und in WO 92/16 510, WO 96/21 652 4-Arylpyrazolidin-3,5-dion-Derivate mit herbiziden, akariziden und insektiziden Eigenschaften beschrieben. Zudem wurden 4-Arylpyrazolidine bekannt, von denen fungizide Eigenschaften beschrieben wurden (WO 96/36 229, WO 96/36 615, WO 96/36 616, WO 96/36 633).

Bestimmte, im Phenylring unsubstituierte Phenyl-pyron-Derivate sind bereits bekannt geworden (vgl. A.M. Chirazi, T. Kappe und E. Ziegler, Arch. Pharm. 309, 558 (1976) und K.-H. Boltze und K. Heidenbluth, Chem. Ber. 91, 2849), wobei für diese Verbindungen eine mögliche Verwendbarkeit als Schädlingsbekämpfungsmittel nicht angegeben wird. Im Phenylring substituierte Phenyl-pyron-Derivate mit herbiziden, akariziden und insektiziden Eigenschaften sind in EP-A-588 137, WO 96/25 395, WO 96/35 664, WO 97/01 535, WO 97/02 243, WO 97/16 436, WO 97/19 941 und WO 97/36 868, WO 98/05 638 beschrieben.

Bestimmte, im Phenylring unsubstituierte 5-Phenyl-1,3-thiazin-Derivate sind bereits bekannt geworden (vgl. E. Ziegler und E. Steiner, Monatsh. 95, 147 (1964), R. Ketcham, T. Kappe und E. Ziegler, J. Heterocycl. Chem. 10, 223 (1973)), wobei für diese Verbindungen eine mögliche Anwendung als Schädlingsbekämpfungsmittel nicht angegeben wird. Im Phenylring substituierte 5-Phenyl-1,3-thiazin-Derivate mit herbizider, akarizider und insektizider Wirkung sind in WO 94/14 785, WO 96/02 539, WO 96/35 664, WO 97/01 535, WO 97/02 243, WO 97/02 243, WO 97/36 868 beschrieben.

Es ist bekannt, daß bestimmte substituierte 2-Arylcyclopentandione herbizide und akarizide Eigenschaften besitzen (vgl. z.B. US-4 283 348; 4 338 122; 4 436 666; 4 526 723; 4 551 547; 4 632 698; WO 96/01798; WO 96/03 366 sowie WO 97/14667, WO 98/39281). Außerdem sind ähnlich substituierte Verbindungen bekannt; 3-Hydroxy-5,5-dimethyl-2-phenylcyclopent-2-en-1-on aus der Publikation Micklefield et al., Tetrahedron, (1992), 7519-26 sowie der Naturstoff Involutin (-)-cis-5-(3,4-dihydroxyphenyl)-3,4-dihydroxy-2-(4-hydroxyphenyl)-cyclopent-2-en-one aus der Publikation Edwards et al., J. Chem. Soc. S, (1967), 405-9. Eine insektizide oder akarizide Wirkung wird nicht beschrieben. Außerdem ist 2-(2,4,6-Trimethylphenyl)-1,3-indandion aus der Publikation J. Economic Entomology, 66, (1973), 584 und der Offenlegungsschrift DE-2 361 084 bekannt, mit Angabe von herbiziden und akariziden Wirkungen.

Es ist bekannt, daß bestimmte substituierte 2-Arylcyclohexandione herbizide und akarizide Eigenschaften besitzen (US-4 175 135, 4 209 432, 4 256 657, 4 256 658, 4 256 659, 4 257 858, 4 283 348, 4 303 669, 4 351 666, 4 409 153, 4 436 666, 4 526 723, 4 613 617, 4 659 372, DE-2 813 341, sowie Wheeler, T.N., J. Org. Chem. 44, 4906 (1979)).

Die Wirksamkeit und Wirkungsbreite dieser Verbindungen ist jedoch insbesondere bei niedrigen Aufwandmengen und Konzentrationen nicht immer voll zufriedenstellend. Weiterhin ist die Pflanzenverträglichkeit dieser Verbindungen nicht immer ausreichend.

Es wurden nun neue Verbindungen der Formel (I) 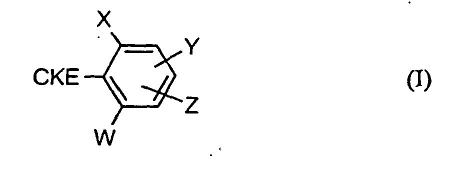
- W: steht für Wasserstoff, Halogen, C₁-C₆-Alkyl, C₁-C₆-Alkoxy, C₁-C₆-Halogenalkyl, C₁-C₆-Halogenalkoxy, Nitro oder Cyano,
- X: steht für Halogen, C₁-C₆-Alkyl, C₁-C₆-Halogenalkyl, C₁-C₆-Alkoxy, C₃-C₆-Alkenyloxy, C₁-C₆-Alkylthlo, C₁-C₆-Alkylsulfinyl, C₁-C₆-Alkylsulfonyl, C₁-C₆-Halogenalkoxy, C₃-C₆-Halogenalkenyloxy, Nitro, Cyano oder jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenylthio, Benzyloxy oder Benzylthio.
- Y: steht für einen der Reste

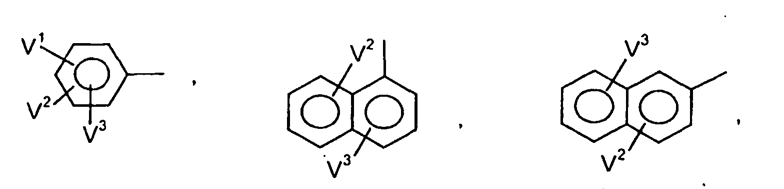

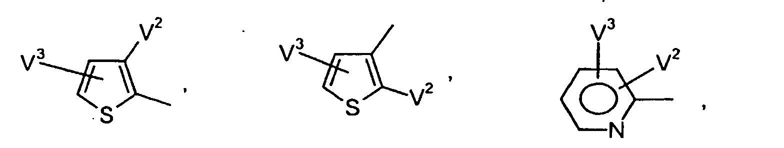

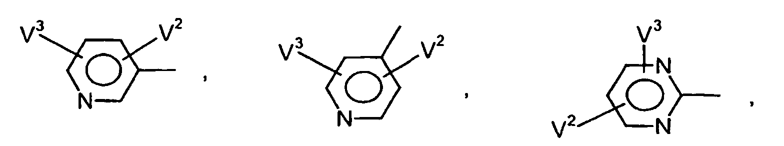

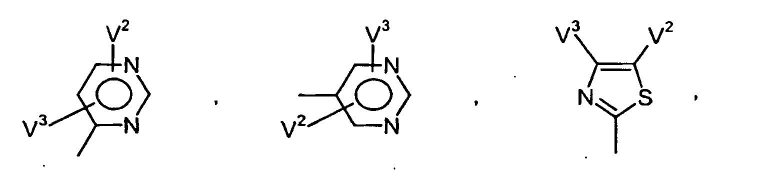

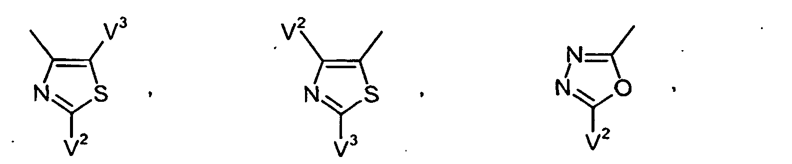

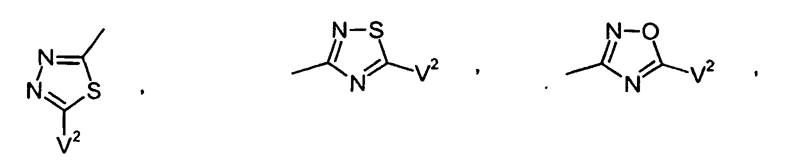

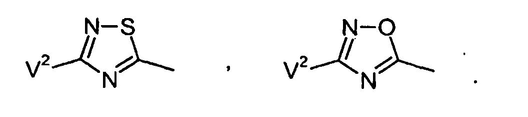

- V¹: steht für Wasserstoff, Halogen, C₁-C₁₂-Alkyl, C₁-C₆-Alkoxy, C₁-C₄-Halogenalkyl, C₁-C₄-Halogenalkoxy, Nitro, Cyano oder jeweils gegebenenfalls einfach oder mehrfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenoxy-C₁-C₄-alkyl, Phenyl-C₁-C₄-alkoxy, Phenylthio-C₁-C₄-alkyl oder Phenyl-C₁-C₄-alkylthio.
- V² und V³: stehen unabhängig voneinander für Wasserstoff, Halogen, C₁-C₆-Alkyl, C₁-C₆-Alkoxy, C₁-C₄-Halogenalkyl oder C₁-C₄-Halogenalkoxy.
- Z: steht für Wasserstoff, Halogen, C₁-C₆-Alkyl, C₁-C₆-Halogenalkyl, C₁-C₆-Alkoxy oder C₁-C₆-Halogenalkoxy.
- CKE: steht für eine der Gruppen 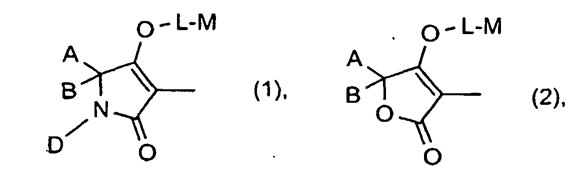 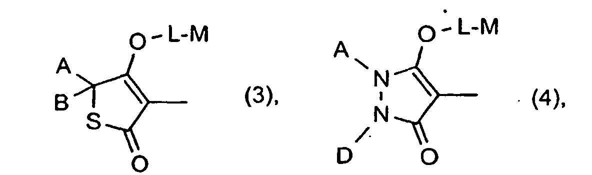 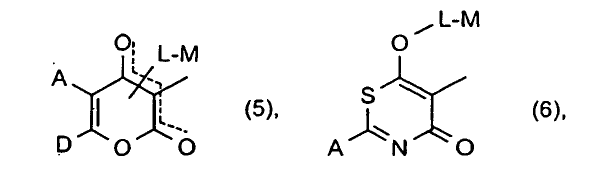 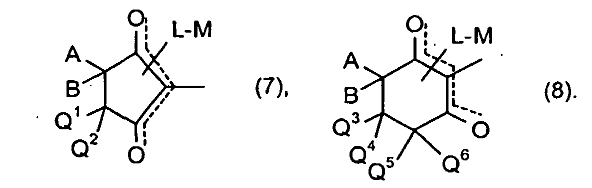
- A: steht für Wasserstoff oder jeweils gegebenenfalls durch Halogen substituiertes C₁-C₁₂-Alkyl, C₃-C₈-Alkenyl, C₁-C₁₀-Alkoxy-C₁-C₈-alkyl, Di-, tri- oder tetra-C₁-C₈-alkoxy-C₁-C₈-alkyl, C₁-C₁₀-Alkylthio-C₁-C₆-alkyl, gegebenenfalls durch Halogen, C₁-C₆-Alkyl oder C₁-C₆-Alkoxy substituiertes C₃-C₈-Cycloalkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes C₆- oder C₁₀-Aryl (Phenyl oder Naphthyl), Hetaryl mit 5 bis 6 Ringatomen (beispielsweise Furanyl, Pyridyl, Imidazolyl, Triazolyl, Pyrazolyl, Pyrimidyl, Thiazolyl oder Thienyl) oder C₆- oder C₁₀-Aryl-C₁-C₆-alkyl (Phenyl-C₁-C₆-alkyl oder Naphthyl-C₁-C₆-alkyl).
- B: steht für Wasserstoff, C₁-C₁₂-Alkyl oder C₁-C₈-Alkoxy-C₁-C₆-alkyl oder
- A, B: und das Kohlenstoffatom an das sie gebunden sind, stehen für gesättigtes C₃-C₁₀-Cycloalkyl oder ungesättigtes C₅-C₁₀-Cycloalkyl, worin gegebenenfalls ein Ringglied durch Sauerstoff oder Schwefel ersetzt ist und welche gegebenenfalls einfach oder zweifach durch C₁-C₈-Alkyl, C₃-C₁₀-Cycloalkyl, C₁-C₈-Halogenalkyl, C₁-C₈-Alkoxy, C₁-C₈-Alkylthio, Halogen oder Phenyl substituiert sind oder
- A, B: und das Kohlenstoffatom, an das sie gebunden sind, stehen für C₃-C₆-Cycloalkyl, welches durch eine gegebenenfalls ein oder zwei nicht direkt benachbarte Sauerstoff- und/oder Schwefelatome enthaltende Alkylendiyl-, oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Ring bildet oder
- A, B: und das Kohlenstoffatom, an das sie gebunden sind, stehen für C₃-C₈-Cycloalkyl oder C₅-C₈-Cycloalkenyl, in welchen zwei Substituenten gemeinsam mit den Kohlenstoffatomen, an die sie gebunden sind, für jeweils gegebenenfalls durch C₁-C₆-Alkyl, C₁-C₆-Alkoxy oder Halogen substituiertes C₂-C₆-Alkandiyl, C₂-C₆-Alkendiyl oder C₄-C₆-Alkandiendiyl stehen, worin gegebenenfalls eine Methylengruppe durch Sauerstoff oder Schwefel ersetzt ist.
- D: steht für Wasserstoff, jeweils gegebenenfalls durch Halogen substituiertes C₁-C₁₂-Alkyl, C₃-C₈-Alkenyl, C₃-C₈-Alkinyl, C₁-C₁₀-Alkoxy-C₂-C₈-alkyl, Poly-C₁-C₈-alkoxy-C₂-C₈-alkyl, C₁-C₁₀-Alkylthio-C₂-C₈-alkyl, gegebenenfalls durch Halogen, C₁-C₄-Alkyl, C₁-C₄-Alkoxy oder C₁-C₄-Halogenalkyl substituiertes C₃-C₈-Cycloalkyl, in welchem gegebenenfalls ein Ringglied durch Sauerstoff oder Schwefel ersetzt ist oder jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Hetaryl mit 5 oder 6 Ringatomen (beispielsweise Furanyl, Imidazolyl, Pyridyl, Thiazolyl, Pyrazolyl, Pyrimidyl, Pyrrolyl, Thienyl oder Triazolyl), Phenyl-C₁-C₆-alkyl oder Hetaryl-C₁-C₆-alkyl mit 5 oder 6 Ringatomen (beispielsweise Furanyl-, Imidazolyl-, Pyridyl-, Thiazolyl-, Pyrazolyl-, Pyrimidyl-, Pyrrolyl-, Thienyl- oder Triazolyl-C₁-C₆-alkyl) oder
- A und D: stehen gemeinsam für jeweils gegebenenfalls substituiertes C₃-C₆-Alkandiyl oder C₃-C₆-Alkendiyl, worin gegebenenfalls eine Methylengruppe durch Sauerstoff oder Schwefel ersetzt ist und
wobei als Substituenten jeweils in Frage kommen:
Halogen, Hydroxy, Mercapto oder jeweils gegebenenfalls durch Halogen substituiertes C₁-C₁₀-Alkyl, C₁-C₆-Alkoxy, C₁-C₆-Alkylthio, C₃-C₇-Cycloalkyl, Phenyl oder Benzyloxy, oder eine weitere C₃-C₆-Alkandiylgruppierung, C₃-C₆-Alkendiylgruppierung oder eine Butadienylgruppierung, die gegebenenfalls durch C₁-C₆-Alkyl substituiert ist oder in der gegebenenfalls zwei benachbarte Substituenten mit den Kohlenstoffatomen, an die sie gebunden sind, einen weiteren gesättigten oder ungesättigten Cyclus mit 5 oder 6 Ring-atomen bilden (im Fall der Verbindung der Formel (I-1) stehen A und D dann gemeinsam mit den Atomen, an die sie gebunden sind beispielsweise für die weiter unten genannten Gruppen AD-1 bis AD-10), der Sauerstoff oder
Schwefel enthalten kann, oder worin gegebenenfalls eine der folgenden Gruppen 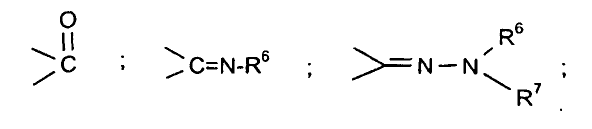 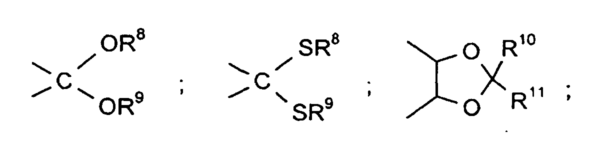 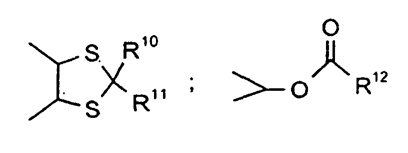 oder 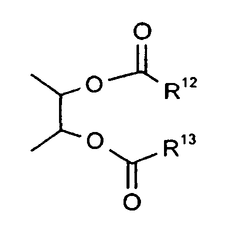 enthalten ist, oder
- A und Q¹: stehen gemeinsam für jeweils gegebenenfalls einfach oder zweifach, gleich oder verschieden durch Halogen, Hydroxy, durch jeweils gegebenenfalls einfach bis dreifach, gleich' oder verschieden durch Halogen substituiertes C₁-C₁₀-Alkyl, C₁-C₆-Alkoxy, C₁-C₆-Alkylthio, C₃-C₇-Cycloalkyl oder durch jeweils gegebenenfalls einfach bis dreifach, gleich oder verschieden durch Halogen, C₁-C₆-Alkyl oder C₁-C₆-Alkoxy substituiertes Benzyloxy oder Phenyl substituiertes C₃-C₆-Alkandiyl oder C₄-C₆-Alkendiyl, welches außerdem gegebenenfalls eine der nachstehenden Gruppen 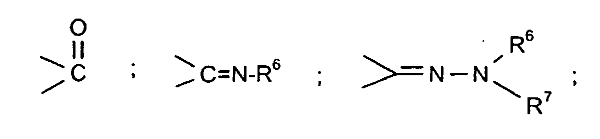 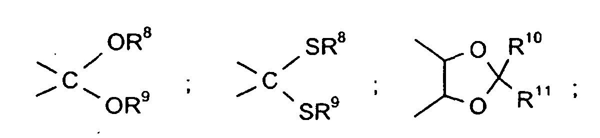 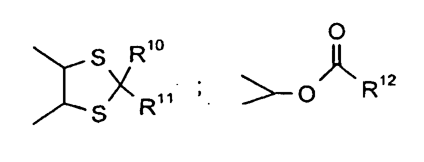 oder 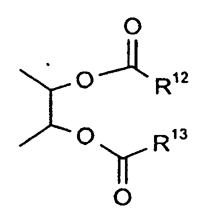 enthält oder durch eine C₁-C₂-Alkandiylgruppe oder durch ein Sauerstoffatom überbrückt ist oder
- Q¹: steht für Wasserstoff oder C₁-C₄-Alkyl.
- Q², Q⁴, Q⁵ und Q⁶: stehen unabhängig voneinander für Wasserstoff oder C₁-C₄-Alkyl.
- Q³: steht für Wasserstoff, C₁-C₆-Alkyl, C₁-C₆-Alkoxy-C₁-C₂-alkyl, C₁-C₆-Alkylthio-C₁-C₂-alkyl, gegebenenfalls durch C₁-C₄-Alkyl oder C₁-C₄-Alkoxy substituiertes C₃-C₈-Cycloalkyl, worin gegebenenfalls eine Methylengruppe durch Sauerstoff oder Schwefel ersetzt ist oder gegebenenfalls durch Halogen, C₁-C₄-Alkyl, C₁-C₄-Alkoxy, C₁-C₂-Halogenalkyl, C₁-C₂-Halogenalkoxy, Cyano oder Nitro substituiertes Phenyl oder
- Q³ und Q⁴: stehen gemeinsam mit dem Kohlenstoffatom, an das sie gebunden sind, für gegebenenfalls durch C₁-C₄-Alkyl, C₁-C₄-Alkoxy oder C₁-C₂-Halogenalkyl substituiertes C₃-C₇-Cycloalkyl, in welchem gegebenenfalls ein Ringglied durch Sauerstoff oder Schwefel ersetzt ist.
- L: steht für eine Alkandiylgruppe mit 1 bis 6 Kohlenstoffatomen.
- M: steht für eine der folgenden Gruppierungen: CN; 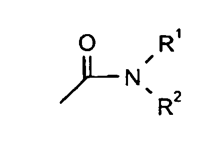 -CO₂R². -OR², -SR², -CO_{R}³, 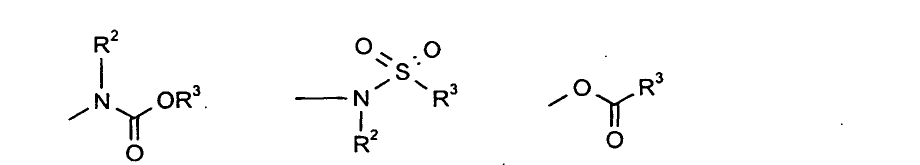 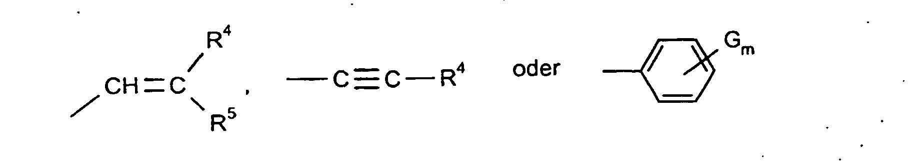
- R¹: steht für Wasserstoff oder C₁-C₁₂-Alkyl.
- R²: steht für jeweils gegebenenfalls durch Halogen substituiertes C₁-C₁₂-Alkyl, C₃-C₈-Alkenyl, C₃-C₈-Alkinyl, C₃-C₈-Cycloalkyl, C₁-C₈-Alkoxy-C₂-C₈-alkyl, C₁-C₈-Alkylthio-C₂-C₈-alkyl, für jeweils gegebenenfalls durch Halogen, Nitro, Cyano, C₁-C₆-Alkoxy, C₁-C₆-Halogenalkoxy, C₁-C₆-Alkylthio, C₁-C₆-Halogenakylthio, C₁-C₆-Alkyl oder C₁-C₆-Halogenalkyl substituiertes Phenyl oder Benzyl.
- R³: steht für gegebenenfalls durch Halogen substituiertes C₁-C₁₂-Alkyl oder für jeweils durch Halogen, C₁-C₆-Alkyl, C₁-C₆-Halogenalkyl, C₁-C₆-Alkoxy oder C₁-C₆-Halogenalkoxy substituiertes Phenyl oder Benzyl.
- R⁴: steht für Wasserstoff, Halogen, gegebenenfalls durch Halogen substituiertes C₁-C₆-Alkyl oder jeweils durch Halogen, C₁-C₆-Alkyl, C₁-C₆-Alkoxyl, C₁-C₄-Halogenalkyl, C₁-C₄-Halogenalkoxy, Cyano oder Nitro substituiertes Phenyl.
- R⁵: steht für Wasserstoff, Halogen oder gegebenenfalls durch Halogen substituiertes C₁-C₆-Alkyl.
- G: steht für Halogen, C₁-C₆-Alkyl, C₁-C₆-Alkoxy, C₁-C₄-Halgenoalkyl, C₁-C₄-Halogenalkoxy, Cyano oder Nitro.
- m: steht für eine Zahl 0, 1, 2 oder 3.
- R⁶: steht für Wasserstoff, für jeweils gegebenenfalls durch Halogen substituiertes C₁-C₈-Alkyl oder C₁-C₈-Alkoxy, für gegebenenfalls durch Halogen, C₁-C₄-Alkyl oder C₁-C₄-Alkoxy substituiertes C₃-C₈-Cycloalkyl, in welchem gegebenenfalls eine Methylengruppe durch Sauerstoff oder Schwefel ersetzt ist, oder für jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenyl-C₁-C₄-alkyl oder Phenyl-C₁-C₄-alkoxy.
- R⁷: steht für Wasserstoff oder C₁-C₈-Alkyl oder
- R⁶ und R⁷: stehen gemeinsam für C₄-C₆-Alkandiyl.
- R⁸ und R⁹: sind gleich oder verschieden und stehen bevorzugt für C₁-C₆-Alkyl oder
- R⁸ und R⁹: stehen gemeinsam für einen C₂-C₄-Alkandiylrest, der gegebenenfalls durch C₁-C₆-Alkyl, C₁-C₆-Halogenalkyl oder durch gegebenenfalls durch Halogen, C₁-C₆-Alkyl, C₁-C₄-Halogenalkyl, C₁-C₆-Alkoxy, C₁-C₄-Halogenalkoxy, Nitro oder Cyano substituiertes Phenyl substituiert ist.
- R¹⁰ und R¹¹: stehen unabhängig voneinander bevorzugt für Wasserstoff, für gegebenenfalls durch Halogen substituiertes C₁-C₈-Alkyl oder für gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl oder
- R¹⁰ und R¹¹: stehen gemeinsam mit dem Kohlenstoffatom, an das sie gebunden sind, für eine Carbonylgruppe oder für gegebenenfalls durch Halogen, C₁-C₄-Alkyl öder C₁-C₄-Alkoxy substituiertes C₅-C₇-Cycloalkyl, in dem gegebenenfalls eine Methylengruppe durch Sauerstoff oder Schwefel ersetzt ist.
- R¹² und R¹³: stehen unabhängig voneinander für C₁-C₁₀-Alkyl, C₂-C₁₀-Alkenyl, C₁-C₁₀-Alkoxy, C₁-C₁₀-Alkylamino, C₃-C₁₀-Alkenylamino, Di-(C₁-C₁₀-alkyl)amino oder Di-(C₃-C₁₀-alkenyl)amino.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sein können.

Unter Einbeziehung der Bedeutungen (1) bis (8) der Gruppe CKE ergeben sich folgende hauptsächliche Strukturen (I-1) bis (I-8): 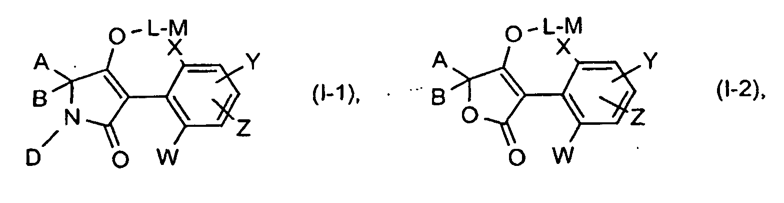 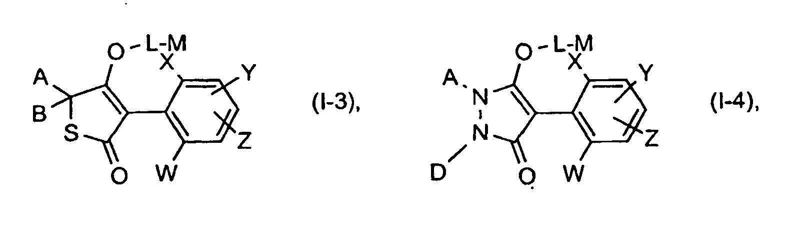 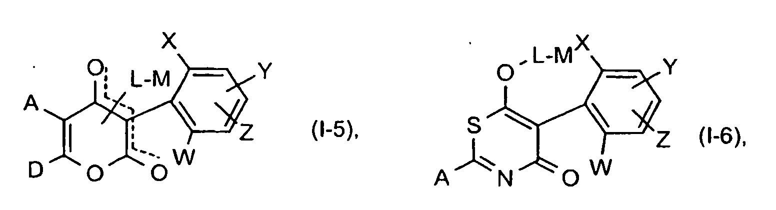 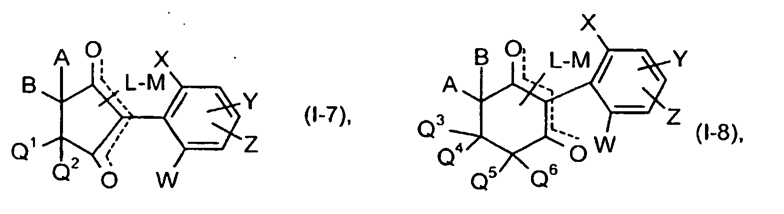 worin

A, B, D, L, M, Q¹, Q², Q³, Q⁴, Q⁵, Q⁶, W, X, Y und Z die oben angegebenen Bedeutungen haben.

Weiterhin wurde gefunden, daß die neuen Verbindungen der Formel (I) eine sehr gute Wirksamkeit als Schädlingsbekämpfungsmittel, vorzugsweise als Arthropodizide, Fungizide und Herbizide aufweisen.

Außerdem wurde gefunden, daß man die neuen Verbindungen der Formel (I-1) bis (I-8) 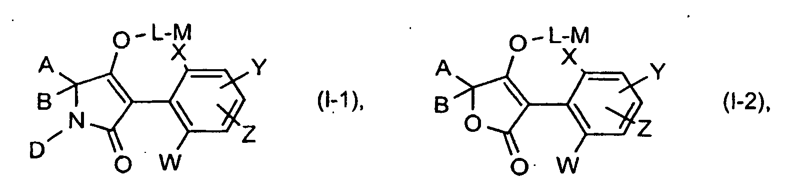 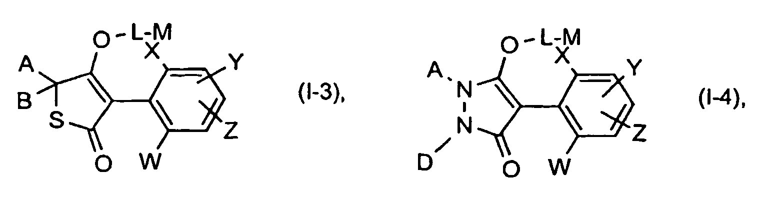 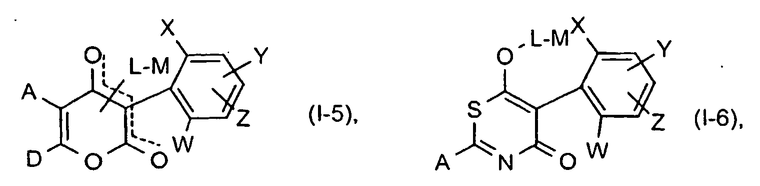 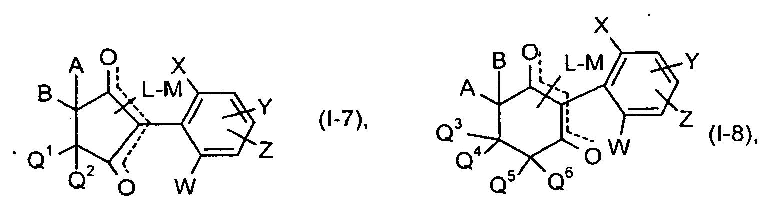 worin
A, B, D, L, M, Q¹, Q², Q³, Q⁴, Q⁵, Q⁶, W, X, Y und Z die oben angegebenen Bedeutungen haben,
erhält, wenn man
(A) Verbindungen der Formel (II-1) bis (II-8) 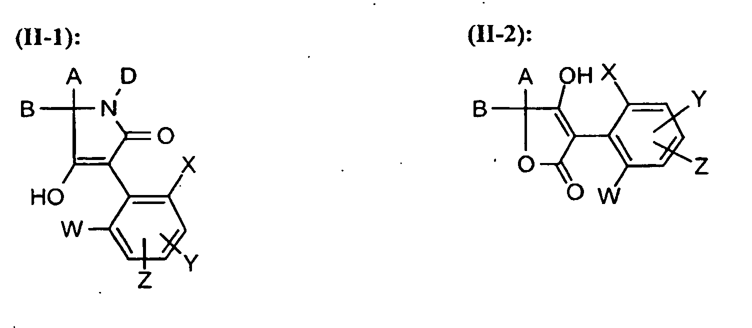 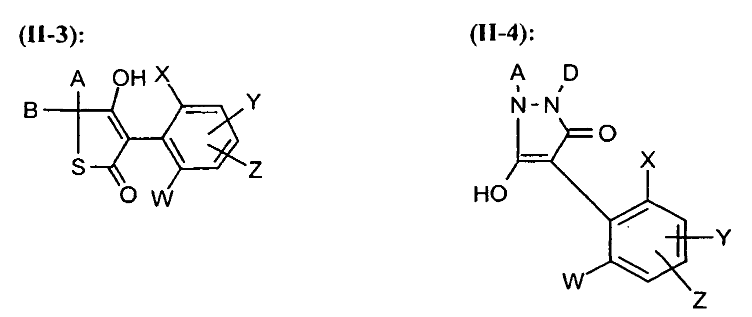 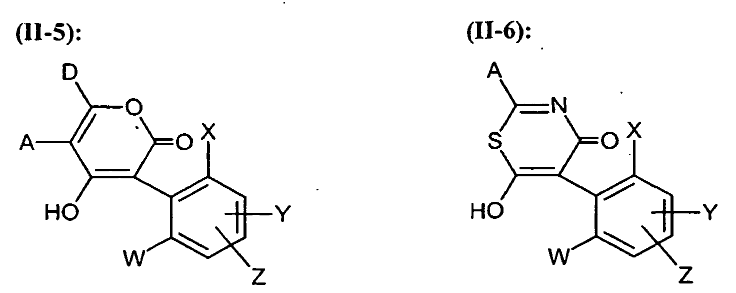 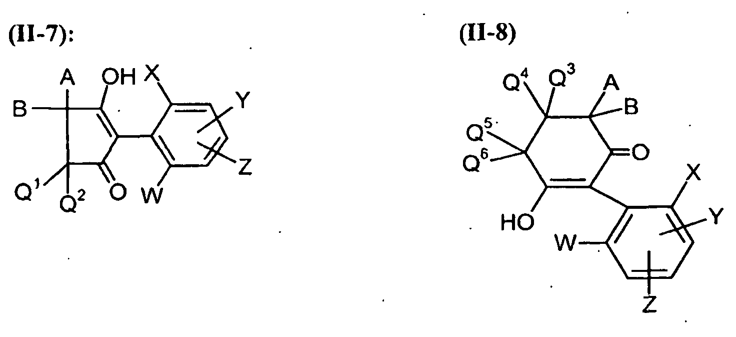
worin
A, B, D, Q¹, Q², Q³, Q⁴, Q⁵, Q⁶, W, X, Y und Z die oben angegebene Bedeutung
haben,
mit Verbindungen der Formel (III)

J-L-M (III)

in welcher
- L und M: die oben angegebene Bedeutung haben und
- J: für eine Abgangsgruppe, wie Halogen, -O-SO₂-Haloalkyl (beispielsweise Triflat, -O-SO₂-Alkyl (beispielsweise Mesylat) oder -O-SO₂-Aryl (beispielsweise Tosylat) steht,
in Gegenwart eines Verdünnungsmittels und in Gegenwart einer Base umsetzt.

Weiterhin wurde gefunden, daß die neuen Verbindungen der Formel (I) eine sehr gute Wirksamkeit als Schädlingsbekämpfungsmittel, vorzugsweise als Insektizide, Akarizide und auch als Herbizide aufweisen.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:

In den als genannten Restedefinitionen steht Halogen, auch als Substituent, wie z.B. in Halogenalkyl, für Fluor, Chlor, Brom und Iod, insbesondere für Fluor und Chlor.
- W: steht besonders bevorzugt für Wasserstoff, Fluor, Chlor, Brom, C₁-C₄-Alkyl, C₁-C₄-Alkoxy, C₁-C₄-Halogenalkyl, C₁-C₄-Halogenalkoxy, Nitro oder Cyano.
- X: steht besonders bevorzugt für Fluor, Chlor, Brom, C₁-C₄-Alkyl, C₁-C₄-Alkoxy, C₃-C₄-Alkenyloxy, C₁-C₄-Halogenalkyl, C₁-C₄-Halogenalkoxy, C₃-C₄-Halogenalkenyloxy, Nitro oder Cyano.
- Y: steht besonders bevorzugt für einen der Reste 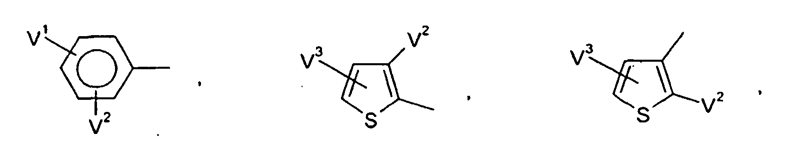 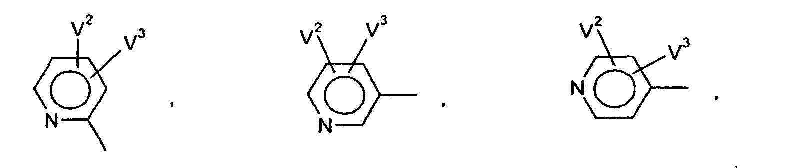 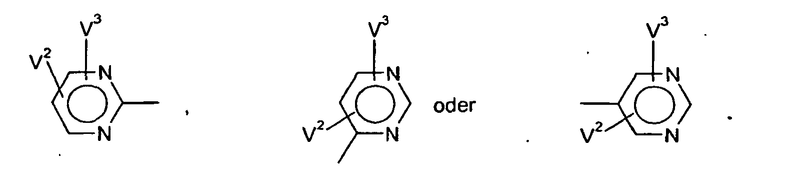
- V¹: steht besonders bevorzugt für Wasserstoff, Fluor, Chlor, Brom, C₁-C₆-Alkyl, C₁-C₄-Alkoxy, C₁-C₂-Halogenalkyl, C₁-C₂-Halogenalkoxy, Nitro, Cyano oder jeweils gegebenenfalls einfach oder zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl, Phenoxy, Phenoxy-C₁-C₂-alkyl, Phenyl-C₁-C₂-alkoxy, Phenylthio-C₁-C₂-alkyl oder Phenyl-C₁-C₂-alkylthio.
- V² und V³: stehen besonders bevorzugt unabhängig voneinander für Wasserstoff, Fluor, Chlor, Brom, C₁-C₄-Alkyl, C₁-C₄-Alkoxy, C₁-C₂-Halogenalkyl oder C₁-C₂-Halogenalkoxy.
- Z: steht besonders bevorzugt für Wasserstoff, Fluor, Chlor, Brom, C₁-C₄-Alkyl, C₁-C₄-Halogenalkyl, C₁-C₄-Alkoxy oder C₁-C₄-Halogenalkoxy.
- CKE: steht besonders bevorzugt für eine der Gruppen 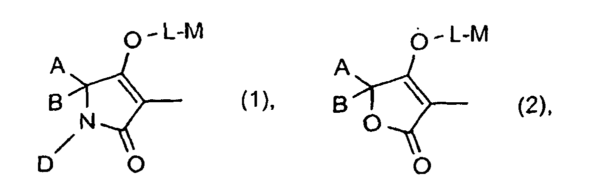 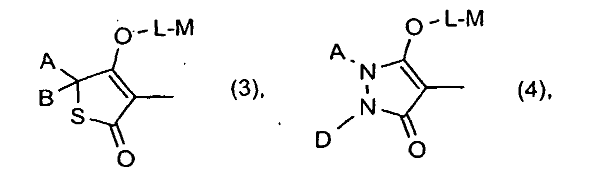 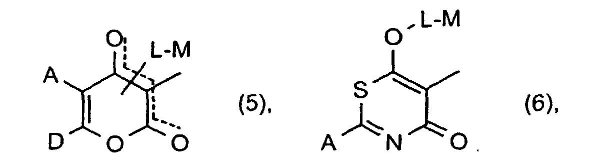 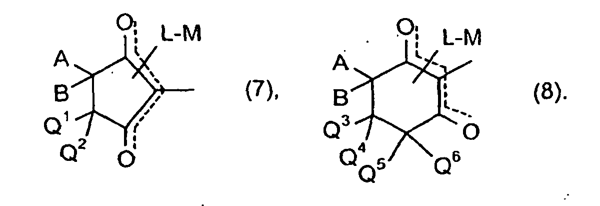
- A: steht besonders bevorzugt für Wasserstoff, jeweils gegebenenfalls durch Fluor oder Chlor substituiertes C₁-C₁₀-Alkyl, C₁-C₈-Alkoxy-C₁-C₆-alkyl, gegebenenfalls durch Fluor, Chlor, C₁-C₄-Alkyl oder C₁-C₄-Alkoxy substituiertes C₃-C₇-Cycloalkyl, in welchem gegebenenfalls ein Ringglied durch Sauerstoff oder Schwefel ersetzt ist oder (jedoch nicht im Fall der Verbindungen der Formeln (1-5), (1-7) und (1-8)) jeweils gegebenenfalls durch Fluor, Chlor, Brom, C₁-C₄-Alkyl, C₁-C₄-Halogenalkyl, C₁-C₄-Alkoxy, Cyano, Nitro oder C₁-C₄-Halogenalkoxy substituiertes Phenyl, Furanyl, Pyridyl, Imidazolyl, Triazolyl, Pyrazolyl, Pyrimidyl, Thiazolyl, Thienyl oder Phenyl-C₁-C₄-alkyl.
- B: steht besonders bevorzugt für Wasserstoff oder C₁-C₆-Alkyl oder
- A, B: und das Kohlenstoffatom an das sie gebunden sind, stehen besonders bevorzugt für gesättigtes oder ungesättigtes C₅-C₇-Cycloalkyl, worin gegebenenfalls ein Ringglied durch Sauerstoff oder Schwefel ersetzt ist und welches gegebenenfalls einfach durch C₁-C₆-Alkyl, C₅-C₈-Cycloalkyl, C₁-C₃-Halogenalkyl, C₁-C₆-Alkoxy, Fluor, Chlor oder Phenyl substituiert ist oder
- A, B: und das Kohlenstoffatom, an das sie gebunden sind, stehen besonders bevorzugt für C₅-C₆-Cycloalkyl, welches durch eine gegebenenfalls ein oder zwei nicht direkt benachbarte Sauerstoff- oder Schwefelatome enthaltende Alkylendiyl- oder durch eine Alkylendioxyl- oder durch eine Alkylendithiol-Gruppe substituiert ist, die mit dem Kohlenstoffatom, an das sie gebunden ist, einen weiteren fünf- oder sechsgliedrigen Ring bildet oder
- A, B: und das Kohlenstoffatom, an das sie gebunden sind, stehen besonders bevorzugt für C₃-C₆-Cycloalkyl oder C₅-C₆-Cycloalkenyl, in welchen zwei Substituenten gemeinsam mit den Kohlenstoffatomen, an die sie gebunden sind, für jeweils gegebenenfalls durch C₁-C₅-Alkyl, C₁-C₅-Alkoxy, Fluor, Chlor oder Brom substituiertes C₂-C₄-Alkandiyl, C₂-C₄-Alkendiyl, worin gegebenenfalls eine Methylengruppe durch Sauerstoff oder Schwefel ersetzt ist oder Butadiendiyl stehen.
- D: steht besonders bevorzugt für Wasserstoff, für jeweils gegebenenfalls durch Fluor oder Chlor substituiertes C₁-C₁₀-Alkyl, C₃-C₆-Alkenyl, C₁-C₈-Alkoxy-C₂-C₆-alkyl oder C₁-C₈-Alkylthio-C₂-C₆-alkyl, für gegebenenfalls durch Fluor, Chlor, C₁-C₄-Alkyl, C₁-C₄-Alkoxy oder C₁-C₂-Halogenalkyl substituiertes C₃-C₇-Cycloalkyl, in welchem gegebenenfalls eine Methylengruppe durch Sauerstoff oder Schwefel ersetzt ist oder (jedoch nicht im Fall der Verbindungen der Formeln (1-1) und (I-4)) für jeweils gegebenenfalls durch Fluor, Chlor, Brom, C₁-C₄-Alkyl, C₁-C₄-Halogenalkyl, C₁-C₄-Alkoxy oder C₁-C₄-Halogenalkoxy substituiertes Phenyl, Furanyl, Imidazolyl, Pyridyl, Thiazolyl, Pyrazolyl, Pyrimidyl, Pyrrolyl, Thienyl, Triazolyl oder Phenyl-C₁-C₄-alkyl oder
- A und D: stehen gemeinsam besonders bevorzugt für gegebenenfalls substituiertes C₃-C₅-Alkandiyl, in welchem eine Methylengruppe durch eine Carbonylgruppe, Sauerstoff oder Schwefel ersetzt sein kann, wobei als Substituenten Hydroxy, C₁-C₆-Alkyl oder C₁-C₄-Alkoxy in Frage kommen oder
- A und D: stehen (im Fall der Verbindungen der Formel (I-1)) gemeinsam mit den Atomen, an die sie gebunden sind, für eine der Gruppen AD-1 bis AD-10: 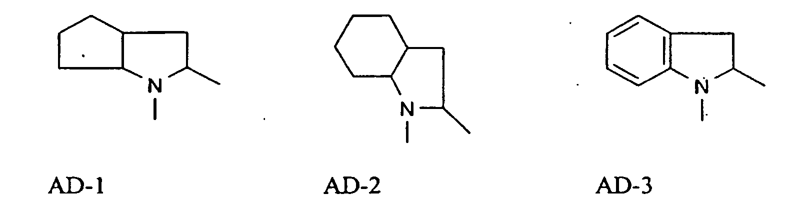 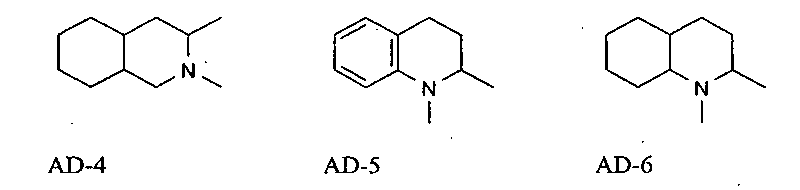 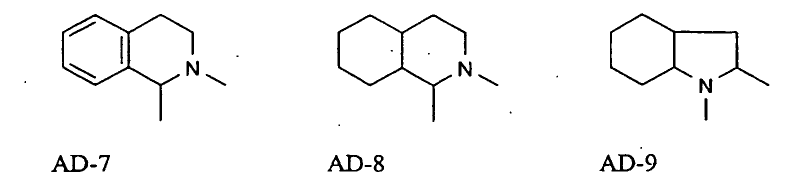 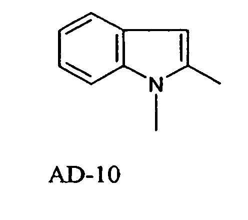 oder
- A und Q¹: stehen gemeinsam besonders bevorzugt für jeweils gegebenenfalls einfach oder zweifach, gleich oder verschieden durch Fluor, Chlor, Hydroxy, durch jeweils gegebenenfalls einfach bis dreifach durch Fluor substituiertes C₁-C₈-Alkyl oder C₁-C₄-Alkoxy substituiertes C₃-C₄-Alkandiyl oder C₃-C₄-Alkendiyl oder
- Q¹: steht besonders bevorzugt für Wasserstoff.
- Q²: steht besonders bevorzugt für Wasserstoff.
- Q⁴, Q⁵ und Q⁶: stehen besonders bevorzugt unabhängig voneinander für Wasserstoff oder C₁-C₃-Alkyl.
- Q³: steht besonders bevorzugt für Wasserstoff, C₁-C₄-Alkyl, C₁-C₄-Alkoxy-C₁-C₂-alkyl, C₁-C₄-Alkylthio-C₁-C₂-alkyl oder gegebenenfalls durch Methyl oder Methoxy substituiertes C₃-C₆-Cycloalkyl, worin gegebenenfalls eine Methylengruppe durch Sauerstoff oder Schwefel ersetzt ist oder
- Q³ und: Q⁴ stehen besonders bevorzugt gemeinsam mit dem Kohlenstoff, an das sie gebunden sind, für gegebenenfalls durch C₁-C₄-Alkyl oder C₁-C₄-Alkoxy substituiertes gesättigtes C₅-C₆-Cycloalkyl, in welchem gegebenenfalls ein Ringglied durch Sauerstoff oder Schwefel ersetzt ist.
- L: steht besonders bevorzugt für eine Alkandiylgruppe mit 1 bis 4 Kohlenstoffatomen.
- M: steht besonders bevorzugt für eine der folgenden Gruppierungen: CN; 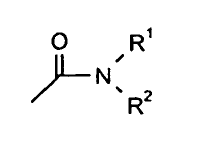 -CO₂R², -OR², -SR², -COR³, 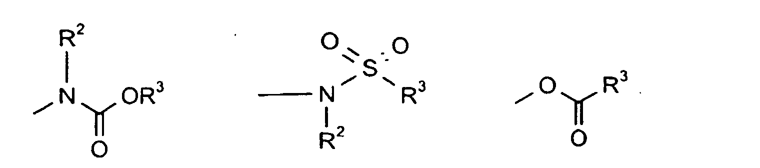 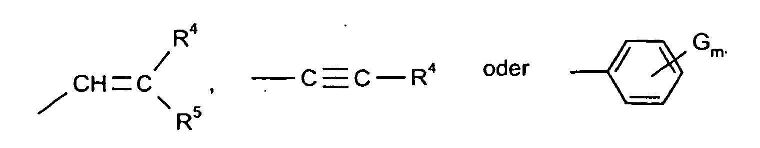
- R¹: steht besonders bevorzugt für Wasserstoff oder C₁-C₁₀-Alkyl.
- R²: steht besonders bevorzugt für jeweils gegebenenfalls durch Fluor, Chlor, Brom substituiertes C₁-C₁₀-Alkyl, C₃-C₆-Alkenyl, C₃-C₆-Alkinyl, C₃-C₈-Cycloalkyl, C₁-C₆-Alkoxy-C₂-C₆-alkyl, C₁-C₆-Alkylthio-C₂-C₆-alkyl, für jeweils gegebenenfalls durch Fluor, Chlor, Brom, Nitro, Cyano, C₁-C₄-Alkylthio, C₁-C₄-Alkoxy, C₁-C₄-Halogenalkoxy, C₁-C₄-Halogenalkylthio, C₁-C₄-Alkyl oder C₁-C₄-Halogenalkyl substituiertes Phenyl oder Benzyl.
- R³: steht besonders bevorzugt für gegebenenfalls durch Fluor, Chlor, Brom substituiertes C₁-C₁₀-Alkyl oder für jeweils durch Fluor, Chlor, C₁-C₄-Alkyl, C₁-C₄-Halogenalkyl, C₁-C₄-Alkoxy oder C₁-C₄-Halogenalkoxy substituiertes Phenyl oder Benzyl.
- R⁴: steht besonders bevorzugt für Wasserstoff, Fluor, Chlor, Brom, gegebenenfalls durch Fluor oder Chlor substituiertes C₁-C₅-Alkyl oder durch Fluor, Chlor, Brom, C₁-C₄-Alkyl, C₁-C₄-Alkoxy, C₁-C₂-Haloalkyl, C₁-C₂-Haloalkoxy, Cyano oder Nitro substituiertes Phenyl.
- R⁵: steht besonders bevorzugt für Wasserstoff, Fluor, Chlor, Brom oder gegebenenfalls durch Fluor oder Chlor substituiertes C₁-C₄-Alkyl.
- G: steht besonders bevorzugt für Fluor, Chlor, Brom, C₁-C₄-Alkyl, C₁-C₄-Alkoxy, C₁-C₂-Halogenalkyl, C₁-C₂-Halogenalkoxy, Cyano oder Nitro.
- m: steht besonders bevorzugt für eine Zahl 0, 1 oder 2.

In den als besonders bevorzugt genannten Restedefinitionen steht Halogen,auch als Substituent wie z.B. in Halogenalkyl, für Fluor, Chlor, Brom und Iod, insbesondere für Fluor, Chlor und Brom, ganz besonders für Fluor oder Chlor.
- W: steht ganz besonders bevorzugt für Wasserstoff, Chlor, Fluor, Methyl, Ethyl, Methoxy, Ethoxy (hervorgehoben für Wasserstoff, Methyl, Ethyl oder Chlor).
- X: steht ganz besonders bevorzugt für Fluor, Chlor, Methyl, Ethyl, n-Propyl, iso-Propyl, Methoxy, Ethoxy, n-Propoxy, iso-Propoxy, Trifluormethyl, Difluormethoxy, Trifluormethoxy, Nitro oder Cyano (hervorgehoben für Fluor, Chlor, Methyl, Ethyl, n-Propyl oder iso-Propyl).
- Y: steht ganz besonders bevorzugt für einen der Reste 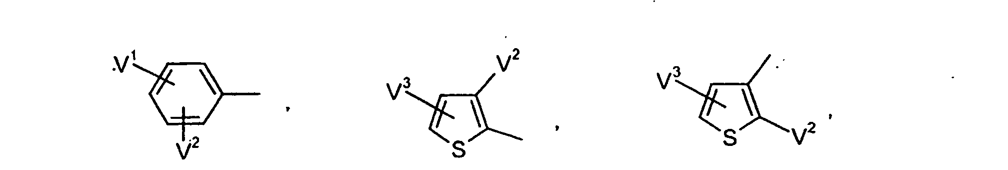 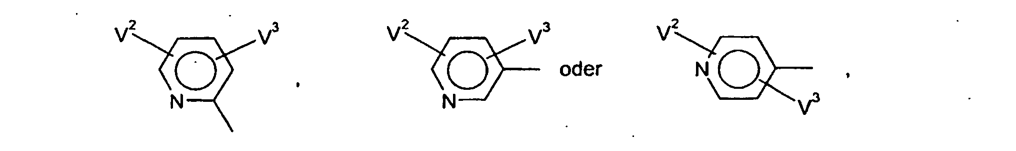 insbesondere für 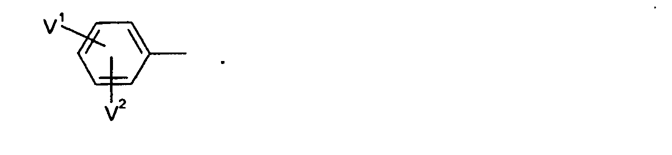
- V¹: steht ganz besonders bevorzugt für Wasserstoff, Fluor, Chlor, Brom, Methyl, Ethyl, n-Propyl, iso-Propyl, n-Butyl, iso-Butyl, tert.-Butyl, Methoxy, Ethoxy, n-Propoxy, iso-Propoxy, Trifluormethyl, Trifluormethoxy, Nitro, Cyano oder Phenyl.
- V² und V³: stehen ganz besonders bevorzugt unabhängig voneinander für Wasserstoff, Fluor, Chlor, Methyl, Ethyl, n-Propyl, iso-Propyl, Methoxy, Ethoxy, Trifluormethyl oder Trifluormethoxy.
- Z: steht ganz besonders bevorzugt für Wasserstoff, Fluor, Chlor, Methyl oder Methoxy (hervorgehoben für Wasserstoff oder Methyl).
- CKE: steht ganz besonders bevorzugt für eine der Gruppen 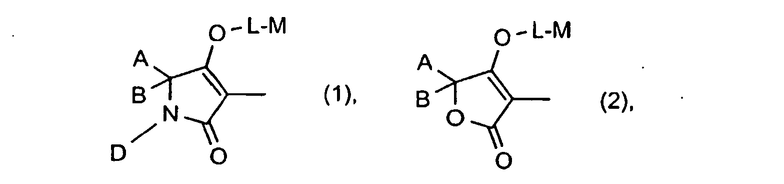 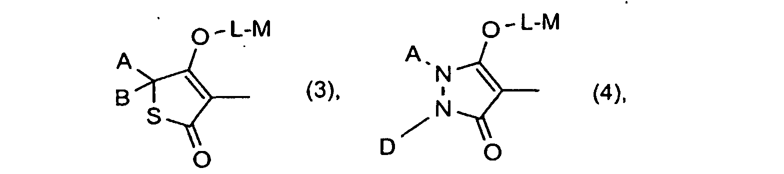 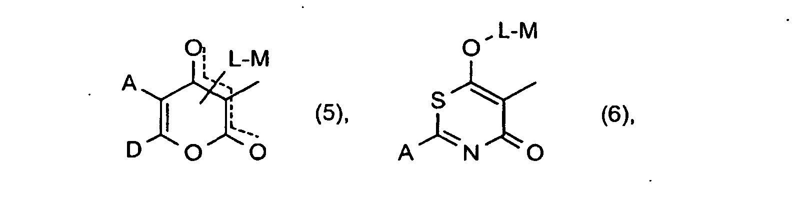 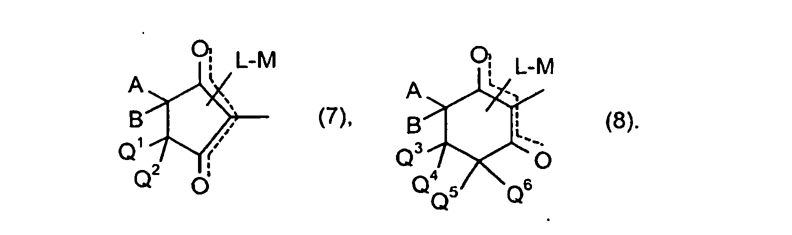
- A: steht ganz besonders bevorzugt für Wasserstoff, jeweils gegebenenfalls durch Fluor substituiertes C₁-C₈-Alkyl oder C₁-C₆-Alkoxy-C₁-C₄-alkyl, gegebenenfalls durch Fluor, Methyl, Ethyl oder Methoxy substituiertes C₃-C₆-Cycloalkyl, in welchem gegebenenfalls ein Ringglied durch Sauerstoff oder Schwefel ersetzt ist oder (jedoch nicht im Fall der Verbindungen der Formeln (1-5), (1-7) und (I-8)) für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl oder Benzyl.
- B: steht ganz besonders bevorzugt für Wasserstoff oder C₁-C₄-Alkyl oder
- A, B: und das Kohlenstoffatom an das sie gebunden sind, stehen ganz besonders bevorzugt für gesättigtes C₅-C₆-Cyloalkyl, in welchem gegebenenfalls ein Ringglied durch Sauerstoff oder Schwefel ersetzt ist und weiches gegebenenfalls einfach durch Methyl, Ethyl, n-Propyl, Isopropyl, Butyl, iso-Butyl, sec.-Butyl, tert.-Butyl, Trifluormethyl, Methoxy, Ethoxy, n-Propoxy, iso-Propoxy, n-Butoxy, iso-Butoxy, sek.-Butoxy, tert.-Butoxy, Fluor oder Chlor substituiert ist oder
- A, B: und das Kohlenstoffatom, an das sie gebunden sind, stehen ganz besonders bevorzugt für C₅-C₆-Cycloalkyl, worin zwei Substituenten gemeinsam mit den Kohlenstoffatomen, an die sie gebunden sind, für C₂-C₄-Alkandiyl oder C₂-C₄-Alkendiyl, worin jeweils gegebenenfalls eine Methylengruppe durch Sauerstoff oder Schwefel ersetzt ist oder Butadiendiyl stehen.
- D: steht ganz besonders bevorzugt für Wasserstoff, für jeweils gegebenenfalls durch Fluor oder Chlor substituiertes C₁-C₈-Alkyl, C₃-C₄-Alkenyl, C₁-C₆-Alkoxy-C₂-C₄-alkyl, C₁-C₄-Alkylthio-C₂-C₄-alkyl oder C₃-C₆-Cycloalkyl, in welchem gegebenenfalls eine Methylengruppe durch Sauerstoff oder Schwefel ersetzt ist oder (jedoch nicht im Fall der Verbindungen der Formeln (I-1) und (I-4)) für jeweils gegebenenfalls durch Fluor, Chlor, Methyl, Ethyl, n-Propyl, iso-Propyl, Methoxy, Ethoxy, Trifluormethyl oder Trifluormethoxy substituiertes Phenyl, Furanyl, Pyridyl, Thienyl oder Benzyl (in Verbindungen der Formel (I-1) hervorgehoben für Wasserstoff),
oder
- A und D: stehen gemeinsam ganz besonders bevorzugt für gegebenenfalls substituiertes C₃-C₄-Alkandiyl, worin gegebenenfalls ein Kohlenstoffatom durch Schwefel ersetzt ist und welches gegebenenfalls durch Hydroxy, Methyl, Ethyl, Methoxy oder Ethoxy substituiert ist oder
- A und D: stehen (im Fall der Verbindungen der Formel (I-1)) gemeinsam mit den Atomen, an die sie gebunden sind, für eine der folgenden Gruppen AD: 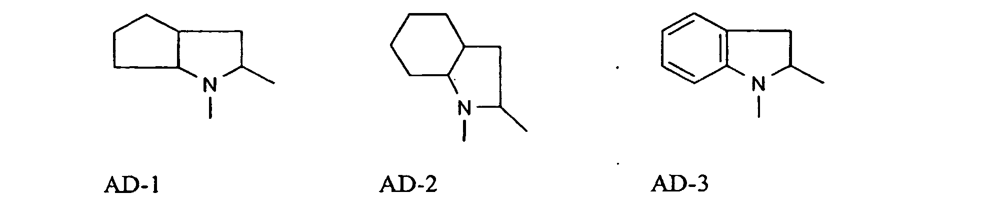 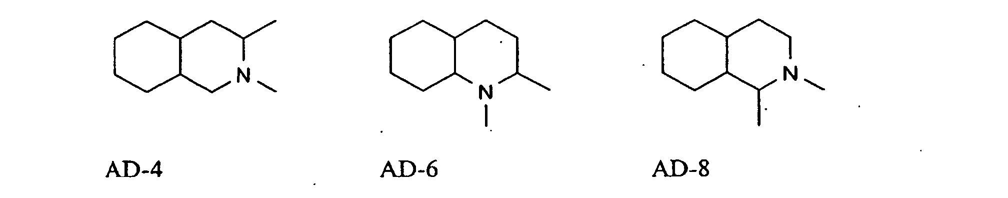 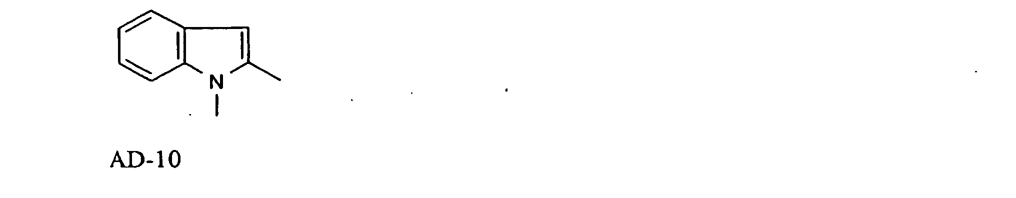
- A und Q¹: stehen gemeinsam ganz besonders bevorzugt für gegebenenfalls einfach oder zweifach durch Fluor, Hydroxy, Methyl oder Methoxy substituiertes C₃-C₄-Alkandiyl oder Butendiyl oder
- Q¹: steht ganz besonders bevorzugt für Wasserstoff.
- Q²: steht ganz besonders bevorzugt für Wasserstoff.
- Q⁴, Q⁵ und Q⁶: stehen ganz besonders bevorzugt unabhängig voneinander für Wasserstoff, Methyl oder Ethyl.
- Q³: steht ganz besonders bevorzugt für Wasserstoff, Methyl, Ethyl oder C₃-C₆-Cycloalkyl, worin gegebenenfalls eine Methylengruppe durch Sauerstoff oder Schwefel ersetzt (hervorgehoben für Wasserstoff, Methyl oder Ethyl) oder
- Q³ und Q⁴: stehen ganz besonders bevorzugt gemeinsam mit dem Kohlenstoff, an den sie gebunden sind, für gegebenenfalls durch Methyl oder Methoxy substituiertes gesättigtes C₅-C₆-Cycloalkyl, in welchem gegebenenfalls ein Ringglied durch Sauerstoff oder Schwefel ersetzt ist.
- L: steht ganz besonders bevorzugt für eine der folgenden Gruppierungen.

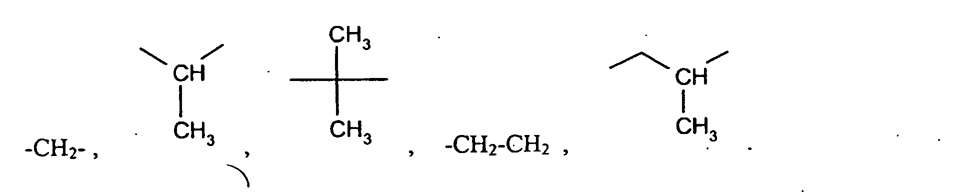

- M: steht ganz besonders bevorzugt für eine der folgenden Gruppierungen. CN; 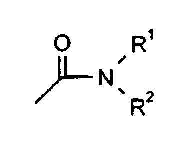 -CO₂R², -OR², -SR², -COR³, 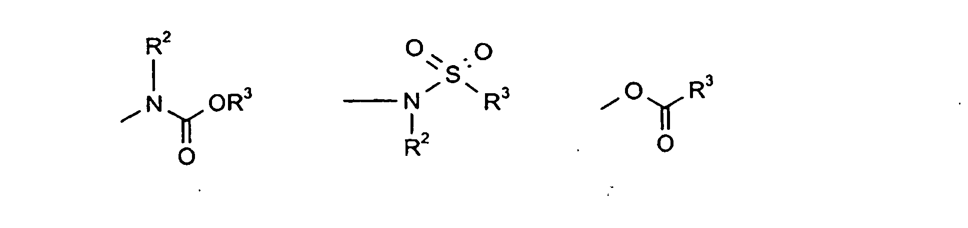 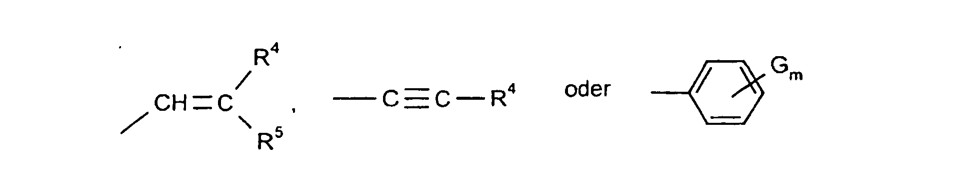
- R¹: steht ganz besonders bevorzugt für Wasserstoff oder C₁-C₈-Alkyl.
- R²: steht ganz besonders bevorzugt für jeweils gegebenenfalls durch Fluor, Chlor, Brom substituiertes C₁-C₈-Alkyl, C₃-C₄-Alkenyl, C₃-C₄-Alkinyl, Cyclopropyl, Cyclopentyl, Cyclohexyl, C₁-C₆-Alkoxy-C₂-C₄-alkyl, C₁-C₆-Alkylthio-C₂-C₄-alkyl, für jeweils gegebenenfalls durch Fluor, Chlor, Brom, Nitro, Cyano, Methylthio, Ethylthio, Methoxy, Ethoxy, Trifluormethylthio, Trifluormethoxy, Methyl, Ethyl, Trifluormethyl substituiertes Phenyl oder Benzyl.
- R³: steht ganz besonders bevorzugt für jeweils gegebenenfalls durch Fluor, Chlor, Brom substituiertes C₁-C₈-Alkyl oder durch Fluor, Chlor, C₁-C₂-Akyl, Trifluormethyl, C₁-C₂-Alkoxy oder Trifluormethoxy substituiertes Phenyl oder Benzyl.
- R⁴: steht ganz besonders bevorzugt für Wasserstoff, Fluor, Chlor, gegebenenfalls durch Fluor oder Chlor substituiertes C₁-C₄-Alkyl oder durch Fluor, Chlor, Methyl, Ethyl, Methoxy, Ethoxy, Trifluormethyl, Difluormethoxy, Trifluormethoxy, Cyano oder Nitro substituiertes Phenyl.
- R⁵: steht ganz besonders bevorzugt für Wasserstoff, Fluor, Chlor oder gegebenenfalls durch Fluor oder Chlor substituiertes Methyl, Ethyl, Propyl oder Isopropyl.
- G: steht ganz besonders bevorzugt für Fluor, Chlor, Methyl, Ethyl, Propyl, Isopropyl, Methoxy, Ethoxy, Propoxy, iso-Propoxy, Trifluormethyl, Difluormethoxy, Trifluormethoxy, Cyano oder Nitro.
- m: steht ganz besonders bevorzugt für eine Zahl von 0 bis 2.
- W: steht insbesondere bevorzugt für Wasserstoff, Chlor, Fluor, Methyl, Ethyl, Methoxy, Ethoxy (hervorgehoben für Wasserstoff, Methyl, Ethyl oder Chlor).
- X: steht insbesondere bevorzugt für Fluor, Chlor, Methyl, Ethyl, n-Propyl, iso-Propyl, Methoxy, Ethoxy, n-Propoxy, iso-Propoxy, Trifluormethyl, Difluormethoxy, Trifluormethoxy, Nitro oder Cyano (hervorgehoben für Chlor, Methyl, Ethyl, n-Propyl oder iso-Propyl).
- Y: steht insbesondere bevorzugt für einen der Reste 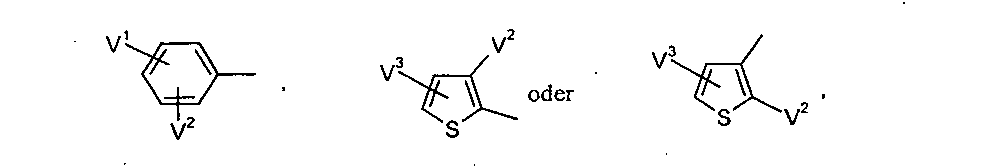 hervorgehoben für 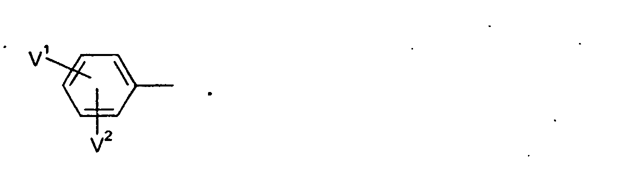
- V¹: steht insbesondere bevorzugt für Wasserstoff, Fluor, Chlor, Methyl, Ethyl, n-Propyl, iso-Propyl, n-Butyl, iso-Butyl, tert.-Butyl, Methoxy, Ethoxy, n-Propoxy, iso-Propoxy, Trifluormethyl, Trifluormethoxy, Nitro, Cyano oder Phenyl.
- V²: steht insbesondere bevorzugt für Wasserstoff, Fluor, Chlor, Methyl, Methoxy, Trifluormethyl oder Trifluormethoxy.
- V³: steht insbesondere bevorzugt für Wasserstoff, Methyl oder Chlor.
- Z: steht insbesondere bevorzugt für Wasserstoff, Fluor, Chlor, Methyl oder Methoxy, hervorgehoben für Wasserstoff oder Methyl.
- CKE: steht insbesondere bevorzugt für eine der Gruppen 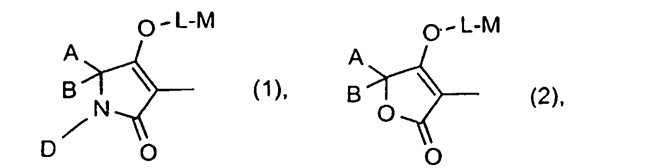 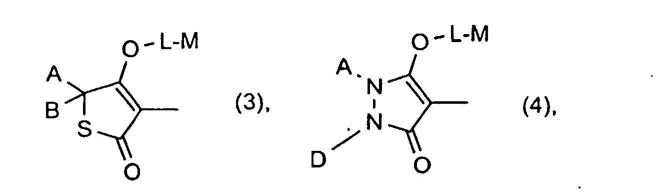 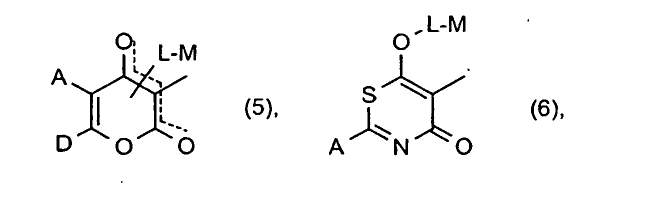 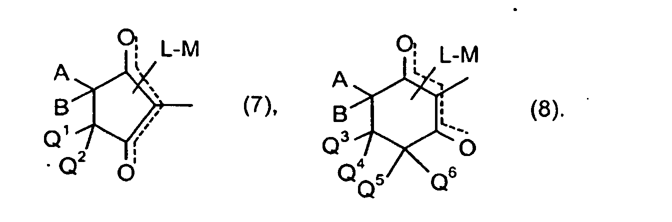
- A: steht insbesondere bevorzugt für Wasserstoff, jeweils gegebenenfalls durch Fluor substituiertes C₁-C₈-Alkyl oder C₁-C₆-Alkoxy-C₁-C₄-alkyl, gegebenenfalls durch Fluor, Methyl oder Methoxy substituiertes C₃-C₆-Cycloalkyl, in welchem gegebenenfalls ein Ringglied durch Sauerstoff oder Schwefel ersetzt ist oder (jedoch nicht im Fall der Verbindungen der Formeln (1-5), (1-7) und (I-8)) für jeweils gegebenenfalls durch Fluor, Chlor, Brom, Methyl, Methoxy, Trifluormethyl oder Trifluormethoxy, substituiertes Phenyl oder Benzyl.
- B: steht insbesondere bevorzugt für Wasserstoff oder C₁-C₄-Alkyl oder
- A, B: und das Kohlenstoffatom an das sie gebunden sind, stehen insbesondere bevorzugt für gesättigtes C₅-C₆-Cycloalkyl, in welchem gegebenenfalls ein Ringglied durch Sauerstoff oder Schwefel ersetzt ist und welches gegebenenfalls einfach durch Methyl, Ethyl, n-Propyl, Isopropyl, Trifluormethyl, Methoxy, Ethoxy, n-Propoxy, iso-Propoxy, n-Butoxy oder iso-Butoxy substituiert ist oder
- A, B: und das Kohlenstoffatom, an das sie gebunden sind, stehen insbesondere bevorzugt für C₅-C₆-Cycloalkyl, worin zwei Substituenten gemeinsam mit den Kohlenstoffatomen, an die sie gebunden sind, für C₂-C₄-Alkandiyl oder C₂-C₄-Alkendiyl, worin jeweils gegebenenfalls eine Methylengruppe durch Sauerstoff oder Schwefel ersetzt ist oder Butadiendiyl stehen.
- D: steht insbesondere bevorzugt für Wasserstoff, für jeweils gegebenenfalls durch Fluor oder Chlor substituiertes C₁-C₆-Alkyl, C₃-C₄-Alkenyl, C₁-C₄-Alkoxy-C₂-C₄-alkyl, C₁-C₄-Alkylthio-C₂-C₄-alkyl oder C₃-C₆-Cycloalkyl, in welchem gegebenenfalls eine Methylengruppe durch Sauerstoff oder Schwefel ersetzt ist oder (jedoch nicht im Fall der Verbindungen der Formeln (1-1) und (I-4)), für jeweils gegebenenfalls durch Fluor, Chlor, Methyl, Methoxy, Trifluormethyl oder Trifluormethoxy substituiertes Phenyl, Pyridyl oder Benzyl
oder
- A und D: stehen gemeinsam insbesondere bevorzugt für gegebenenfalls substituiertes C₃-C₄-Alkandiyl, worin gegebenenfalls ein Kohlenstoffatom durch Schwefel ersetzt ist und welches gegebenenfalls durch Methyl oder Methoxy substituiert ist.
- A und Q¹: stehen gemeinsam insbesondere bevorzugt für gegebenenfalls einfach oder zweifach durch Methyl oder Methoxy substituiertes C₃-C₄-Alkandiyl oder Butendiyl oder
- Q¹: steht insbesondere bevorzugt für Wasserstoff.
- Q²: steht insbesondere bevorzugt für Wasserstoff.
- Q⁴, Q⁵ und Q⁶: stehen insbesondere bevorzugt unabhängig voneinander für Wasserstoff, Methyl oder Ethyl.
- Q³: steht insbesondere bevorzugt für Wasserstoff, Methyl, Ethyl oder C₃-C₆-Cycloalkyl, worin gegebenenfalls eine Methylengruppe durch Sauerstoff oder Schwefel ersetzt (hervorgehoben für Wasserstoff, Methyl oder Ethyl) oder
- Q³ und Q⁴: stehen insbesondere bevorzugt gemeinsam mit dem Kohlenstoff, an den sie gebunden sind, für gegebenenfalls durch Methyl oder Methoxy substituiertes gesättigtes C₅-C₆-Cycloalkyl, in welchem gegebenenfalls ein Ringglied durch Sauerstoff oder Schwefel ersetzt ist.
- L: steht insbesondere bevorzugt für eine der folgenden Gruppierungen. -CH₂-, 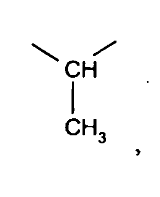 -CH₂CH₂.
- M: steht insbesondere bevorzugt für eine der folgenden Gruppierungen. CN; 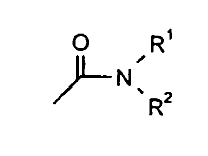 -CO₂R², -OR², -SR², -COR³, 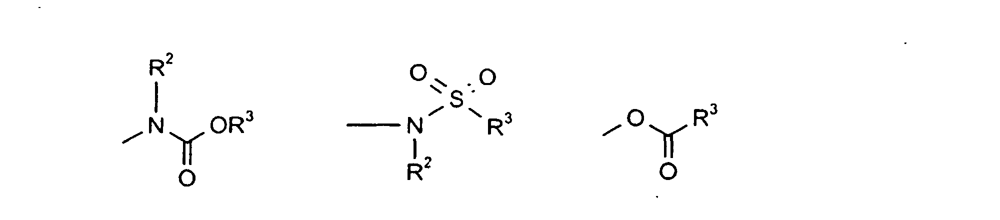 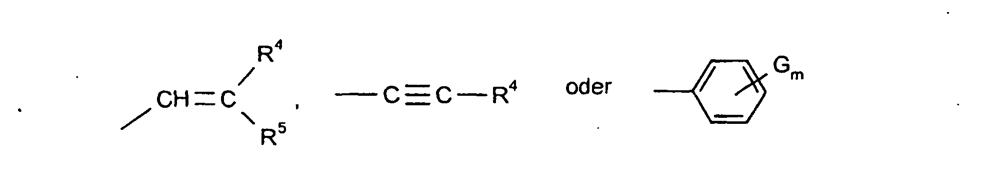
- R¹: steht insbesondere bevorzugt für Wasserstoff oder C₁-C₄-Alkyl.
- R²: steht insbesondere bevorzugt für jeweils gegebenenfalls durch Fluor substituiertes C₁-C₄-Alkyl, C₃-C₄-Alkenyl, C₃-C₄-Alkinyl, Cyclopropyl, Cyclopentyl, Cyclohexyl, C₁-C₄-Alkoxy-C₂-C₄-alkyl, für jeweils gegebenenfalls durch Fluor, Chlor, Brom, Nitro, Cyano, Methylthio, Ethylthio, Methoxy, Ethoxy, Trifluormethylthio, Trifluormethoxy, Methyl, Ethyl, Trifluormethyl substituiertes Phenyl oder Benzyl.
- R³: steht insbesondere bevorzugt für jeweils gegebenenfalls durch Fluor substituiertes C₁-C₄-Alkyl oder durch Fluor, Chlor, Methyl, Trifluormethyl, Methoxy oder Trifluormethoxy substituiertes Phenyl.
- R⁴: steht insbesondere bevorzugt für Wasserstoff, Fluor, Chlor, gegebenenfalls durch Fluor substituiertes C₁-C₄-Alkyl oder durch Fluor, Chlor, Methyl, Ethyl, Methoxy, Ethoxy, Trifluormethyl, Difluormethoxy, Trifluormethoxy, Cyano oder Nitro substituiertes Phenyl.
- R⁵: steht insbesondere bevorzugt für Wasserstoff, Fluor, Chlor oder gegebenenfalls durch Fluor substituiertes Methyl.
- G: steht insbesondere bevorzugt für Fluor, Chlor, Methyl, Methoxy, Trifluormethyl, Difluormethoxy, Trifluormethoxy, Cyano oder Nitro.
- m: steht insbesondere bevorzugt für eine Zahl von 0 bis 1.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (1), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Insbesondere bevorzugt sind Verbindungen der Formel (I), in denen G für Wasserstoff steht.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-1) genannt:

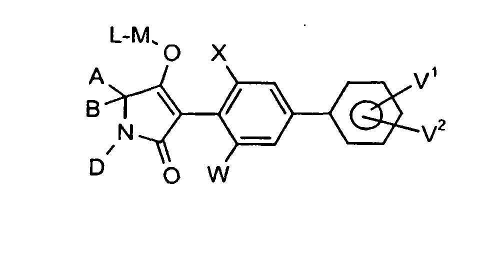

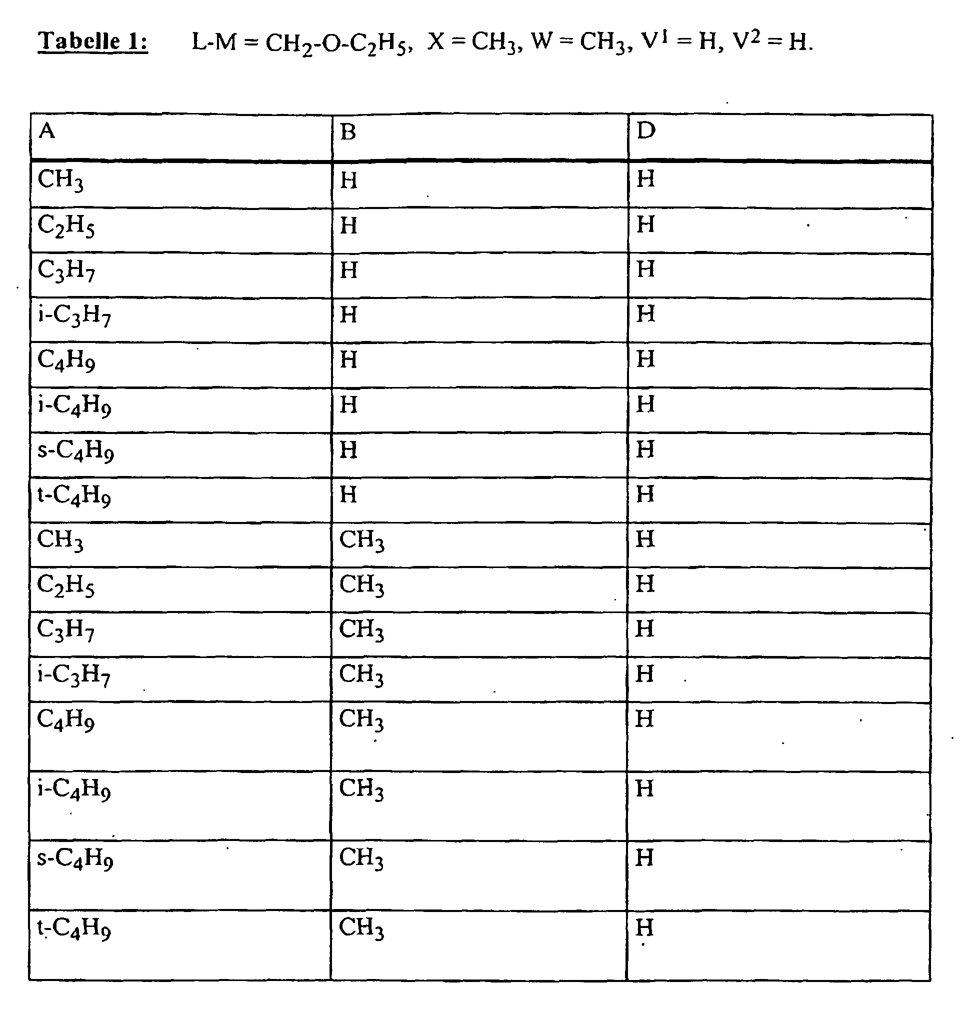

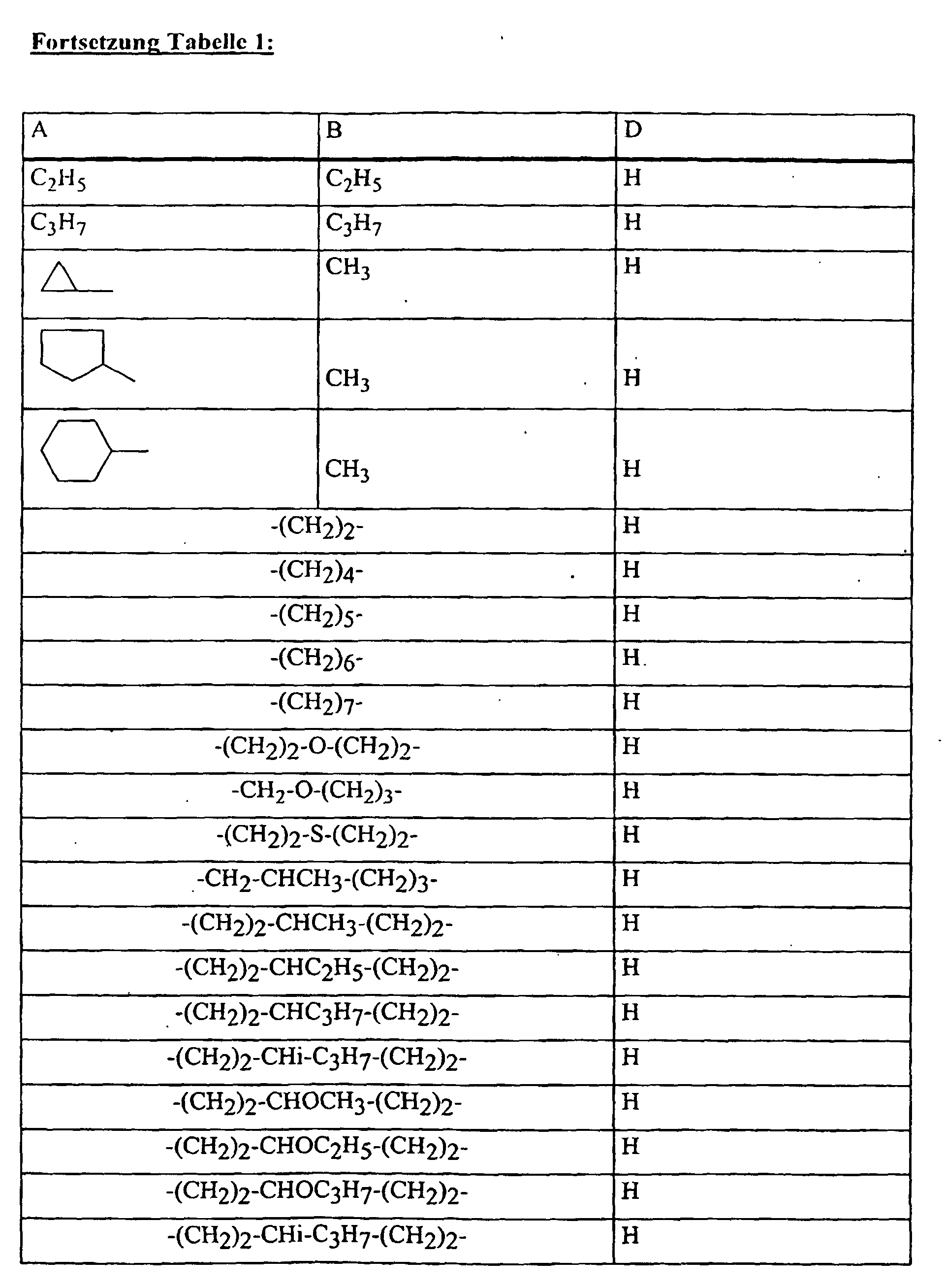

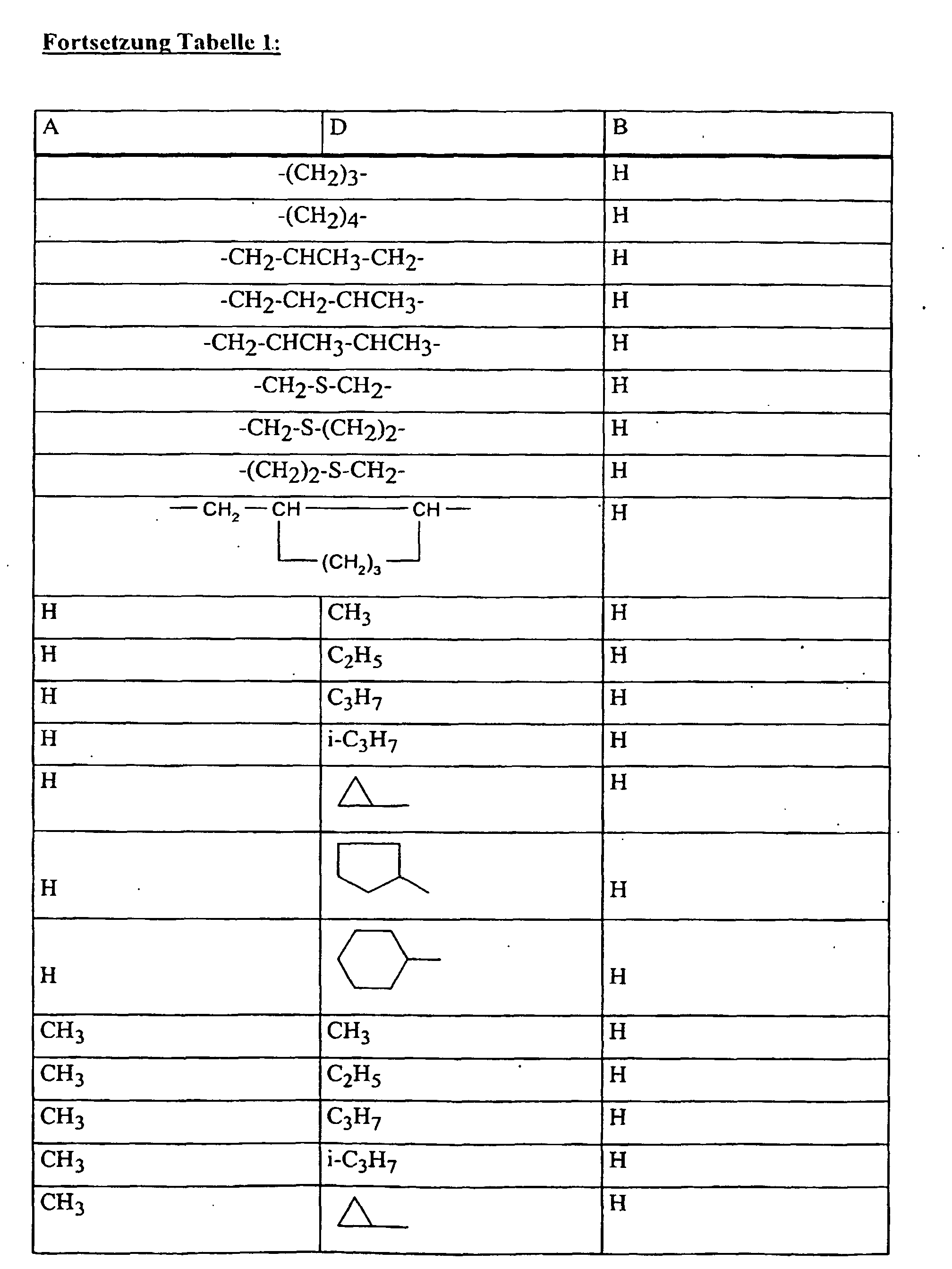

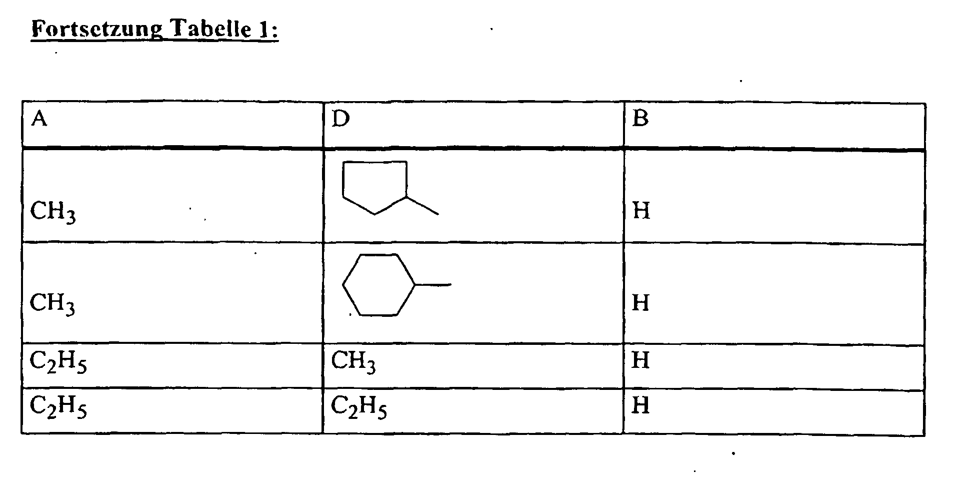

- **Tabelle 2:**: A, B, D, L und M wie in Tabelle 1 angegeben
X = C₂H₅; W = CH₃; V¹ = H; V² = H.
- **Tabelle 3:**: A, B, D, L und M wie in Tabelle 1 angegeben
X = C₂H₅; W = C₂H₅; V¹ = H; V² = H.
- **Tabelle 4:**: A, B, D, L und M wie in Tabelle 1 angegeben
X = CH₃; W = CH₃; V¹ = 4-Cl; V² = H.
- **Tabelle 5:**: A, B, D, L und M wie in Tabelle 1 angegeben
X = C₂H₅; W = CH₃; V¹ = 4-Cl; V² = H.
- **Tabelle 6:**: A, B, D, L und M wie in Tabelle 1 angegeben
X = C₂H₅; W = C₂H₅; V = 4-Cl; V² = H.
- **Tabelle 7:**: A, B, D, L und M wie in Tabelle 1 angegeben
X = CH₃; W = CH₃; V¹ = 3-Cl; V² = H.
- **Tabelle 8:**: A, B, D, L und M wie in Tabelle 1 angegeben
X = C₂H₅; W = CH₃; V¹ = 3-Cl; V² = H.
- **Tabelle 9:**: A, B, D, L und M wie in Tabelle 1 angegeben
X = C₂H₅; W = C₂H₅; V ¹ = 3-Cl; V² = H.
- **Tabelle 10:**: A, B, D, L und M wie in Tabelle 1 angegeben
X = CH₃; W = CH_{3;} V¹ = 2-Cl; V² = 4-Cl.
- **Tabelle 11:**: A, B, D, L und M wie in Tabelle 1 angegeben
X = C₂H₅; W = CH₃; V¹ = 2-Cl; V² = 4-Cl.
- **Tabelle 12:**: A, B, D, L und M wie in Tabelle 1 angegeben
X = C₂H₅; W = C₂H₅; V¹ = 2-Cl; V² = 4-Cl.
- **Tabelle 13:**: A, B, D, L und M wie in Tabelle 1 angegeben
X = CH₃; W = CH₃; V¹ = 4-CF₃; V² = H.
- **Tabelle 14:**: A, B, D, L und M wie in Tabelle 1 angegeben
X = C₂H₅; W = CH₃; V¹ = 4-CF₃; V² = H.
- **Tabelle 15:**: A, B, D, L und M wie in Tabelle 1 angegeben
X = C₂H₅; W = C₂H₅; V¹ = 4-CF₃; V² = H.
- **Tabelle 16:**: A, B, D, L und M wie in Tabelle 1 angegeben
X = CH₃; W = CH₃; V ¹ = 4-CH₃; V² = H.
- **Tabelle 17:**: A, B, D, L und M wie in Tabelle 1 angegeben
X = C₂H₅; W = CH₃; V¹ = 4-CH₃; V² = H.
- **Tabelle 18:**: A, B, D, L und M wie in Tabelle 1 angegeben
X = C₂H₅; W = C₂H₅; V¹ = 4-CH₃; V² = H.
- **Tabelle 19:**: A, B, D, L und M wie in Tabelle 1 angegeben
X = CH₃; W = CH₃; V¹ = 4-OCH₃; V² = H.
- **Tabelle 20:**: A, B, D, L und M wie in Tabelle 1 angegeben
X = C₂H₅; W = CH₃; V¹ = 4-OCH₃; V² = H.
- **Tabelle 21:**: A, B, D, L und M wie in Tabelle 1 angegeben
X = C₂H₅; W = C₂H₅; V¹ = 4-OCH₃; V² = H.

Im einzelnen seien außer den bei den Herstellungsbeispielen genannten Verbindungen die folgenden Verbindungen der Formel (I-1) genannt:

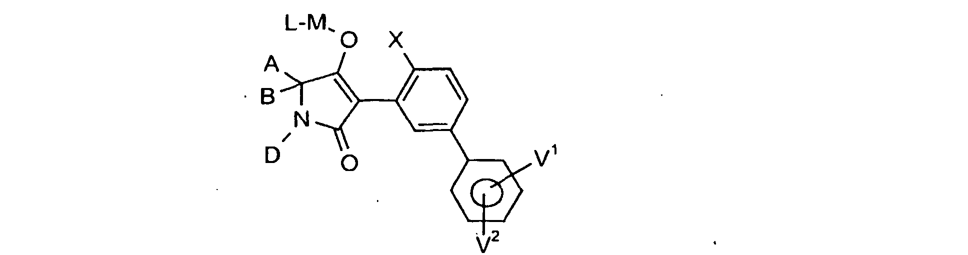

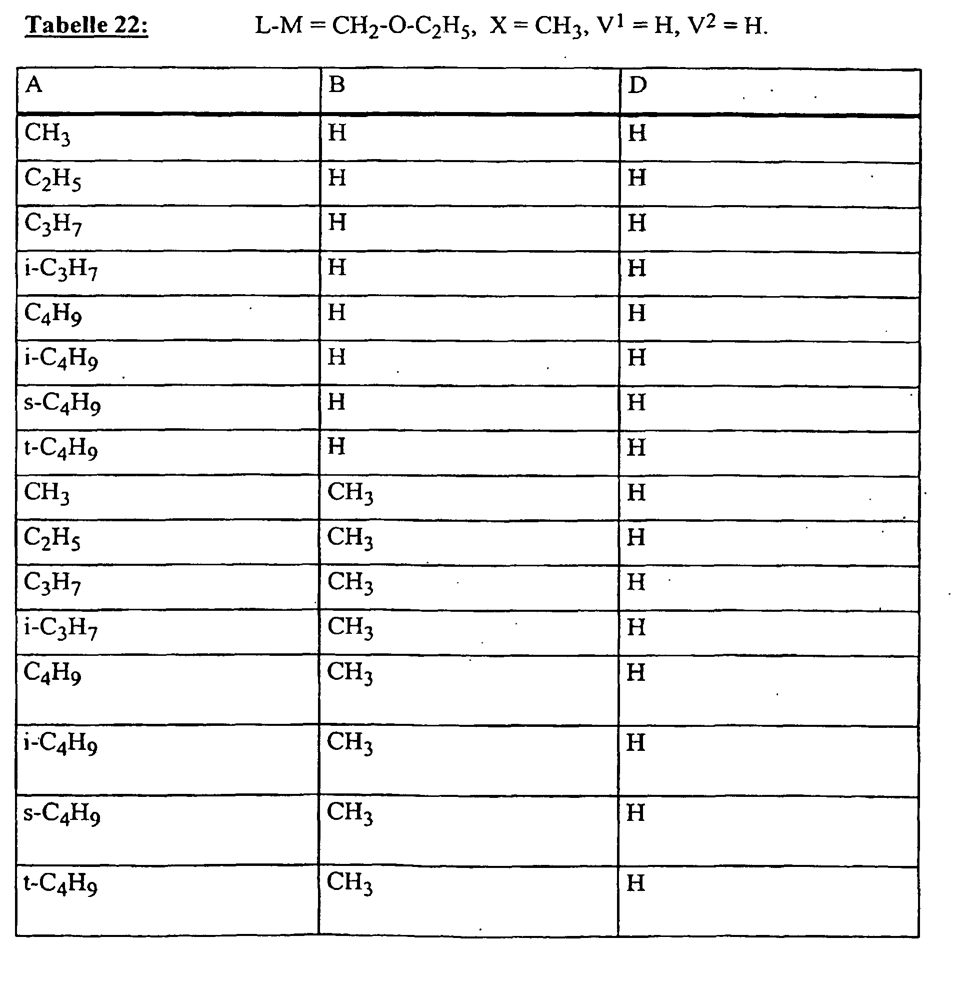

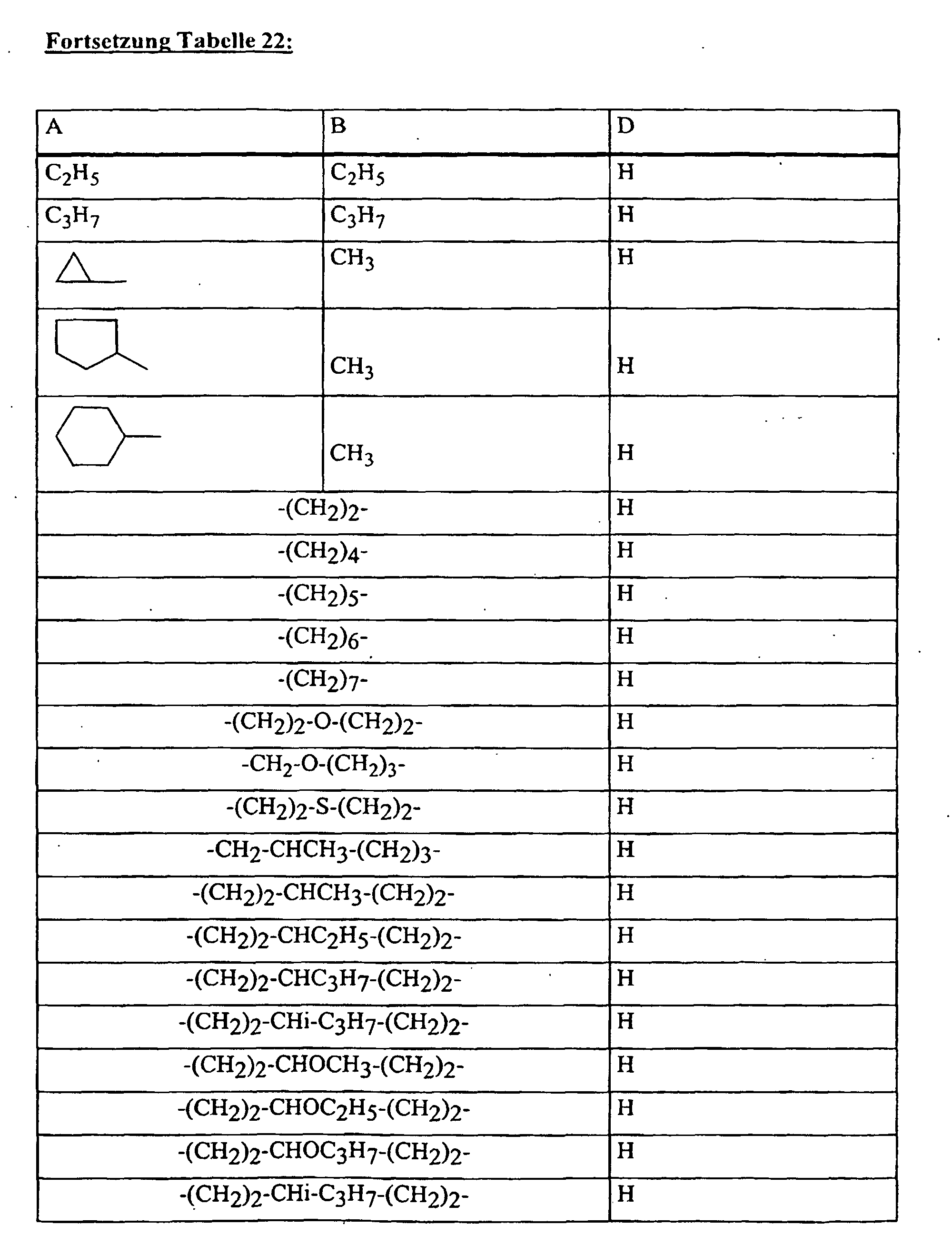

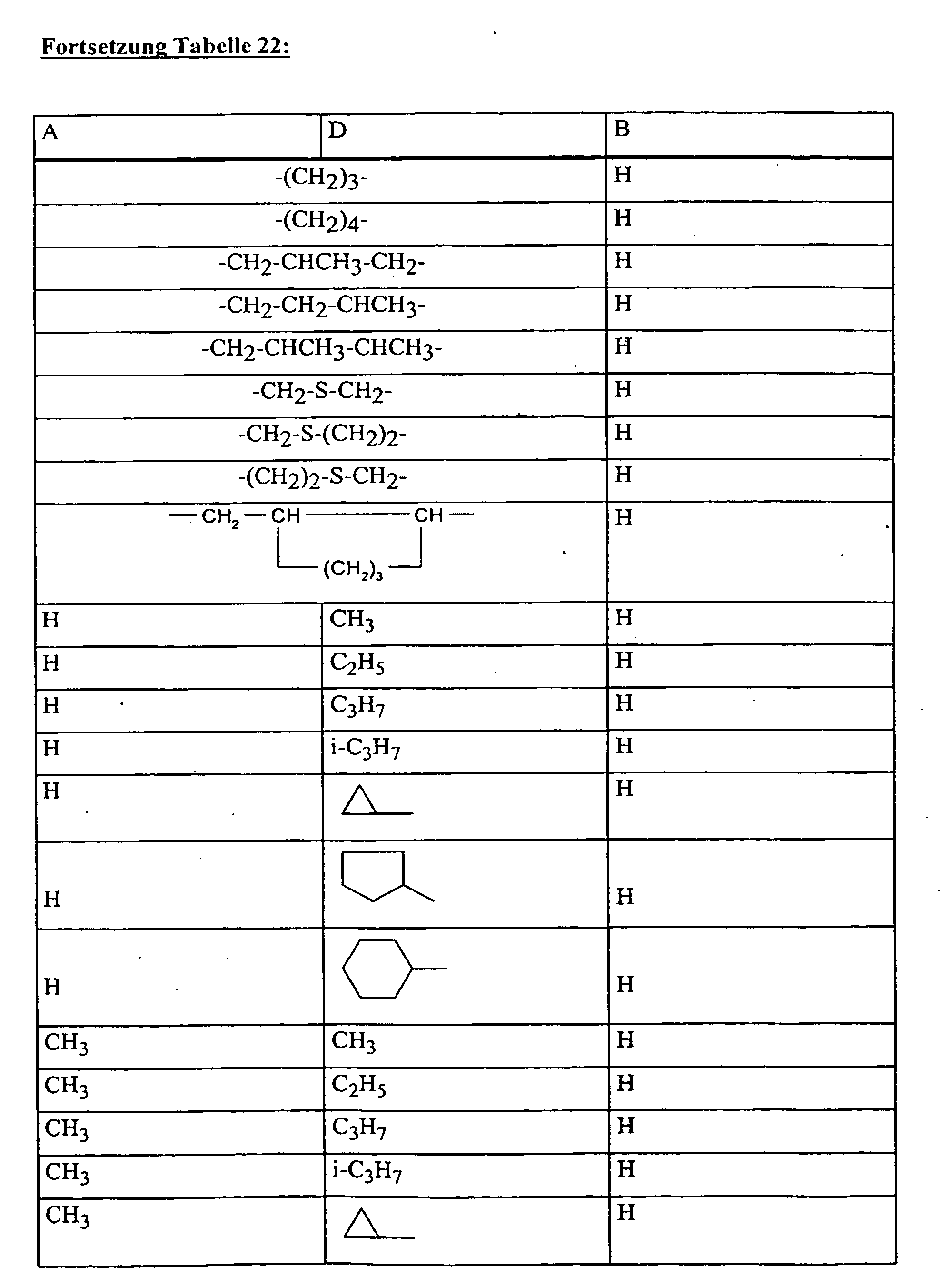

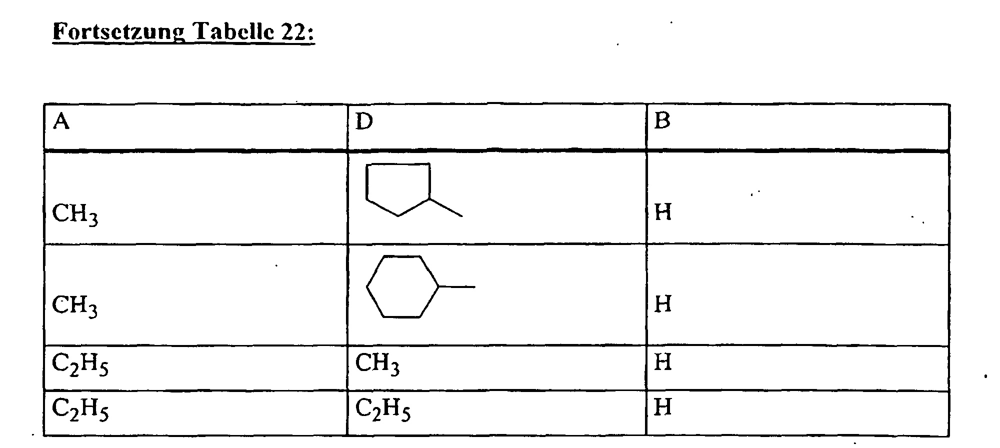

- **Tabelle 23:**: A, B, D, L und M, wie in Tabelle 22 angegeben
X=Cl V¹=H; V²=H.
- **Tabelle 24:**: A, B, D, L und M, wie in Tabelle 22 angegeben
X = C₂H₅; V¹ = H; V² = H.
- **Tabelle 25:**: A, B, D, L und M, wie in Tabelle 22 angegeben
X = CH₃; V¹ = 4-Cl; V² = H.
- **Tabelle 26:**: A, B, D, L und M, wie in Tabelle 22 angegeben
X = C₂H₅; V¹ = 4-Cl; V² = H.
- **Tabelle 27:**: A, B, D, L und M, wie in Tabelle 22 angegeben
X = Cl; V¹ = 4-Cl; V² = H.
- **Tabelle 28:**: A, B, D, L und M, wie in Tabelle 22 angegeben
X = CH₃; V¹ = 3-Cl; V² = H.
- **Tabelle 29:**: A, B, D, L und M, wie in Tabelle 22 angegeben
X = C₂H₅; V¹ = 3-Cl; V² = H.
- **Tabelle 30:**: A, B, D, L und M, wie in Tabelle 22 angegeben
X = Cl; V¹ = 3-Cl; V² = H.
- **Tabelle 31:**: A, B, D, L und M, wie in Tabelle 22 angegeben
X = CH₃; V ¹ = 2-Cl; V² = 4-Cl.
- **Tabelle 32:**: A, B, D, L und M, wie in Tabelle 22 angegeben
X = C₂H₅; V¹ = 2-Cl; V² = 4-Cl.
- **Tabelle 33:**: A, B, D, L und M, wie in Tabelle 22 angegeben
X = Cl; V¹ = 2-Cl; V² = 4-Cl.
- **Tabelle 34:**: A, B, D, L und M, wie in Tabelle 22 angegeben
X = CH₃; V¹ = 4-CF₃; V² = H.
- **Tabelle 35:**: A, B, D, L und M, wie in Tabelle 22 angegeben
**.** X = C₂H₅; V¹ = 4-CF₃; V² = H.
- **Tabelle 36:**: A, B, D, L und M, wie in Tabelle 22 angegeben
X = Cl; V¹ = 4-CF₃; V² = H.
- **Tabelle 37:**: A, B, D, L und M, wie in Tabelle 22 angegeben
X = CH₃; V¹ = 4-CH₃; V² = H.
- **Tabelle 38:**: A, B, D, L und M, wie in Tabelle 22 angegeben
X = C₂H₅; V¹ = 4-CH₃; V² = H.
- **Tabelle 39:**: A, B, D, L und M, wie in Tabelle 22 angegeben
X = Cl; V¹ = 4-CH₃; V² = H.
- **Tabelle 40:**: A, B, D, L und M, wie in Tabelle 22 angegeben
X = CH₃; V¹ = 4-OCH₃; V² = H.
- **Tabelle 41:**: A, B, D, L und M, wie in Tabelle 22 angegeben
X = C₂H₅; V¹ = 4-OCH₃; V² = H.
- **Tabelle 42:**: A, B, D, L und M, wie in Tabelle 22 angegeben
X = Cl; V¹ = 4-OCH₃; V² = H.

Im einzelnen seien außer den bei den Herstellungsbeispielen genannten Verbindungen die folgenden Verbindungen der Formel (1-1) genannt:

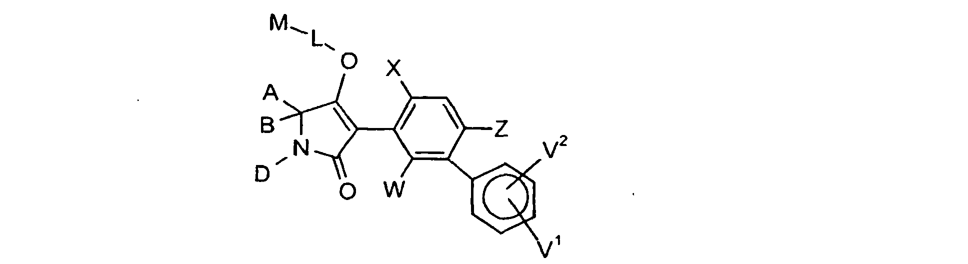

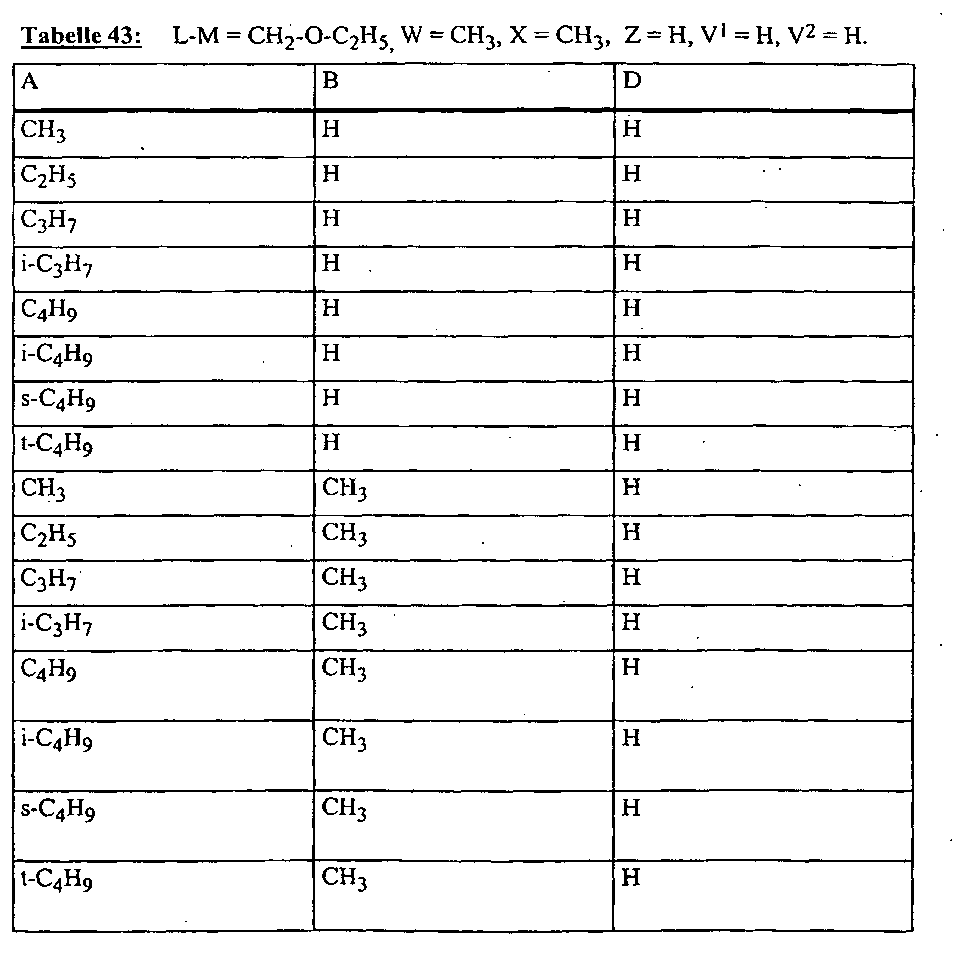

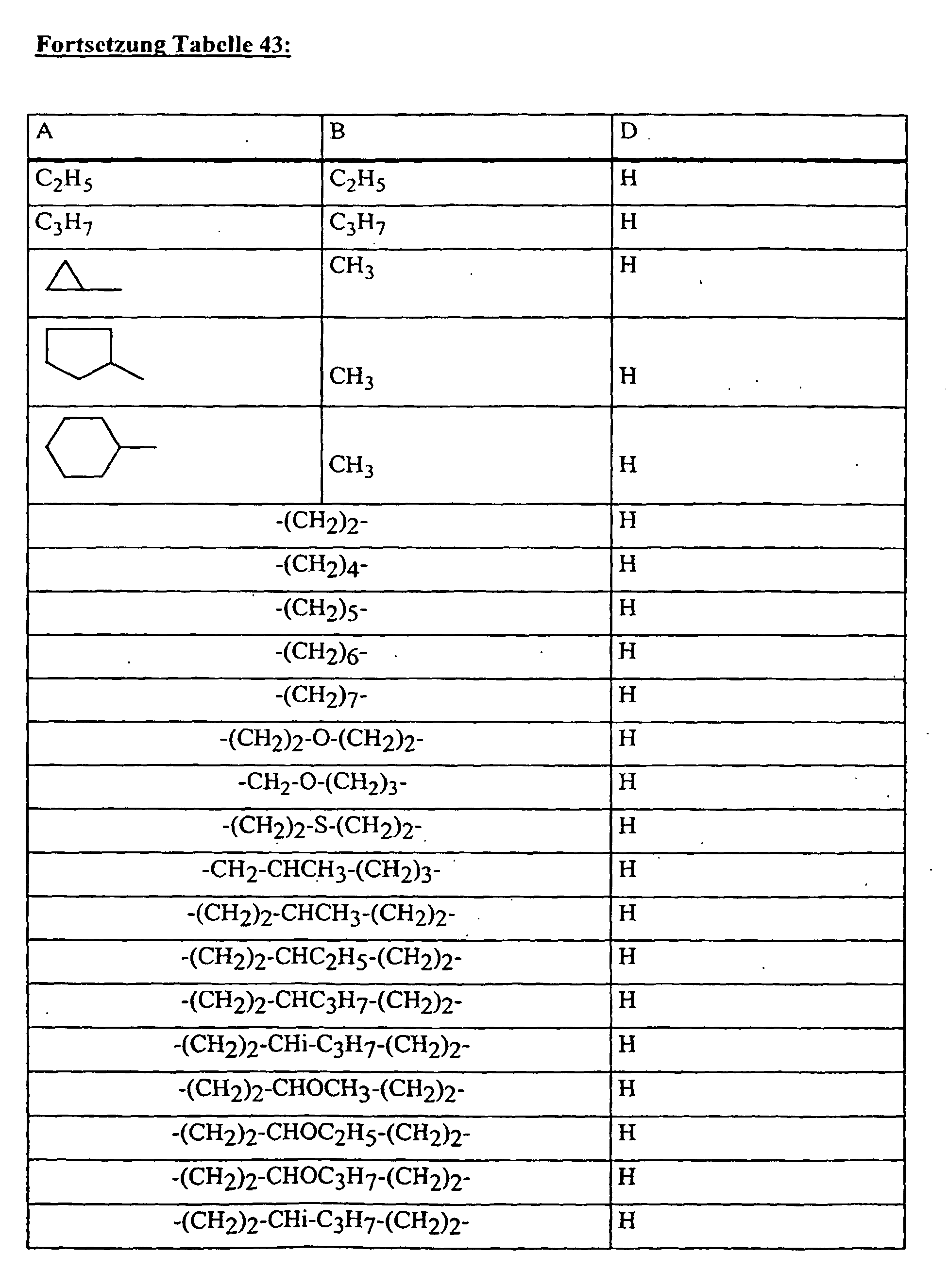

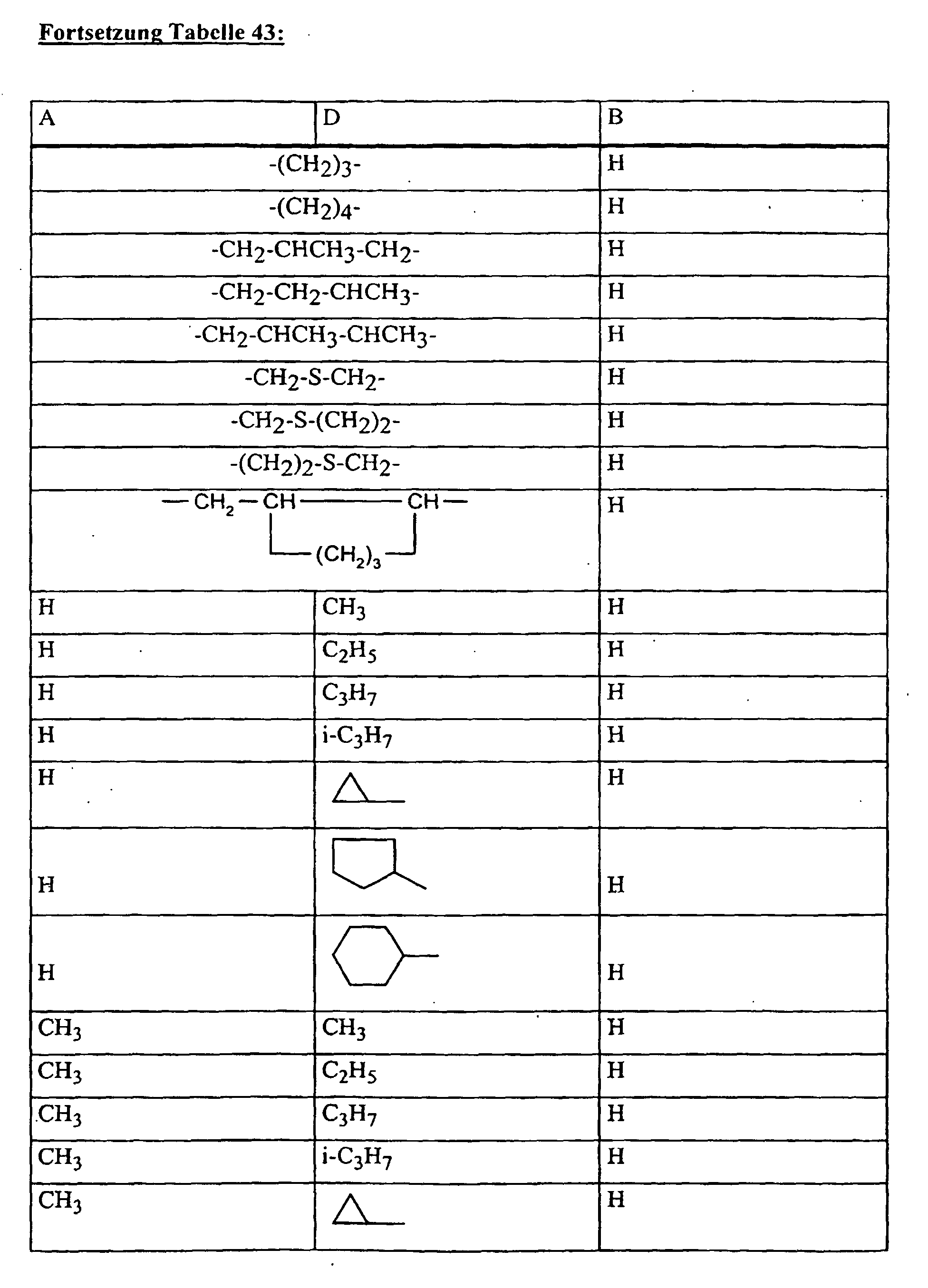

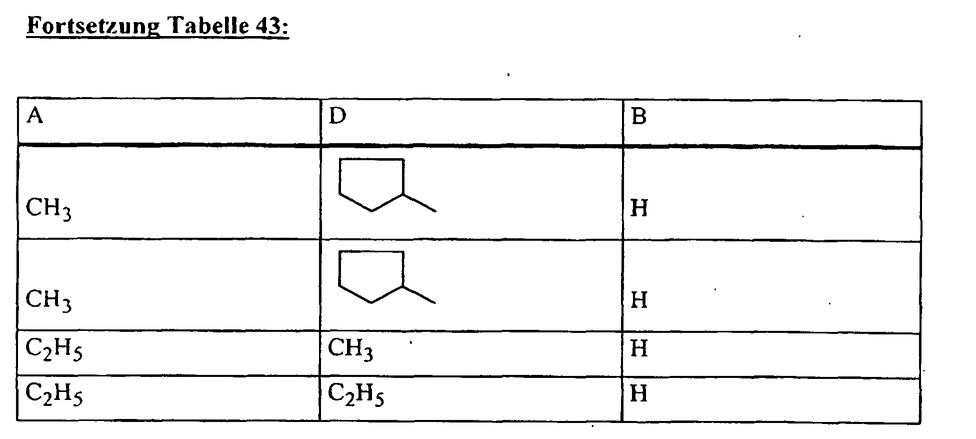

- **Tabelle 44:**: A, B, D, L und M wie in Tabelle 43 angegeben
W = H; X = CH₃; Z = CH₃; V¹=H; V²=H.
- **Tabelle 45:**: A, B, D, L und M wie in Tabelle 43 angegeben
W = CH₃; X = CH₃; Z = H; V¹ = 4-Cl; V² = H.
- **Tabelle 46:**: A, B, D, L und M wie in Tabelle 43 angegeben
W = H; X = CH₃; Z = CH₃; V¹ = 4-Cl; V² = H.
- **Tabelle 47:**: A, B, D, L und M wie in Tabelle 43 angegeben
W =CH₃; X = CH₃; Z = H; V¹ = 3-Cl; V² = H.
- **Tabelle 48:**: A, B, D, L und M wie in Tabelle 43 angegeben
W = H; X = CH₃; Z = CH₃; V¹ = 3-Cl; V² = H.
- **Tabelle 49:**: A, B, D, L und M wie in Tabelle 43 angegeben
W = CH₃; X = CH₃; Z = H; V¹ = 2-Cl; V² = 4-Cl.
- **Tabelle 50:**: A, B, D, L und M wie in Tabelle 43 angegeben
W = H; X = CH₃; Z = CH₃; V¹ = 2-Cl; V² = 4-Cl.
- **Tabelle 51**: **:** A, B, D, L und M wie in Tabelle 43 angegeben
W = CH₃; X = CH₃; Z = H; V¹ = 4-CF₃; V² = H.
- **Tabelle 52:**: A, B, D, L und M wie in Tabelle 43 angegeben
W = H; X = C₂H₅; Z = CH₃; V¹ = 4-CF₃; V² = H.
- **Tabelle 53:**: A, B, D, L und M wie in Tabelle 43 angegeben
W = CH₃; X = CH₃; Z = H; V¹ = 4-CH₃; V² = H.
- **Tabelle 54:**: A, B, D, L und M wie in Tabelle 43 angegeben
W = H; X = C₂H₅; Z = CH₃; V¹ = 4-CH₃; V² = H.
- **Tabelle 55:**: A, B, D, L und M wie in Tabelle 43 angegeben
W = CH₃; X = CH₃; Z = H; V = 4-OCH₃; V² = H.
- **Tabelle 56:**: A, B, D, L und M wie in Tabelle 43 angegeben
W = H; X = C₂H₅; Z = CH₃; V¹ = 4-OCH₃; V² = H.

Im einzelnen seien außer den bei den Herstellungsbeispielen genannten Verbindungen die folgenden Verbindungen der Formel (1-2) genannt:

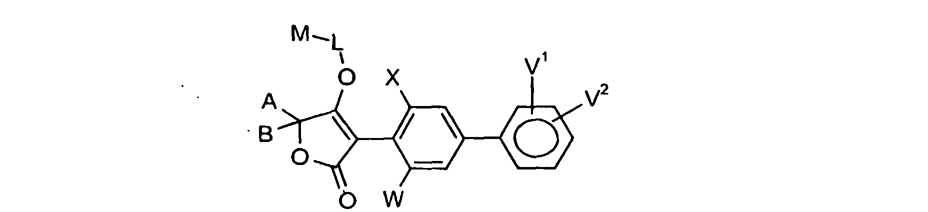

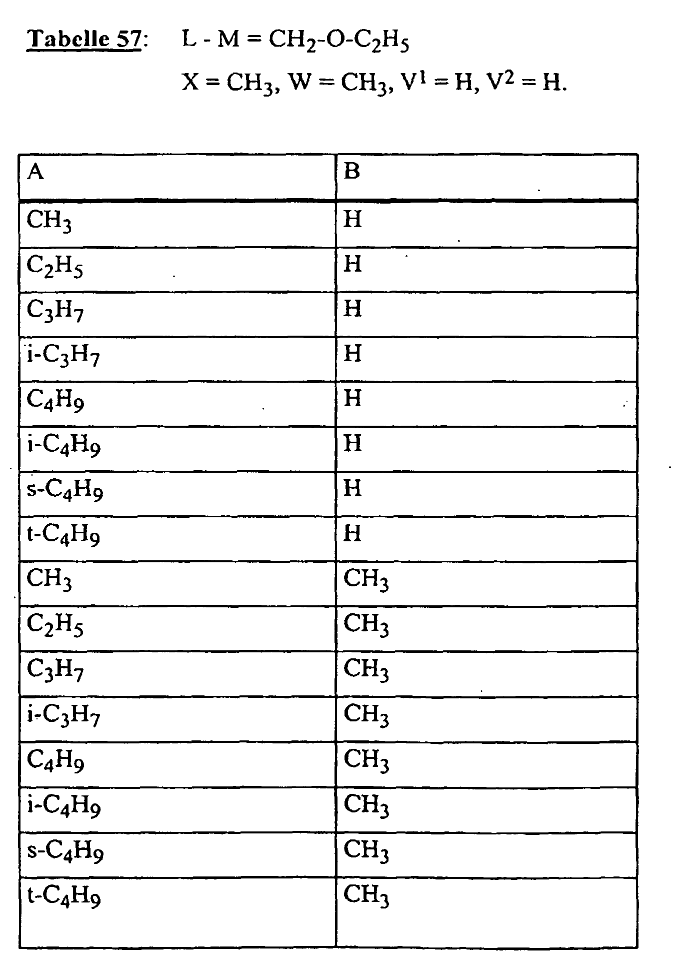

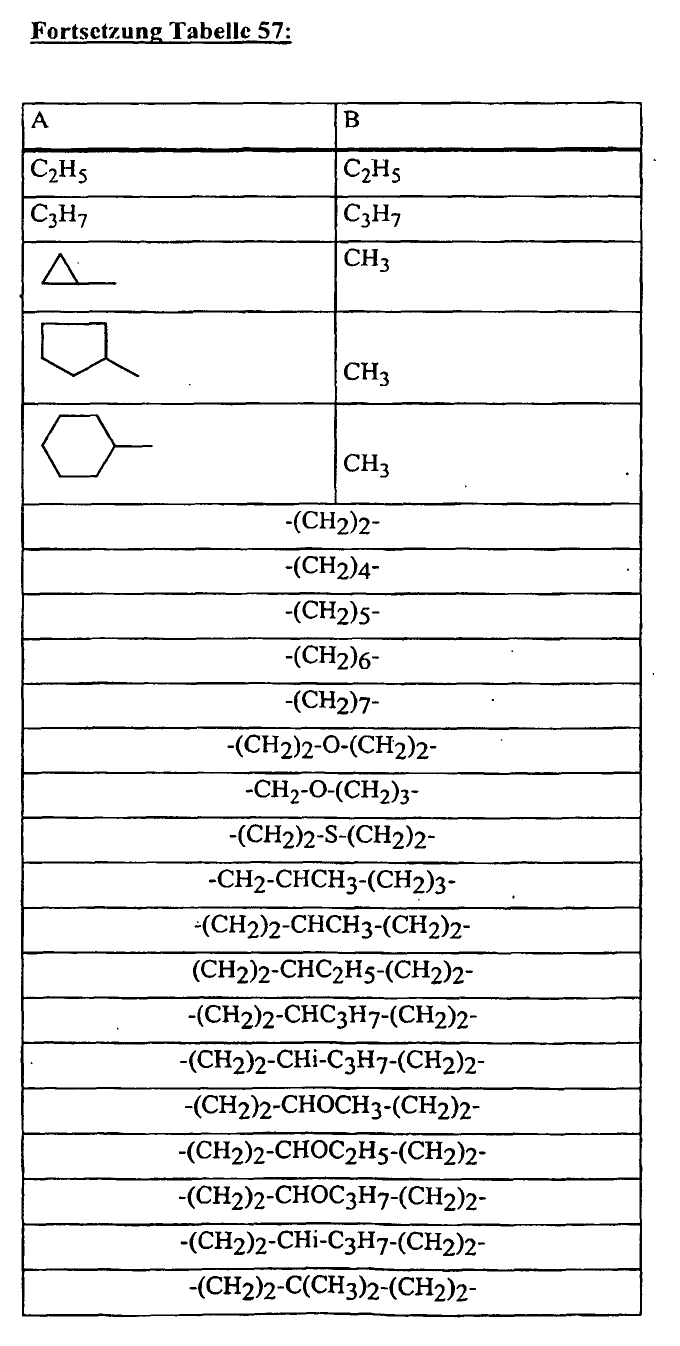

>

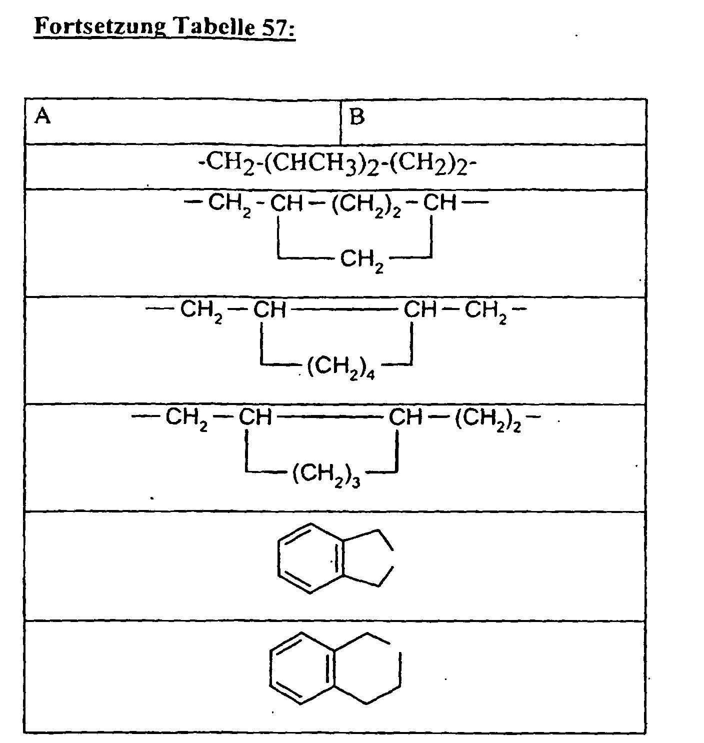

- **Tabelle 58:**: A, B, L und M wie in Tabelle 57 angegeben
X = C₂H₅; W = CH₃; V¹ = H; V² = H.
- **Tabelle 59:**: A, B, L und M wie in Tabelle 57 angegeben
X = C₂H₅; W = C₂H₅; V¹ = H; V² =H.
- **Tabelle 60:**: A, B, L und M wie in Tabelle 57 angegeben
X = CH₃; W = CH₃; V¹ = 4-Cl; V² = H.
- **Tabelle 61:**: A, B, L und M wie in Tabelle 57 angegeben
X = C₂H₅; W = CH₃; V¹ = 4-Cl; V² = H.
- **Tabelle 62:**: A, B, L und M wie in Tabelle 57 angegeben
X = C₂H₅; W = C₂H₅; V¹ = 4-Cl; V² = H.
- **Tabelle 63:**: A, B, L und M wie in Tabelle 57 angegeben
X = CH₃; W = CH₃; V¹ = 3-Cl; V² = H.
- **Tabelle 64:**: A, B, L und M wie in Tabelle 57 angegeben
X = C₂H₅; W = CH₃; V¹ = 3-Cl; V² = H.
- **Tabelle 65:**: A, B, L und M wie in Tabelle 57 angegeben
X = C₂H₅; W = C₂H₅; V¹ = 3-Cl; V² = H.
- **Tabelle 66:**: A, B, L und M wie in Tabelle 57 angegeben
X = CH₃; W = CH₃; V¹ = 4-CF₃; V² = H.
- **Tabelle 67:**: A, B, L und M wie in Tabelle 57 angegeben
X = C₂H₅; W = CH₃; V¹ = 4-CF₃; V² = H.
- **Tabelle 68:**: A, B, L und M wie in Tabelle 57 angegeben
X = C₂H₅; W = C₂H₅; V¹ = 4-CF₃; V² = H.
- **Tabelle 69:**: A, B, L und M wie in Tabelle 57 angegeben
X = CH₃; W = CH₃; V¹ = 2-Cl; V² = 4-Cl.
- **Tabelle 70:**: A, B, L und M wie in Tabelle 57. angegeben
X = C₂H₅; W= CH₃; V¹ = 2-Cl; V² = 4-Cl.
- **Tabelle 71:**: A, B, L und M wie in Tabelle 57 angegeben
X = C₂H₅; W = C₂H₅; V¹ = 2-Cl; V² = 4-Cl.
- **Tabelle 72:**: A, B, L und M wie in Tabelle 5,7 angegeben
X = CH₃; W = CH₃; V¹ = 4-CH₃; V² = H.
- **Tabelle 73:**: A, B, L und M wie in Tabelle 57 angegeben
X = C₂H₅; W = CH₃; V¹ = 4-CH₃; V² = H.
- **Tabelle 74:**: A, B, L und M wie in Tabelle 57 angegeben
X = C₂H₅; W = C₂H₅; V¹ = 4-CH₃; V² = H.
- **Tabelle 75:**: A, B, L und M wie in Tabelle 57 angegeben
X = CH₃; W = CH₃; V¹ = 4-OCH₃; V² = H.
- **Tabelle 76:**: A, B, L und M wie in Tabelle 57 angegeben
X = C₂H₅; W= CH₃; V¹ = 4-OCH₃; V² = H.
- **Tabelle 77:**: A, B, L und M wie in Tabelle 57 angegeben
X = C₂H₅; W = C₂H₅; V ¹ = 4-OCH₃; V² = H.

Im einzelnen seien außer den bei den Herstellungsbeispielen genannten Verbindungen die folgenden Verbindungen der Formel (I-2) genannt:

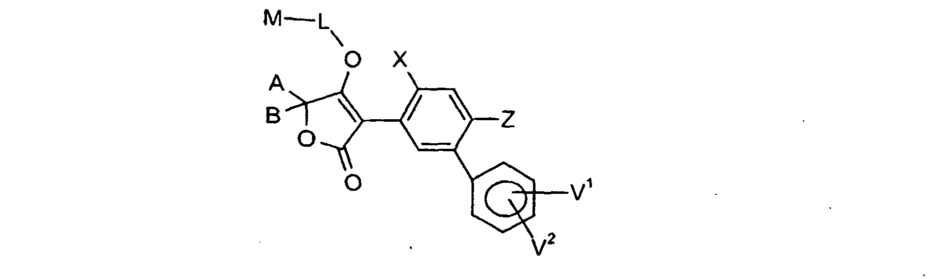

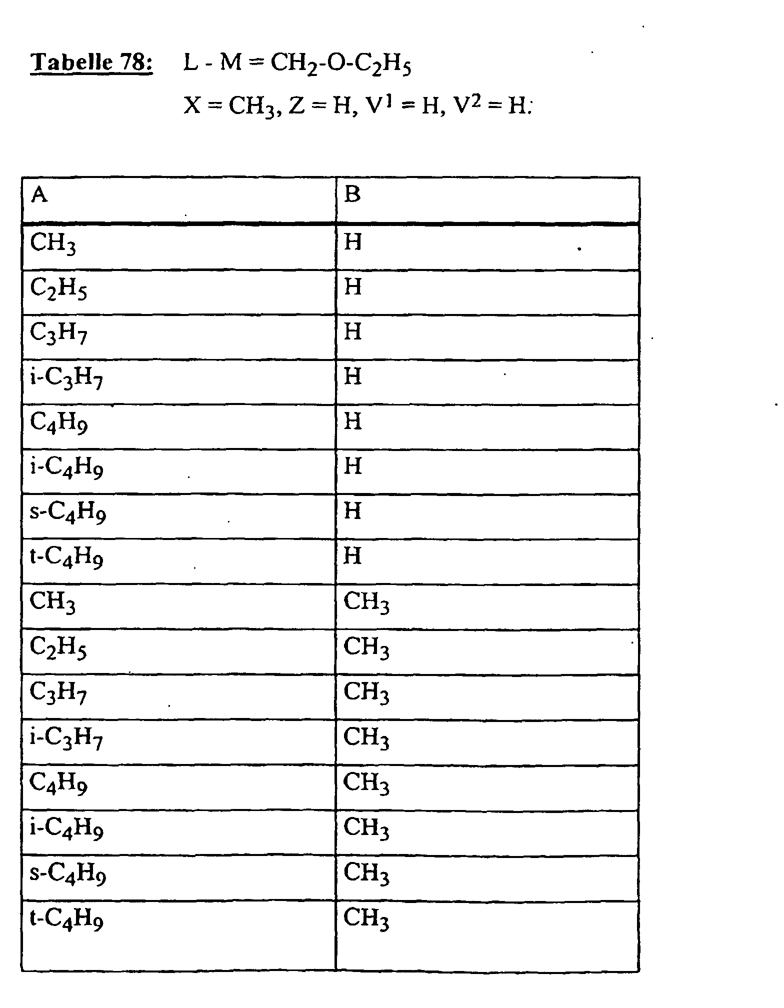

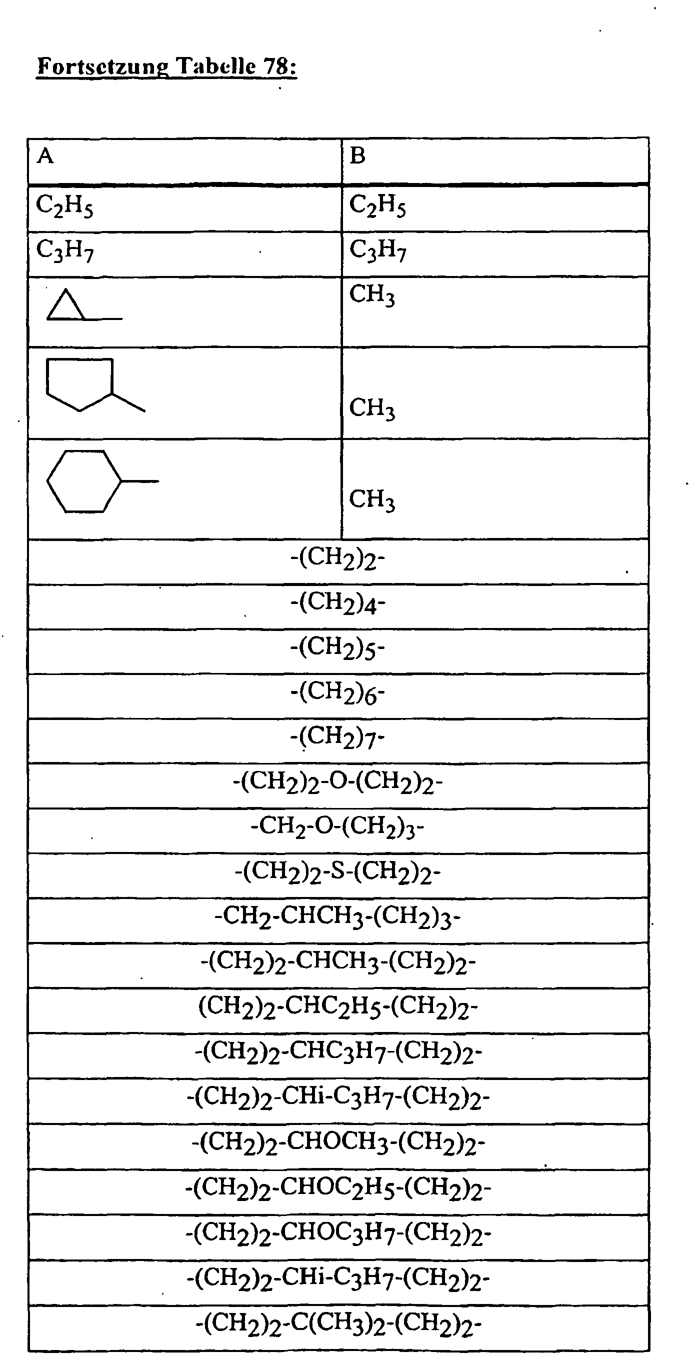

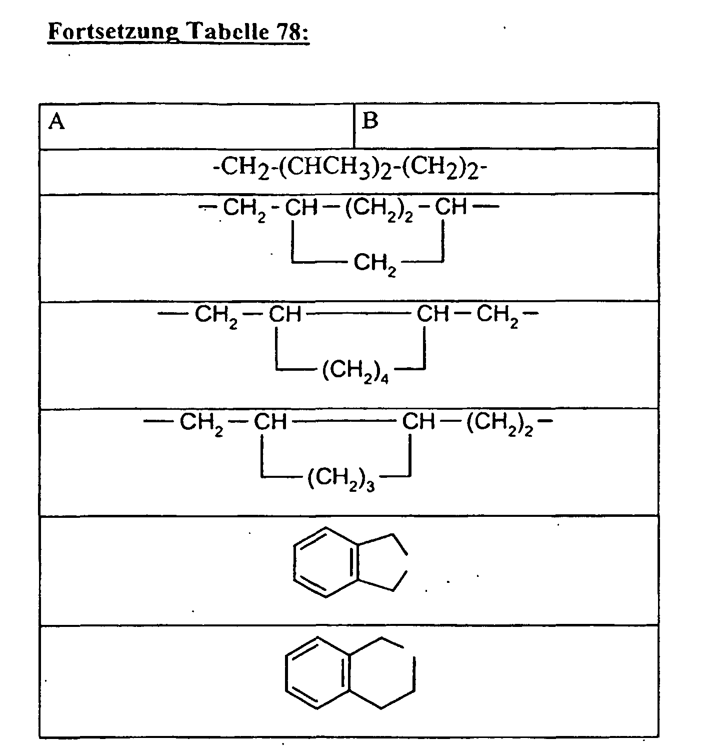

- **Tabelle 79:**: A, B, L und M wie in Tabelle 78 angegeben
X = Cl; Z = H; V¹ =H; V²=H.
- **Tabelle 80:**: A, B, L und M wie in Tabelle 78 angegeben
X = CH₃; Z = CH₃; V¹ = H; V² =H.
- **Tabelle 81:**: A, B, L und M wie in Tabelle 78 angegeben
X = CH₃; Z = H; V¹ = 4-Cl; V2 = H.
- **Tabelle 82:**: A, B, L und M wie in Tabelle 78 angegeben
X = Cl; Z = H; V¹ = 4-Cl; V² = H.
- **Tabelle 83:**: A, B, L und M wie in Tabelle 78 angegeben
X = CH₃; Z = CH₃; V¹ = 4-Cl; V² = H.
- **Tabelle 84:**: A, B, L und M wie in Tabelle 78 angegeben
X = CH₃; Z = H; V¹ =3-Cl; V²=H.
- **Tabelle 85:**: A, B, L und M wie in Tabelle 78 angegeben
X = Cl; Z = H; V¹ = 3-Cl; V² = H.
- **Tabelle 86.**: A, B, L und M wie in Tabelle 78 angegeben
X = CH₃; Z = CH₃; V¹ = 3-Cl; V² = H.
- **Tabelle 87:**: A, B, L und M wie in Tabelle 78 angegeben
X = CH₃; Z = H; V ¹ = 4-CF₃; V² = H.
- **Tabelle 88:**: A, B, L und M wie in Tabelle 78 angegeben
X = Cl; Z = H; V¹ = 4-CF₃; V² = H.
- **Tabelle 89:**: A, B, L und M wie in Tabelle 78 angegeben
X = CH₃; Z = CH₃; V¹ = 4-CF₃; V² = H.
- **Tabelle 90:**: A, B, L und M wie in Tabelle 78 angegeben
X = CH₃; Z = H; V¹ = 2-Cl; V² = 4-Cl.
- **Tabelle 91:**: A, B, L und M wie in Tabelle 78 angegeben
X = Cl; Z = H; V¹ = 2-Cl; V² = 4-Cl.
- **Tabelle 92:**: A, B, L und M wie in Tabelle 78 angegeben
X = CH₃; Z = CH₃; V¹ = 2-Cl; V² = 4-Cl.
- **Tabelle 93:**: A, B, L und M wie in Tabelle 78 angegeben
X = CH₃; Z = H; V¹ = 4-CH₃; V² = H.
- **Tabelle 94:**: A, B, L und M wie in Tabelle 78 angegeben
X = Cl; Z = H; V¹ = 4-CH₃; V² = H.
- **Tabelle 95**: **:** A, B, L und M wie in Tabelle 78 angegeben
X = CH₃; Z = CH₃; V¹ = 4-CH₃; V² = H.
- **Tabelle 96:**: A, B, L und M wie in Tabelle 78 angegeben
X = CH₃; Z = H; V¹ = 4-OCH₃; V² = H.
- **Tabelle 97:**: A, B, L und M wie in Tabelle 78 angegeben
X = Cl; Z = H; V¹ = 4-OCH₃; V² = H.
- **Tabelle 98:**: A, B, L und M wie in Tabelle 78 angegeben
X = CH₃; Z = CH₃; V¹ = 4-OCH₃; V² = H.

Im einzelnen seien außer den bei den Herstellungsbeispielen genannten Verbindungen die folgenden Verbindungen der Formel (1-7) genannt:

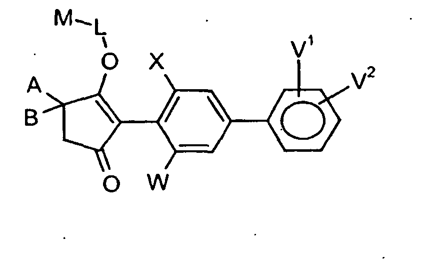

**Tabelle 99:**

| L - M = CH₂-O-C₂H₅ X = CH₃, W = CH₃, V¹ = H, V² = H. | |
|---|---|
| A | B |
| CH₃ | H |
| C₂H₅ | H |
| C₃H₇ | H |
| i-C₃H₇ | H |
| CH₃ | CH₃ |
| C₂H₅ | CH₃ |
| C₃H₇ | CH₃ |
| i-C₃H₇ | CH₃ |
| -(CH₂)₄- | |
| -(CH₂)₅- | |
| -(CH₂)₂-O-(CH₂)₂- | |
| -CH₂-O-(CH₂)₃- | |
| -(CH₂)₂-CHCH₃-(CH₂)₂- | |
| -(CH₂)₂-CHOCH₃-(CH₂)₂- | |
| -(CH₂)₂-CHOC₂H₅-(CH₂)₂- | |
| -(CH₂)₂-CHOC₃H₇-(CH₂)₂- | |

- **Tabelle 100:**: A, B, L und M wie in Tabelle 99 angegeben
X = C₂H₅; W = CH₃; V¹ = H; V² = H.
- **Tabelle 101:**: A, B, L und M wie in Tabelle 99 angegeben
X = C₂H₅; W = C₂H₅; V¹ = H; V² =H.
- **Tabelle 102:**: A, B, L und M wie in Tabelle 99 angegeben
X = CH₃; W = CH₃; V¹ = 4-Cl; V² = H.
- **Tabelle 103:**: A, B, L und M wie in Tabelle 99 angegeben
X = C₂H₅; W = CH₃; V¹ = 4-Cl; V² = H.
- **Tabelle 104:**: A, B, L und M wie in Tabelle 99 angegeben
X = C₂H₅; W = C₂H₅; V¹ = 4-Cl; V² = H.
- **Tabelle 105:**: A, B, L und M wie in Tabelle 99 angegeben
X = CH₃; W = CH₃; V¹ = 3-Cl; V² = H.
- **Tabelle 106:**: A, B, L und M wie in Tabelle 99 angegeben
X = C₂H₅; W = CH₃; V¹ = 3-Cl; V² = H.
- **Tabelle 107:**: A, B, L und M wie in Tabelle 99 angegeben
X = C₂H₅; W = C₂H₅; V¹ = 3-Cl; V² = H.
- **Tabelle 108:**: A, B, L und M wie in Tabelle 99 angegeben
X = CH₃; W = CH₃; V¹ = 4-CF₃; V² = H.
- **Tabelle 109:**: A, B, L und M wie in Tabelle 99 angegeben
X = C₂H₅; W = CH₃; V¹ = 4-CF₃; V² = H.
- **Tabelle 110:**: A, B, L und M wie in Tabelle 99 angegeben
X = C₂H₅; W = C₂H₅; V¹ = 4-CF₃; V² = H.
- **Tabelle 111:**: A, B, L und M wie in Tabelle 99 angegeben
X = CH₃; W = CH₃; V¹ = 2-Cl; V² = 4-Cl.
- **Tabelle 112:**: A, B, L und M wie in Tabelle 99 angegeben
X = C₂H₅; W = CH₃; V¹ = 2-Cl; V² = 4-Cl.
- **Tabelle 113:**: A, B, L und M wie in Tabelle 99 angegeben
X = C₂H₅; W = C₂H₅; V¹ = 2-Cl; V² = 4-Cl.
- **Tabelle 114:**: A, B, L und M wie in Tabelle 99 angegeben
X = CH₃; W = CH₃; V¹ = 4-CH₃; V² = H.
- **Tabelle** 115:: A, B, L und M wie in Tabelle 99 angegeben
X = C₂H₅; W = CH₃; V¹ = 4-CH₃; V² = H.
- **Tabelle 116:**: A, B, L und M wie in Tabelle 99 angegeben
X = C₂H₅; W = C₂H₅; V¹ = 4-CH₃; V² = H.
- **Tabelle 117:**: A, B, L und M wie in Tabelle 99 angegeben
X = CH₃; W = CH₃; V¹ = 4-OCH₃; V² = H.
- **Tabelle 118:**: A, B, L und M wie in Tabelle 99 angegeben
X = C₂H₅; W = CH₃; V¹ = 4-OCH₃; V² = H.
- **Tabelle 119:**: A, B, L und M wie in Tabelle 99 angegeben
X = C₂H₅; W = C₂H₅; V¹ = 4-OCH₃; V² = H.

Im einzelnen seien außerdem bei den Herstellungsbeispielen genannten Verbindungen die folgenden Verbindungen der Formel (1-7) genannt:

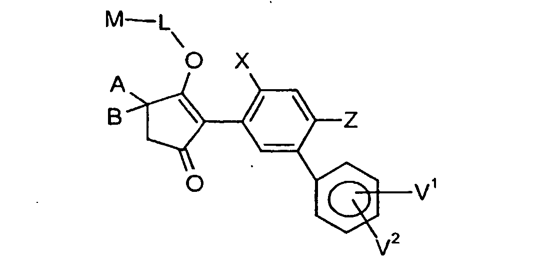

**Tabelle 120:**

| L - M = CH₂-O-C₂H₅ X=CH₃, Z=H, V¹ = H, V² = H. | |
|---|---|
| A | B |
| CH₃ | H |
| C₂H₅ | H |
| C₃H₇ | H |
| i-C₃H₇ | H |
| CH₃ | CH₃ |
| C₂H₅ | CH₃ |
| C₃H₇ | CH₃ |
| i-C₃H₇ | CH₃ |
| -(CH2)4- | |
| -(CH2)5- | |
| -(CH₂)₂-O-(CH₂)₂- | |
| -CH₂-O-(CH₂)₃- | |
| -(CH₂)₂-CHCH₃-(CH₂)₂- | |
| -(CH₂)₂-CHC₂H₅-(CH₂)₂- | |
| -(CH₂)₂-CHOCH₃-(CH₂)₂- | |
| -(CH₂)₂-CHOC₂H₅-(CH₂)₂- | |
| -(CH₂)₂-CHOC₃H₇-(CH₂)₂- | |

- **Tabelle 121:**: A, B, L und M wie in Tabelle 120 angegeben
X=Cl; Z=H; V¹ =N; V²=H.
- **Tabelle 122:**: A, B, L und M wie in Tabelle 120 angegeben
X = CH₃; Z = CH₃; V¹ = H; V² = H.
- **Tabelle 123:**: A, B, L und M wie in Tabelle 120 angegeben
X = CH₃; Z = H; V¹ = 4-Cl; V² = H.
- **Tabelle 124:**: A, B, L und M wie in Tabelle 120 angegeben
X = Cl; Z = H; V¹ = 4-Cl; V² = H.
- **Tabelle 125:**: A, B, L und M wie in Tabelle 120 angegeben
X = CH₃; Z = CH₃; V¹ = 4-Cl; V² = H.
- **Tabelle 126:**: A, B, L und M wie in Tabelle 120 angegeben
X = CH₃; Z = H; V¹ = 3-Cl; V² = H.
- **Tabelle 127:**: A, B, L und M wie in Tabelle 120 angegeben
X=Cl; Z=H; V¹ =3-Cl; V²=H.
- **Tabelle 128:**: A, B, L und M wie in Tabelle 120 angegeben
X = CH₃; Z = CH₃; V¹ = 3-Cl; V² = H.
- **Tabelle 129:**: A, B, L und M wie in Tabelle 120 angegeben
X = CH₃; Z = H; V¹ = 2-Cl; V² = 4-Cl.
- **Tabelle 130:**: A, B, L und M wie in Tabelle 120 angegeben
X = Cl; Z = H; V¹ = 2-Cl; V² = 4-Cl.
- **Tabelle 131:**: A, B, L und M wie in Tabelle 120 angegeben
X = CH₃; Z = CH₃; V¹ = 2-Cl; V² = 4-Cl.
- **Tabelle 132:**: A, B, L und M wie in Tabelle 120 angegeben
X = CH₃; Z = H; V¹ = 4-CF₃; V² = H.
- **Tabelle 133:**: A, B, L und M wie in Tabelle 120 angegeben
X = Cl; Z = H; V¹ = 4-CF₃; V² = H.
- **Tabelle 134:**: A, B, L und M wie in Tabelle 120 angegeben
X = CH₃; Z = CH₃; V¹ = 4-CF₃; V² = H.
- **Tabelle 135:**: A, B, L und M wie in Tabelle 120 angegeben
X = CH₃; Z = H; V¹ = 4-CH₃; V² = H.
- **Tabelle 136:**: A, B, L und M wie in Tabelle 120 angegeben
X = Cl; Z = H; V¹ = 4-CH₃; V² = H.
- **Tabelle 137:**: A, B, L und M wie in Tabelle 120 angegeben
X = CH₃; Z = CH₃; V¹ = 4-CH₃; V² = H.
- **Tabelle 138:**: A, B, L und M wie in Tabelle 120 angegeben
X = CH₃; Z = H; V¹ = 4-OCH₃; V² = H.
- **Tabelle 139:**: A, B, L und M wie in Tabelle 120 angegeben
X = Cl; Z = H; V¹ = 4-OCH₃; V² = H.
- **Tabelle 140:**: A, B, L und M wie in Tabelle 120 angegeben
X = CH₃; Z = CH₃; V¹ = 4-OCH₃; V² = H.

Im einzelnen seien außer den bei den Herstellungsbeispielen genannten Verbindungen die folgenden Verbindungen der Formel (1-8) genannt:

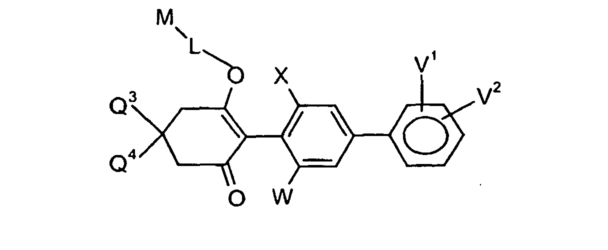

**Tabelle 141:**

| L - M = CH₂-O-C₂H₅ X = CH₃, W = CH₃, V¹ = H, V² = H. | |
|---|---|
| Q³ | Q⁴ |
| H | H |
| CH₃ H | |
| C₂H₅ | H |
| C₃H₇ | H |
| i-C₃H₇ | H |
| CH₃ | CH₃ |
| C₂H₅ | CH₃ |
| C₃H₇ | CH₃ |
| i-C₃H₇ | CH₃ |
| -(CH2)4- | |
| -(CH₂)₅- | |
| -(CH₂)₂-O-(CH₂)₂- | |
| -CH₂-O-(CH₂)₃- | |
| -(CH₂)₂-CHCH₃-(CH₂)₂- | |
| (CH₂)₂-CHC₂H₅-(CH₂)₂- | |
| -(CH₂)₂-CHOCH₃-(CH₂)₂- | |
| -(CH₂)₂-CHOC₂H₅-(CH₂)₂- | |
| -(CH₂)₂-CHOC₃H₇-(CH₂)₂- | |

- **Tabelle 142:**: Q³, Q⁴, L und M wie in Tabelle 141 angegeben
X = C₂H₅; W = CH₃; V¹ = H; V² = H.
- **Tabelle 143:**: Q³, Q⁴, L und M wie in Tabelle 141 angegeben
X = C₂H₅; W = C₂H₅; V¹ = H; V² = H.
- **Tabelle 144:**: Q³, Q⁴, L und M wie in Tabelle 141 angegeben
X = CH₃; W = CH₃; V¹ = 4-Cl; V² = H.
- **Tabelle 145:**: Q³, Q⁴, L und M wie in Tabelle 141 angegeben
X = C₂H₅; W = CH₃; V¹ = 4-Cl; V² = H.
- **Tabelle 146:**: Q³, Q⁴, L und M wie in Tabelle 141 angegeben
X = C₂H₅; W = C₂H₅; V¹ = 4-Cl; V² = H.
- **Tabelle 147:**: Q³, Q⁴, L und M wie in Tabelle 141 angegeben
X = CH₃; W = CH₃; V¹ = 3-Cl; V² = H.
- **Tabelle 148:**: Q³, Q⁴, L und M wie in Tabelle 141 angegeben
X = C₂H₅; W = CH₃; V¹ = 3-Cl; V² = H.
- **Tabelle 149:**: Q³, Q⁴, L und M wie in Tabelle 141 angegeben
X = C₂H₅; W = C₂H₅; V¹ = 3-Cl; V² = H.
- **Tabelle 150:**: Q³, Q⁴, L und M wie in Tabelle 141 angegeben
X = CH₃; W= CH₃; V¹= 4-CF₃; V² = H.
- **Tabelle 151:**: Q³, Q⁴, L und M wie in Tabelle 141 angegeben
X = C₂H₅; W = CH₃; V¹ = 4-CF₃; V² = H.
- **Tabelle 152:**: Q³, Q⁴, L und M wie in Tabelle 141 angegeben
X = C₂H₅; W = C₂H₅; V¹ = 4-CF₃; V² = H.
- **Tabelle 153:**: Q³, Q⁴, L und M wie in Tabelle 141 angegeben
X = CH₃; W = CH₃; V¹ = 2-Cl; V² = 4-Cl.
- **Tabelle 154:**: Q³, Q⁴, L und M wie in Tabelle 141 angegeben
X = C₂H₅; W = CH₃; V¹ = 2-Cl; V² = 4-Cl.
- **Tabelle 155:**: Q³, Q⁴, L und M wie in Tabelle 141 angegeben
X = C₂H₅; W = C₂H₅; V¹ = 2-Cl; V² = 4-Cl.
- **Tabelle 156:**: Q³, Q⁴, L und M wie in Tabelle 141 angegeben
X = CH₃; W = CH₃; V¹ = 4-CH₃; V² = H.
- **Tabelle 157:**: Q³, Q⁴, L und M wie in Tabelle 141 angegeben
X = C₂H₅; W = CH₃; V¹ = 4-CH₃; V² = H.
- **Tabelle 158:**: Q³, Q⁴, L und M wie in Tabelle 141 angegeben
X = C₂H₅; W = C₂H₅; V¹ = 4-CH₃; V2 = H.
- **Tabelle 159:**: Q³, Q⁴, L und M wie in Tabelle 141 angegeben
X = CH₃; W= CH₃; V¹ = 4-OCH₃; V² = H.
- **Tabelle 160:**: Q³, Q⁴, L und M wie in Tabelle 141 angegeben
X = C₂H₅; W= CH₃; V¹ = 4-OCH₃; V² = H.
- **Tabelle 161:**: Q³, Q⁴, L und M wie in Tabelle 141 angegeben
X = C₂H₅; W = C₂H₅; V¹ = 4-OCH₃; V² = H.

Im einzelnen seien außer den bei den Herstellungsbeispielen genannten Verbindungen die folgenden Verbindungen der Formel (I-8) genannt:

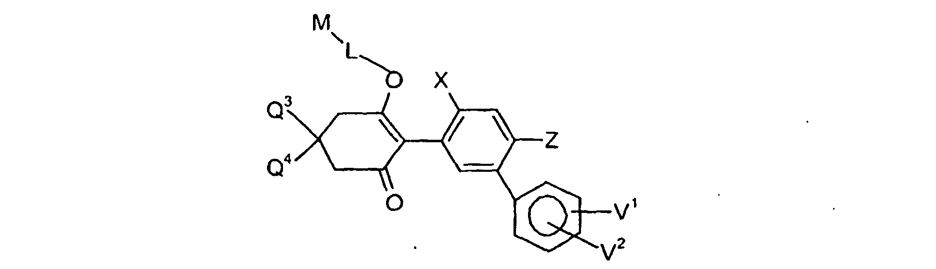

**Tabelle 162:**

| L - M = CH₂-O-C₂H₅ X=CH₃, Z=H, V¹=H, V²=H | |
|---|---|
| Q³ | Q⁴ |
| H | H |
| CH₃ | H |
| C₂H₅ | H |
| C₃H₇ | H |
| i-C₃H₇ | H |
| CH₃ | CH₃ |
| C₂H₅ | CH₃ |
| C₃H₇ | CH₃ |
| i-C₃H₇ | CH₃ |
| -(CH2)4- | |
| -(CH2)5- | |
| -(CH₂)₂-O-(CH₂)₂- | |
| -CH₂-O-(CH₂)₃- | |
| -(CH₂)₂-CHCH₃-(CH₂)₂- | |
| (CH₂)₂-CHC₂H₅-(CH₂)₂- | |
| -(CH₂)₂-CHOCH₃-(CH₂)₂- | |
| -(CH₂)₂-CHOC₂H₅-(CH₂)₂- | |
| -(CH₂)₂-CHOC₃H₇-(CH₂)₂- | |

- **Tabelle 163:**: Q³, Q⁴, L und M wie in Tabelle 162 angegeben
X=Cl; Z=H; V¹=H; V²=H.
- **Tabelle 164:**: Q³, Q⁴, L und M wie in Tabelle 162 angegeben X = CH₃; Z = CH₃; V¹ = H; V² =H.
- **Tabelle 165:**: Q³, Q⁴, L und M wie in Tabelle 162 angegeben
X = CH₃; Z = H; V¹ = 4-Cl; V² = H.
- **Tabelle 166:**: Q³, Q⁴, L und M wie in Tabelle 162 angegeben
X = Cl; Z = H; V¹ = 4-Cl; V² = H.
- **Tabelle 167:**: Q³, Q⁴, L und M wie in Tabelle 162 angegeben
X = CH₃; Z = CH₃; V¹ = 4-Cl; V² = H.
- **Tabelle 168:**: Q³, Q⁴, L und M wie in Tabelle 162 angegeben
X = CH₃; Z = H; V¹ = 3-Cl; V² = H.
- **Tabelle 169:**: Q³, Q⁴, L und M wie in Tabelle 162 angegeben
X = Cl; Z = H; V¹ = 3-Cl; V² = H.
- **Tabelle 170:**: Q³, Q⁴, L und M wie in Tabelle 162 angegeben
X = CH₃; Z = CH₃; V¹ = 3-Cl; V² = H.
- **Tabelle 171:**: Q³, Q⁴, L und M wie in Tabelle 162 angegeben X = CH₃; Z= H; V¹ = 4-CF₃; V² = H.
- **Tabelle 172:**: Q³, Q⁴, L und M wie in Tabelle 162 angegeben
X = Cl; Z = H; V¹ = 4-CF₃; V² = H.
- **Tabelle 173:**: Q³, Q⁴, L und M wie in Tabelle 162 angegeben
X = CH₃; Z = CH₃; V¹ = 4-CF₃; V² = H.
- **Tabelle 174:**: Q³, Q⁴, L und M wie in Tabelle 162 angegeben
X = CH₃; Z = H; V¹ = 2-Cl; V² = 4-Cl.
- **Tabelle 175:**: Q³, Q⁴, L und M wie in Tabelle 162 angegeben
X = Cl; Z = H; V¹ = 2-Cl; V² = 4-Cl.
- **Tabelle 176:**: Q³, Q⁴, L und M wie in Tabelle 162 angegeben
X = CH₃; Z = CH₃; V¹ = 2-Cl; V² = 4-Cl.
- **Tabelle 177:**: Q³, Q⁴, L und M wie in Tabelle 162 angegeben
X = CH₃; Z = H; V¹ = 4-CH₃; V² = H.
- **Tabelle 178:**: Q³, Q⁴, L und M wie in Tabelle 162 angegeben
X = Cl; Z = H; V¹ = 4-CH₃; V² = H.
- **Tabelle 179:**: Q³, Q⁴, L und M wie in Tabelle 162 angegeben
X = CH₃; Z = CH₃; V¹ = 4-CH₃; V² = H.
- **Tabelle 180:**: Q³, Q⁴, L und M wie in Tabelle 162 angegeben
X = CH₃; Z= H; V¹ = 4-OCH₃; V² = H.
- **Tabelle 181:**: Q³, Q⁴, L und M wie in Tabelle 162 angegeben
X = Cl; Z= H; V¹ = 4-OCH₃; V² = H.
- **Tabelle 182:**: Q³, Q⁴, L und M wie in Tabelle 162angegeben
X = CH₃; Z = CH₃; V¹ = 4-OCH₃; V² = H.

Verwendet man gemäß Verfahren (A) 3-[4-(4-Chlorphenyl)-2,6-dimethyl-phenyl]-4-hydroxy-1-isopropyl-Δ³-pyrrolin-2-on und N-Chlormethyl-N-methyl-carbaminsäureethylester, so kann der Verlauf des erfindungsgemäßen Verfahrens durch folgendes Reaktionsschema wiedergegeben werden:

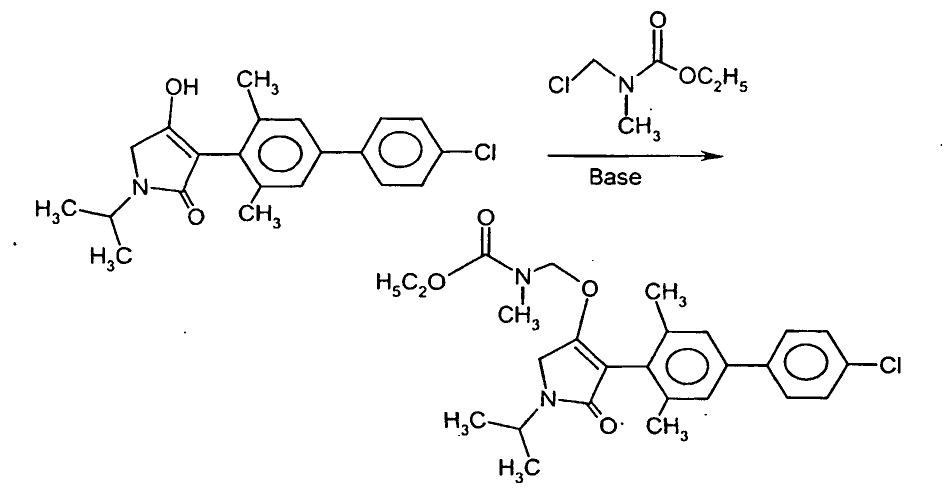

Die bei dem Verfahren (A) als Ausgangsstoffe benötigten Verbindungen der Formel (II-1) bis (II-8) 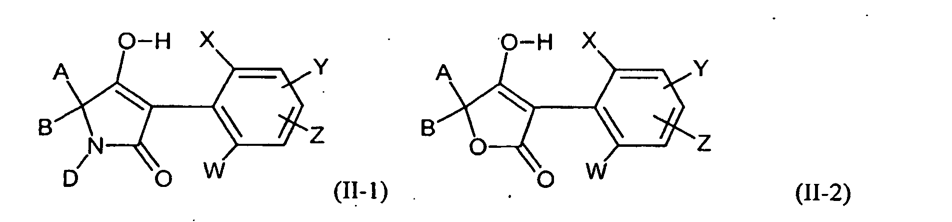 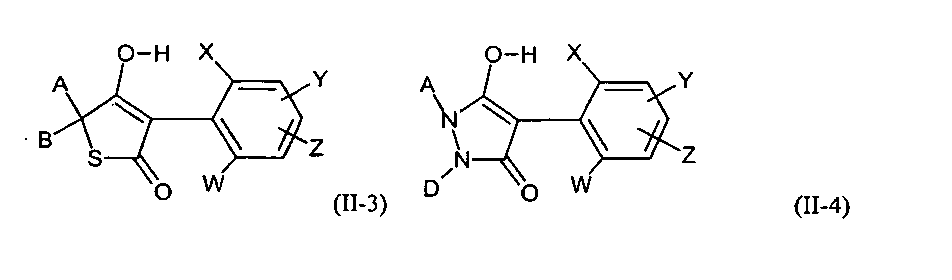 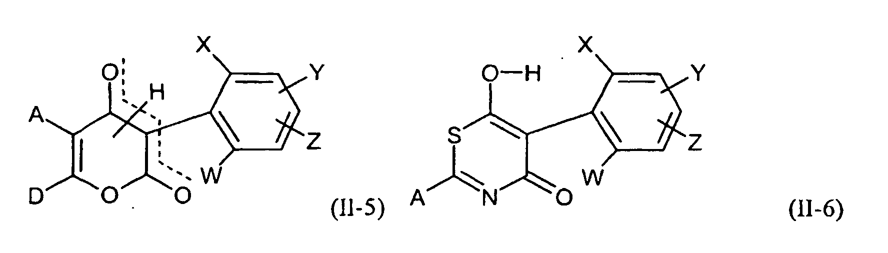 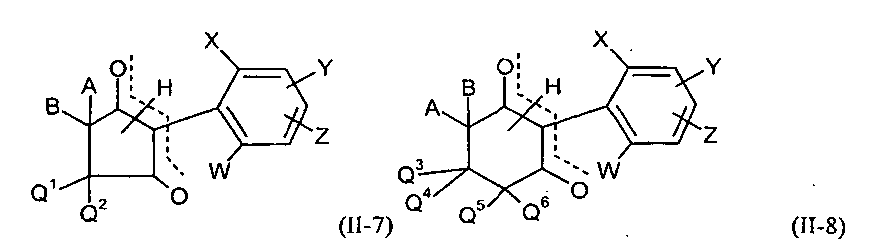 worin
A, B, D, L, M, Q¹, Q², Q³, Q⁴, Q⁵, Q⁶, W, X, Y und Z die oben angegebenen Bedeutungen haben,
sind bekannt aus noch nicht offengelegten Anmeldungen der Anmelderin (DE 19 808 261.4, DE 19813354.5 und DE 19818732.7)

Die zur Durchführung des erfindungsgemäßen Verfahrens (A) weiterhin als Ausgangsverbindungen benötigten Verbindungen der Formel (III) sind allgemein bekannte Verbindungen der organischen Chemie.

Als Verdünnungsmittel können für die Durchführung des erfindungsgemäßen Verfahrens (A) alle gegenüber diesen Verbindungen inerten Solventien eingesetzt werden. Vorzugsweise verwendbar sind Kohlenwasserstoffe, wie Benzin, Benzol, Toluol, Xylol und Tetralin; ferner Halogenkohlenwasserstoffe, wie Methylenchlorid, Chloroform, Tetrachlorkohlenstoff, Chlorbenzol und o-Dichlorbenzol; Ether, wie Diethylether, Tetrahydrofuran und Dioxane; Nitrile, wie Acetonitril oder Propionitril; ferner polare, gegenüber den Verbindungen der Formel (III) inerte Lösungsmittel, wie Dimethylsulfoxid, Dimethylformamid, Sulfolan oder N-Methylpyrrolidon aber auch Ester wie Essigsäureethylester.

Als Basen können bei der Durchführung des erfindungsgemäßen Verfahrens (A) alle üblichen Säureakzeptoren eingesetzt werden. Vorzugsweise verwendbar sind Alkalimetall- und Erdalkalimetall-oxide, -hydroxide und-carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren, wie z.B. Triethylbenzylammoniumchlorid, Tetrabutylammoniumbromid, Adogen 464^{*)} oder TDA 1^{***)} eingesetzt werden können. Ferner sind Alkalimetall- und Erdalkalimetall-hydride, wie Natriumamid, Natriumhydrid und Calciumhydrid, und außerdem auch Alkalimetallalkoholate, wie Natriummethylat, Natriumethylat und Kalium-tert.-butylat einsetztbar. Weiterhin tertiäre Amine, wie Triethylamin, Pyridin, DABCO, DBU, DBA, Hünig-Base und N,N-Dimethylanilin.
*) Adogen 464 = Methyltrialkyl(C₈-C₁₀)ammoniumchlorid
***) TDA 1 = Tris-(methoxyethoxyethyl)-amin

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen -20°C und 180°C, vorzugsweise zwischen 0°C und 130°C.

Bei der Durchführung des erfindungsgemäßen Verfahrens (A) setzt man die Reaktionskomponenten (II-1) bis (II-8) und (III) und die Base im allgemeinen in etwa äquimolaren Mengen ein. Es ist jedoch auch möglich, die eine oder andere Komponente in einem größeren Überschuß (bis zu 3 Mol) zu verwenden.

Das erfindungsgemäße Verfahren (A) wird im allgemeinen unter Normaldurck durchgeführt. Die Aufarbeitung erfolgt nach üblichen Methoden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spp..

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Acheta domesticus, Gryllotalpa spp., Locusta migratoria migratorioides, Melanoplus spp., Schistocerca gregaria.

Aus der Ordnung der Blattaria z.B. Blatta orientalis, Periplaneta americana, Leucophaea maderae, Blattella germanica.

Aus der Ordnung der Dermaptera z.B. Forficula auricularia.

Aus der Ordnung der Isoptera z.B. Reticulitermes spp..

Aus der Ordnung der Phthiraptera z.B. Pediculus humanus corporis, Haematopinus spp., Linognathus spp., Trichodectes spp., Damalinia spp..

Aus der Ordnung der Thysanoptera z.B. Hercinothrips femoralis, Thrips tabaci, Thrips palmi, Frankliniella occidentalis.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp.

Aus der Ordnung der Siphonaptera z.B. Xenopsylla cheopis, Ceratophyllus spp.

Aus der Klasse der Arachnida z.B. Scorpio maurus, Latrodectus mactans, Acarus siro, Argas spp., Omithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp.

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp.

Die erfindungsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-Imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims; Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat, CalciumpoIysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-b-propyl-1H-1,2,4-triazol-1-ethanol,
α-(2,4-Dichlorphenyl)-β-methoxy-a-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS, 6RS)-6-Hydroxy-2,2,7,7-tetramethyl-5-(1H-1,2,4-triazol-1-yl)-3-octanon,
(E)-α-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-triluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl)-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl)-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat,
cis- 1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichior-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3 -pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioat,
S-Methyl-1,2,3-benzothiadiazol-7-carbothioat,
spiro [2H]-1-Benzopyran-2,1'(3'H)-isobenzofuran]-3'-on,

### Bakterizide

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazine, Deltamethrin, Demeton M, Demeton S, Demeton-S-methyl, Diafenthiuron, Diazinon, Dichlorvos, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Esfenvalerate, Ethiofencarb, Ethion, Ethoprophos, Etofenprox, Etoxazole, Etrimfos, Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
Granuloseviren
Halofenozide, HCH, Heptenophos, Hexaflumuron, Hexythiazox, Hydroprene,
Imidacloprid, Isazofos, Isofenphos, Isoxathion, Ivermectin,
Kempolyederviren
Lambda-cyhalothrin, Lufenuron
Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos,
Naled, Nitenpyram, Nithiazine, Novaluron
Omethoat, Oxamyl, Oxydemethon M
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propoxur, Prothiofos, Prothoat, Pymetrozine, Pyraclofos, Pyresmethrin, Pyrethrum, Pyridaben, Pyridathion, Pyrimidifen, Pyriproxyfen,
Quinalphos,
Ribavirin
Salithion, Sebufos, Silafluofen, Spinosad, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Thetacypermethrin, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanii
YI 5302
Zeta-cypermethrin, Zolaprofos
(1 R-cis)-[5-(Phenylmethyl)-3-furanyl]-methyl-3-[(dihydro-2-oxo-3(2H)-furanyliden)-methyl]-2,2-dimethylcyclopropancarboxylat
(3-Phenoxyphenyl)-methyl-2,2,3,3-tetramethylcyclopropanecarboxylat
1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol
2-(Acetyloxy)-3-dodecyl-1,4-naphthalindion
2-Chlor-N-[[[4-(1-phenylethoxy)-phenyl]-amino]-carbonyl]-benzamid
2-Chlor-N-[[[4-(2,2-dichlor-1,1-difluorethoxy)-phenyl]-amino]-carbonyl]-benzamid
3-Methylphenyl-propylcarbamat
4-[4-(4-Ethoxyphenyl)-4-methylpentyl]-1-fluor-2-phenoxy-benzol
4-Chlor-2-(1,1-dimethylethyl)-5-[[2-(2,6-dimethyl-4-phenoxyphenoxy)ethyl]thio]-3(2H)-pyridazinon
4-Chlor-2-(2-chlor-2-methylpropyl)-5-[(6-iod-3-pyridinyl)methoxy]-3(2H)-pyridazinon
4-Chlor-5-[(6-chlor-3-pyridinyl)methoxy]-2-(3,4-dichlorphenyl)-3(2H)-pyridazinon
Bacillus thuringiensis strain EG-2348
Benzoesäure [2-benzoyl-1-(1,1-dimethylethyl)-hydrazid
Butansäure 2,2-dimethyl-3-(2,4-dichlorphenyl)-2-oxo-1-oxaspiro[4.5]dec-3-en-4-yl-ester
[3-[(6-Chlor-3-pyridinyl)methyl]-2-thiazolidinyliden]-cyanamid
Dihydro-2-(nitromethylen)-2H-1,3-thiazine-3(4H)-carboxaldehyd
Ethyl-[2-[[1,6-dihydro-6-oxo-1-(phenylmethyl)-4-pyridazinyl]oxy]ethyl]-carbamat
N-(3,4,4-Trifluor-1-oxo-3-butenyl)-glycin
N-(4-Chlorphenyl)-3-[4-(difluormethoxy)phenyl]-4,5-dihydro-4-phenyl-1H-pyrazol-1-carboxamid
N-[(2-Chlor-5-thiazolyl)methyl]-N'-methyl-N"-nitro-guanidin
N-Methyl-N'-(1-methyl-2-propenyl)-1,2-hydrazindicarbothioamid
N-Methyl-N'-2-propenyl-1,2-hydrazindicarbothioamid
O,O-Diethyl-[2-(dipropylamino)-2-oxoethyl]-ethylphosphoramidothioat

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp.
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.
Aus der Ordnung der Siphonapterida z.B. Pulex spp., Ctenocephalides spp., Xenopsylla spp., Ceratophyllus spp.
Aus der Ordnung der Heteropterida z.B. Cimex spp., Triatoma spp., Rhodnius spp., Panstrongylus spp.
Aus der Ordnung der Blattarida z.B. Blatta orientalis, Periplaneta americana, Blattela germanica, Supella spp.
Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.
Aus der Ordnung der Actinedida (Prostigmata) und Acaridida (Astigmata) z.B. Acarapis spp., Cheyletiella spp., Omithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die erfindungsgemäßen Wirkstoffe eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec., Tryptodendron spec., Apate monachus, Bostrychus capucins, Heterobostrychus brunneus, Sinoxylon spec., Dinoderus minutus.
Hautflügler wie
   Sirex juvencus, Urocerus gigas, Urocerus gigas taignus, Urocerus augur.
Termiten wie
   Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze wie Lepisma saccharina.

Unter technischen Materialien sind im vorliegenden Zusammenhang nicht-lebende Materialien zu werstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel-(gemisch) oder ein Weichmacher(gemisch) ersetzt werden. Diese Zusätze sollen einer Verflüchtigung der Wirkstoffe sowie einer Kristallisation bzw. einem Ausfällen vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermotekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten:

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid und Triflumuron,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflußkrebse) zusammengefaßt werden, besondere Bedeutung zu.

Es wurde nun überraschenderweise gefunden, daß die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis-(trialkylzinn)-sulfiden, Tri-n-butylzinnlaurat, Tri-n-butylzinnchlorid, Kupfer(I)-oxid, Triethylzinnchlorid, Tri-n-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-n-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:
Algizide wie
   2-tert-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;
Fungizide wie
   Benzo[b]thiophencarbonsäurecyclohexylamid-S,S-dioxid, Dichlofluanid, Fluorfolpet, 3-Iod-2-propinyl-butylcarbamat, Tolylfluanid und Azole wie
   Azaconazole, Cyproconazole, Epoxyconazole, Hexaconazole, Metconazole, Propiconazole und Tebuconazole;
   Molluskizide wie
   Fentinacetat, Metaldehyd, Methiocarb, Niclosamid, Thiodicarb und Trimethacarb;
   oder herkömmliche Antifouling-Wirkstoffe wie
   4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, Chem. Ind. 1985, 37, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wäßrigen System, Vinylchlorid/Vinylacetat-Copolymersysteme in Form wäßriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp.
Aus der Ordnung der Chilopoda z.B. Geophilus spp.
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindemia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf- Verfahren.

Die erfindungsgemäßen Wirkstoffe können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den' Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mittel, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise Acetochlor, Acifluorfen (-sodium), Aclonifen, Alachlor, Alloxydim (-sodium), Ametryne, Amicarbazone, Amidochlor, Amidosulfuron, Anilofos, Asulam, Atrazine, Azafenidin, Azimsulfuron, BAS-662H, Beflubutamid, Benazolin (-ethyl), Benfuresate, Bensulfuron (-methyl), Bentazon, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Picolinafen, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Tralkoxydim, Triallate, Triasulfuron, Tribenuron (-methyl), Triclopyr, Tridiphane, Trifluralin, Trifloxysulfuron, Triflusulfuron (-methyl), Tritosulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele

### Beispiel I-1-1

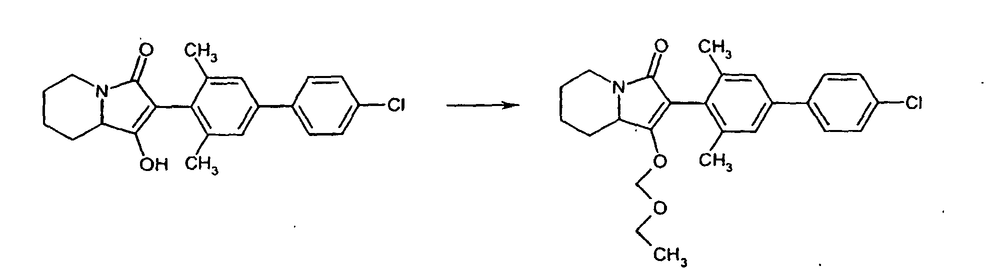

### Beispiel II-1

Zu einer Lösung der Verbindung gemäß Formel II-1 (bekannt aus DE 19 808 261.4) (1,1 g) in 30 ml absolutem Essigsäureethylester gibt man bei 0°C 0,29 g Chlormethylethylether in 5 ml absolutem Essigsäureethylester gelöst hinzu, rührt bei Raumtemperatur unter DC-Kontrolle, engt ein und chromatographiert an Kieselgel (n-Hexan : Essigsäureethylester 2: 1). Man isoliert 0,98 g (77 %) der oben gezeigten Verbindung I-1-1 (Feststoff, Fp.: 169°C).

In Analogie zu Beispiel I-1-1 und gemäß den allgemeinen Angaben zur Herstellung von Verbindungen der Formel I-1 wurden folgende Verbindungen hergestellt.

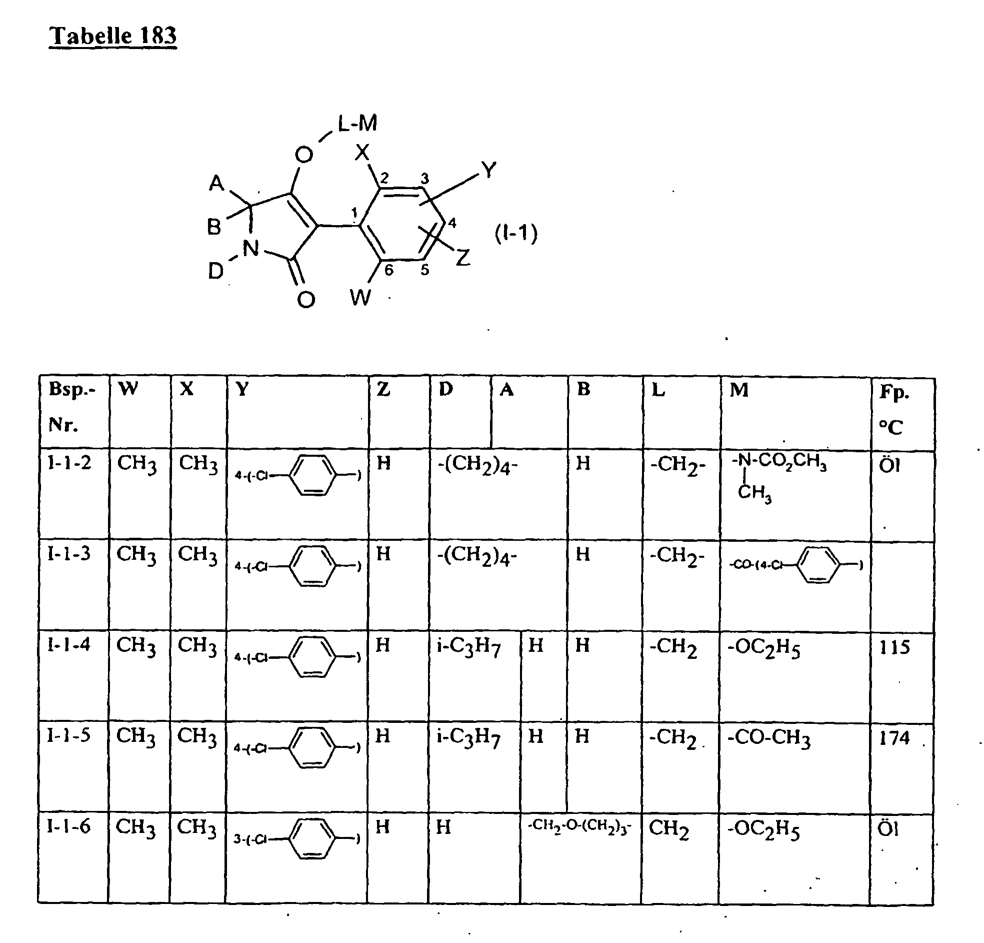

### Beispiel I-2-1

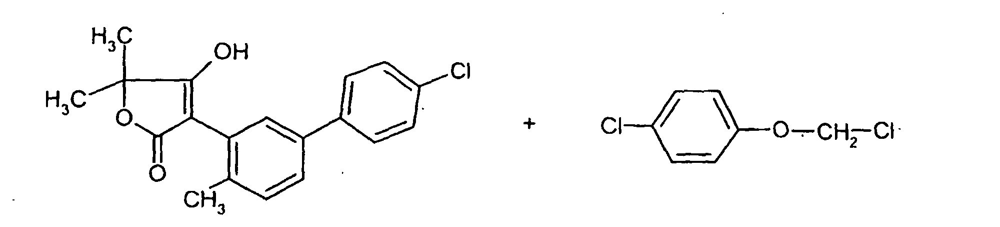

### Beispiel II-2

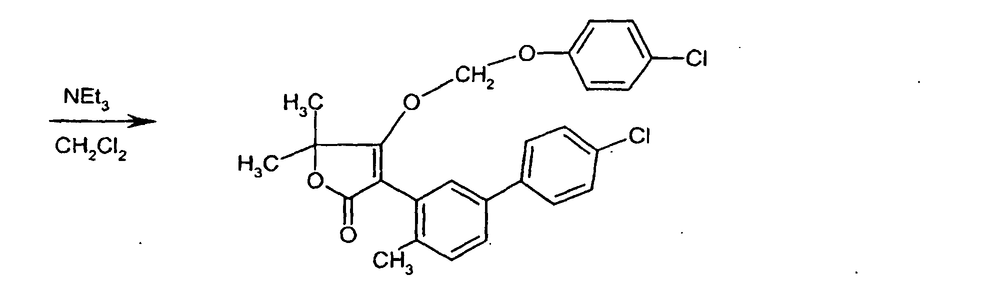

0,98 g der Verbindung gemäß Formel II-2 (bekannt aus DE 19 813 354.5) wurden in 20 ml wasserfreiem Methylenchlorid mit 0,4 g Triethylamin versetzt und bei 0-10°C. Chlormethyl-(4-chlorphenol)-ether zugetropft. Nach Rühren über Nacht wird mit 10 %iger Zitronensäure und anschließend mit 10 %iger Natronlauge gewaschen, getrocknet und das Lösungsmittel im Vakuum abgedampft und der Rückstand mit Methylenchlorid als Laufmittel an Kieselgel chromatographiert. Man isoliert 0,39 g (≙ 21 % der Theorie) der oben gezeigten Verbindung I-2-1.
¹H-NMR (300 MHz, CDCl₃):δ = 1.48 (s, 3H, CH₃), 1.52 (s, 3H, CH₃), 2.25 (s, 3H, Ar-CH₃), 5.5 (m, 2H, O-CH₂-O), 6.9 ("d", 2H, ArH), 7.25-7.65 (m, 9H, ArH) ppm.

In Analogie zu Beispiel I-2-1 und gemäß den allgemeinen Angaben zur Herstellung von Verbindungen der Formel 1-2 wurden folgende Verbindungen hergestellt.

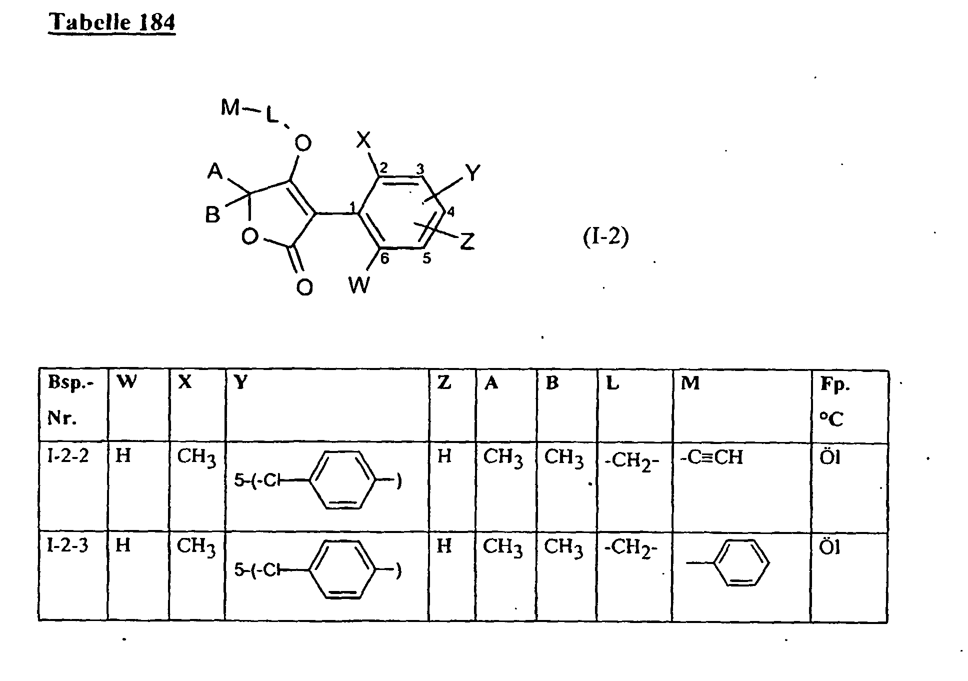

### Beispiel I-7-1

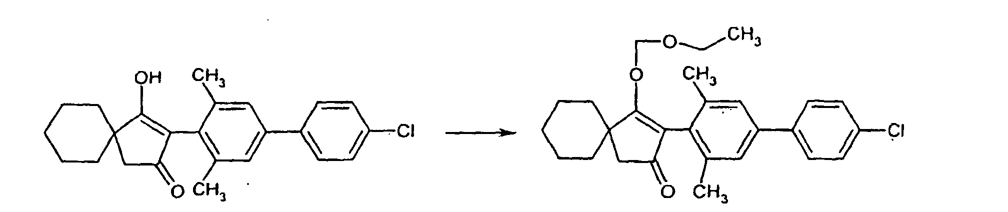

### Beispiel II-7

Zu einer Lösung der Verbindung gemäß Formel II-7 (bekannt aus DE 19 808 261.4) (1,0 g; 2,6 mmol) in 10 ml Essigsäureethylester und Triethylamin (0,36 ml; 2,6 mmol) gibt man bei 0°C 0,24 ml Chlormethylethylether in 3 ml Essigsäureethylether gelöst hinzu und rührt 8 h bei Raumtemperatur. Der Niederschlag wird abfiltriert und mit Essigsäureethylester gewaschen, anschließend wäscht man das Filtrat mit Natriumchloridlösung, trocknet und engt ein.
Man isoliert 1 g (88 %) der oben gezeigten Verbindung 1-7-1 (Öl).
¹H-NMR (400 MHz, d₆-DMSO) δ = 1,15-1.85 (m, 10H, (CH₂)₅), 2.09, 2.12 (2s, 6H, CH₃), 3.57 (q; 2H, O-CH₂CH₃); 5.32 (s, 2H, O-CH₂-O) 7.48 ("d"(AA', BB'), 2H, Ar-H), 7.67 ("d"(AA'BB'), 2H, Ar-H) ppm.

In Analogie zu Beispiel 1-7-1 und gemäß den allgemeinen Angaben zur Herstellung von Verbindungen der Formel 1-7 wurden folgende Verbindungen hergestellt.

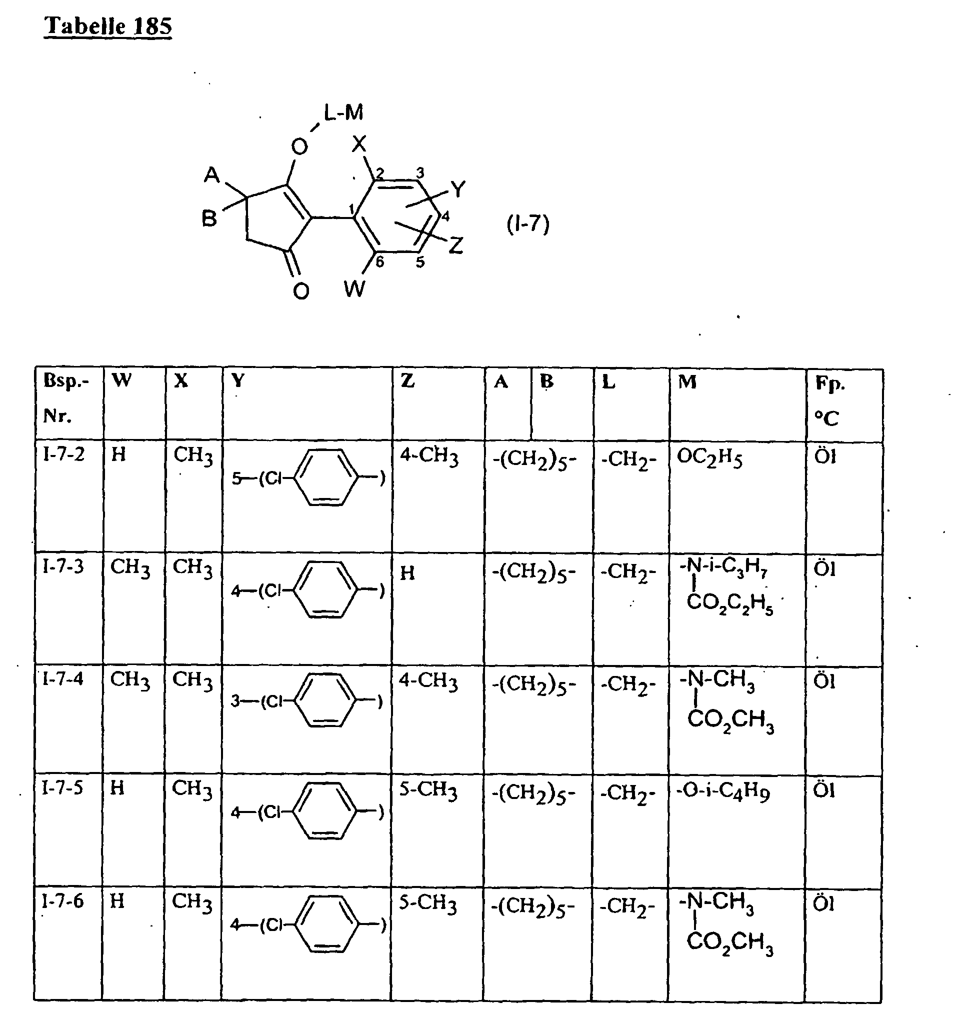

### Beispiel I-8-1

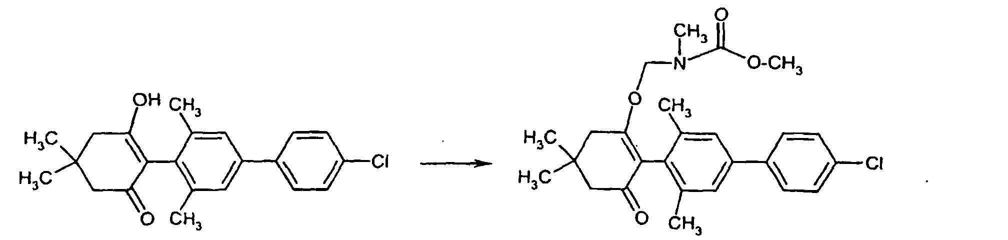

### Beispiel II-8

Zu einer Lösung der Verbindung gemäß Formel II-8 (bekannt aus DE 198 08261.4) (1,0 g; 2,8 mmol) in 10 ml Dichlormethan und Triethylamin (0,58 ml; 4,2 mmol) gibt man bei 0°C (0,58 g; 4,2 mmol) N-Chlormethyl-N-methyl-methylcarbamat in 3 ml Dichlormethan gelöst hinzu und rührt 8 h bei Raumtemperatur. Die Reaktionslösung wird mit 10 %iger Zitronensäure gewaschen und mit Dichlormethan extrahiert. Die organische Phase wäscht man mit 1 N Natriumhydroxidlösung, extrahiert mit Dichlormethan und trocknet und engt die organische Phase ein.
Man isoliert 1,15 g (90 %) der oben gezeigten Verbindung I-8-1 (Öl).
¹H-NMR (400 MHz, d₆-DMSO): δ = 1.16 (s, 6H, C(CH₃)₂); 2.04 (s, 6H, ArCH₃), 3.6 (s, 3H, OCH₃), 5.16 (s, 2H, O-CH₂-N 〈), 7.3 (s, 2H, ArH), 7.48 ("d" (AA'BB'), 2H, ArH), 7.67 ("d"(AA'BB'), 2H, Ar-H)ppm.

In Analogie zu Beispiel (I-8-1) und gemäß der allgemeinen Angaben zur Herstellung von Verbindungen der Formel (1-8) wurden folgende Verbindungen hergestellt:

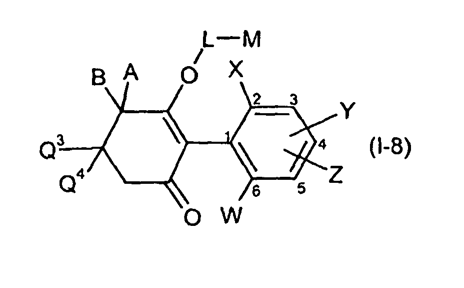

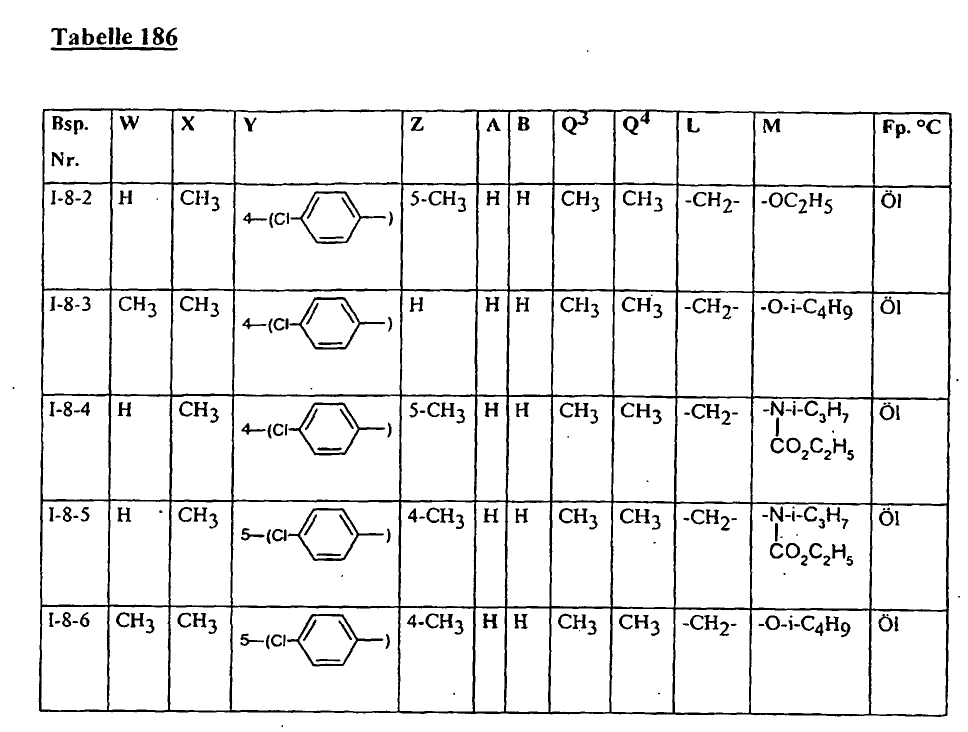

### Anwendungsbeispiel

### Beispiel 1

### Phaedon-Larven-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Larven des Meerrettichkäfers (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käferlarven abgetötet wurden.

Bei diesem Test zeigen z.B. die folgenden Verbindungen der Herstellungsbeispiele gute Wirksamkeit: I-7-2, I-8-2, I-8-1, I-8-5

### Beispiel 2

### Spodoptera frugiperda-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele gute Wirksamkeit: I-8-5, I-8-6

### Beispiel 3

### Tetranychus-Test (OP-resistent/Tauchbehandlung)

- Lösungsmittel:: 7 Gewichtsteile Dimethylformamid
- Emulgator:: 1 Gewichtsteil Alkylarylpolyglygolether

Zur Herstellung eineer zweckmäßigen Wirkstoffzubereitung vermischt man 1 Gewichtsteil Wirkstoffmit den angegebenen Mengen Lösungsmittel und Emulgator und verdünnt das Konzentrat mit emulgatorhaltigem Wasser auf die gewünschte Konzentration.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Wirkung in % bstimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele gute Wirksamkeit: I-7-2, I-8-5, I-7-4

### Beispiel 4

### Meloidogyne-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Gefäße werden mit Sand, Wirkstofflösung, Meloidogyne incognita-Ei-Larvensuspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, daß keine Gallen gefunden wurden; .0 % bedeutet, daß die Zahl der Gallen an den behandelten Pflanzen der der unbehandelten Kontrolle entspricht.

Nach der gewünschten Zeit wird die Abtötung in % bestimmt. Dabei bdeutet 100 %, daß alle Tiere abgetötet wurden; 0 % bedeutet, daß keine Tiere abgetötet wurden.

Bei diesem Test zeigen z.B. die folgenden Verbindungen der Herstellungsbeispiele gute Wirksamkeit: I-7-3, I-8-1, I-8-5, I-7-4

### Beispiel 5

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalem Boden ausgesät. Nach ca. 24 Stunden wird der Boden mit der Wirkstoffzubereitung so bespritzt, daß die jeweils gewünschten Wirkstoffmengen pro Flächeneinheit ausgebracht werden. Die Konzentration der Spritzbrühe wird so gewählt, daß in 1000 1 Wasser/ha die jeweils gewünschten Wirkstoffmengen ausgebracht werden. Nach drei Wochen wird der Schädigungsgrad der Pflanzen bonitiert in %Schädigung im Vergleich zur Entwicklung der unbehandelten Kontrolle.

Es bedeuten:
- 0 % =: keine Wirkung (wie unbehandelte Kontrolle)
- 100 % =: totale Vernichtung

### Pre-emergence/Gewächshaus

| | g ai./ha | Acenafatua | Echinochloa | Setaria | Sinapis |
|---|---|---|---|---|---|
| Bsp. I-7-3 | 250 | 95 | 100 | 100 | 70 |

| | g ai./ha | Alopecurus | Avenafatua | Echinochloa | Setaria | Abutilon |
|---|---|---|---|---|---|---|
| Bsp. I-8-1 | 250 | 90 | 90 | 100 | 100 | 80 |

### Beispiel 6

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
- 0 % =: keine Wirkung (wie unbehandelte Kontrolle)
- 100 % =: totale Vernichtung

### Post-emergence/Gewächshaus

| | g ai./ha | Avenafatua | Echinochloa | Setaria | Sinapis |
|---|---|---|---|---|---|
| Bsp. I-7-3 | 250 | 80 | 100 | 100 | 80 |

| | g ai./ha | Zuckerrübe | Alopecurus | Echinochloa | Setaria |
|---|---|---|---|---|---|
| Bsp. I-8-1 | 250 | 0 | 80 | 100 | 100 |

### Beispiel 7

### Schabentest

- Testtiere:: Blattella germanica oder Periplaneta americana
- Lösungsmittel:: Dimethylsulfoxid

20 mg Wirkstoff werden in 1 ml Dimethylsulfoxid gelöst, geringere Konzentrationen werden durch verdünnen mit dest. H₂O hergestellt.

5 ml dieser Wirkstoffzubereitung werden auf Back-Oblaten (Ø 9,5 cm) pipettiert, die sich in Petrischalen entsprechender Größe befinden. Nach Trocknung der Back-Oblaten werden 4 Testtiere überführt und abgedeckt.

Nach 7 Tagen Aufbewahrung in einem klimatisierten Raum wird der Abtötungsgrad bestimmt:

Dabei bedeutet 100 %, daß alle Schaben abgetötet wurden, 0 % bedeutet, daß keine Schaben abgetötet wurden.

Bei diesem Test zeigen z.B. die folgenden Verbindungen der Herstellungsbeispiele 1-7-2 und 1-8-5 bei einer beispielhaften Wirkstoffkonzentration von 100 ppm eine Abtötung von 100 %.

### Beispiel 8

### Schabentest

- Testtiere:: Blattella germanica oder Periplaneta americana
- Lösungsmittel:: Dimethylsulfoxid

20 mg Wirkstoff werden in 1 ml Dimethylsulfoxid gelöst, geringere Konzentrationen werden durch verdünnen mit dest. H₂O hergestellt.

4 Testtiere werden in die zu testende Wirkstoffzubereitung 1 Minute getaucht. Nach Überführung in Plastikbecher und 7 Tagen Aufbewahrung in einem klimatisierten Raum wird der Abtötungsgrad bestimmt.

Dabei bedeutet 100 %, daß alle Schaben abgetötet wurden, 0 % bedeutet, daß keine Schaben abgetötet wurden.

Bei diesem Test zeigen z.B. die folgenden Verbindungen der Herstellungsbeispiele 1-7-2 und 1-8-5 bei einer beispielhaften Wirkstoffkonzentration von 100 ppm eine Abtötung von 100 % (1-7-2) bzw. 75 % (I-8-5).

## Patentansprüche

1. Verbindungen der Formel (I) 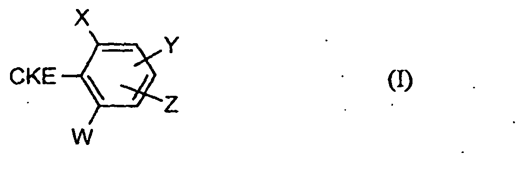 in welcher
W für Wasserstoff, Halogen, C₁-C₆-Alkyl, C₁-C₆-Alkoxy, C₁-C₆-Halogenalkyl, C₁-C₆-Halogenalkoxy, Nitro oder Cyano steht,
X für Halogen, C₁-C₆-Alkyl, C₁-C₆-Halogenalkyl, C₁-C₆-Alkoxy, C₃-C₆-Alkenyloxy, C₁-C₆-Alkylthio, C₁-C₆-Alkylsulfinyl, C₁-C₆-Alkylsulfonyl, C₁-C₆-Halogenalkoxy, C₃-C₆-Halogenalkenyloxy, Nitro, Cyano oder jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenylthio, Benzyloxy oder Benzylthio steht,
Y für einen der Reste 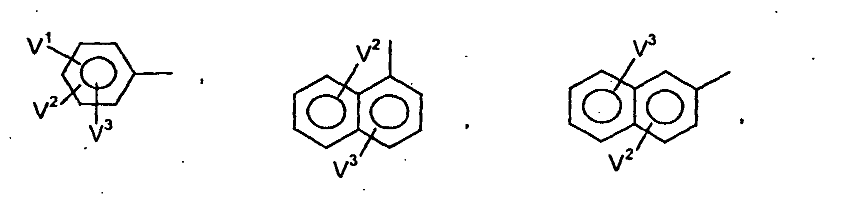 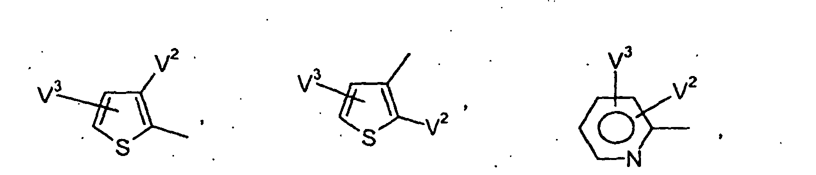 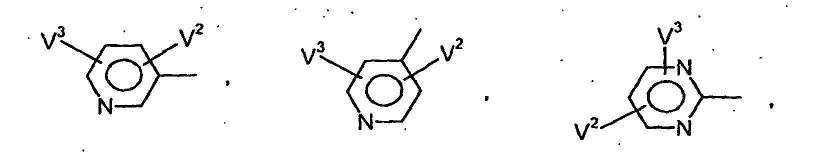 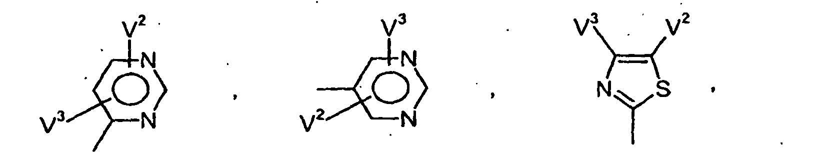 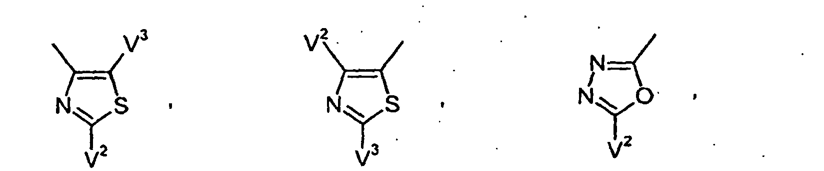 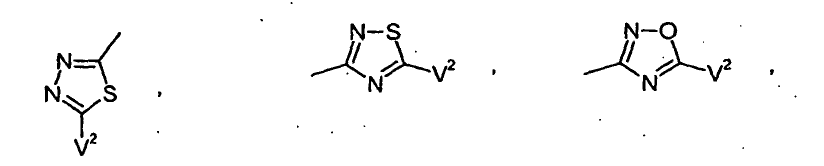 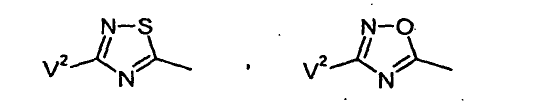 steht,
V¹ für Wasserstoff, Halogen, C₁-C₁₂-Alkyl, C₁-C₆-Alkoxy, C₁-C₄-Halogenalkyl, C₁-C₄-Halogenalkoxy, Nitro, Cyano oder jeweils gegebenenfalls einfach oder mehrfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenoxy-C₁-C₄-alkyl, Phenyl-C₁-C₄-alkoxy, Phenylthio-C₁-C₄-alkyl oder Phenyl-C₁-C₄-alkylthio steht,
V² und V³ unabhängig voneinander für Wasserstoff, Halogen, C₁-C₆-Alkyl, C₁-C₆-Alkoxy, C₁-C₄-Halogenalkyl oder C₁-C₄-Halogenalkoxy stehen,
Z für Wasserstoff, Halogen, C₁-C₆-Alkyl, C₁-C₆-Halogenalkyl, C₁-C₆-Alkoxy oder C₁-C₆-Halogenalkoxy steht,
CKE für eine der Gruppen 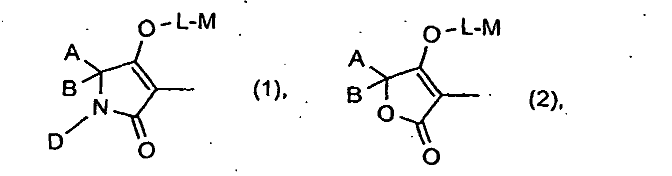 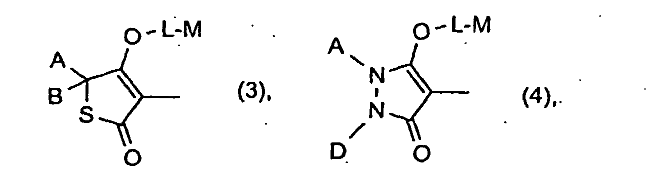 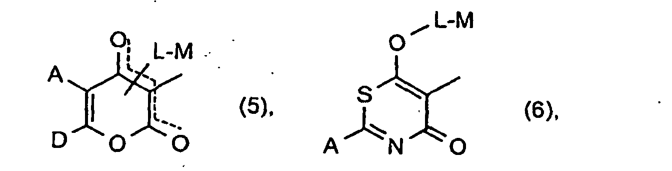 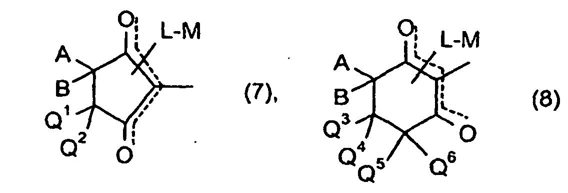 steht,
A für Wasserstoff oder jeweils gegebenenfalls durch Halogen substituiertes C₁-C₁₂-Alkyl, C₃-C₈-Alkenyl, C₁-C₁₀-Alkoxy-C₁-C₈-alkyl, Di-, tri- oder tetra-C₁-C₈-alkoxy-C₁-C₈-alkyl, C₁-C₁₀-Alkylthio-C₁-C₆-alkyl, gegebenenfalls durch Halogen, C₁-C₆-Alkyl oder C₁-C₆-Alkoxy substituiertes C₃-C₈-Cycloalkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes C₆- oder C₁₀-Aryl, Hetaryl mit 5 bis 6 Ringatomen oder C₆- oder C ₁₀-Aryl-C₁-C₆-alkyl steht,
B für Wasserstoff, C₁-C₁₂-Alkyl oder C₁-C₈-Alkoxy-C₁-C₆-alkyl steht, oder
A, B und das Kohlenstoffatom an das sie gebunden sind, für gesättigtes C₃-C₁₀-Cycloalkyl oder ungesättigtes C₅-C₁₀-Cycloakyl stehen, worin gegebenenfalls ein Ringglied durch Sauerstoff oder Schwefel ersetzt ist und welche gegebenenfalls einfach oder zweifach durch C₁-C₈-Alkyl, C₃-C₁₀-Cycloalkyl, C₁-C₈-Halogenalkyl, C₁-C₈-Alkoxy, C₁-C₈-Alkylthio, Halogen oder Phenyl substituiert sind oder
A, B und das Kohlenstoffatom, an das sie gebunden sind, für C₃-C₆-Cycloalkyl stehen, welches durch eine gegebenenfalls ein oder zwei nicht direkt benachbarte Sauerstoff- und/oder Schwefelatome enthaltende Alkylendiyl-, oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Ring bildet oder
A, B und das Kohlenstoffatom, an das sie gebunden sind, für C₃-C₈-Cycloalkyl oder C₅-C₈-Cycloalkenyl stehen, in welchen zwei Substituenten gemeinsam mit den Kohlenstoffatomen, an die sie gebunden sind, für jeweils gegebenenfalls durch C₁-C₆-Alkyl, C₁-C₆-Alkoxy oder Halogen substituiertes C₂-C₆-Alkandiyl, C₂-C₆-Alkendiyl oder C₄-C₆-Alkandiendiyl stehen, worin gegebenenfalls eine Methylengruppe durch Sauerstoff oder Schwefel ersetzt ist,
D für Wasserstoff, jeweils gegebenenfalls durch Halogen substituiertes C₁-C₁₂-Alkyl, C₃-C₈-Alkenyl, C₃-C₈-Alkinyl, C₁-C₁₀-Alkoxy-C₂-C₈-alkyl, Poly-C₁-C₈-alkoxy-C₂-C₈-alkyl, C₁-C₁₀-Alkylthio-C₂-C₈-alkyl, gegebenenfalls durch Halogen, C₁-C₄-Alkyl, C₁-C₄-Alkoxy oder C₁-C₄-Halogenalkyl substituiertes C₃-C₈-Cycloalkyl, in welchem gegebenenfalls ein Ringglied durch Sauerstoff oder Schwefel ersetzt ist oder jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Hetaryl mit 5 oder 6 Ringatomen, Phenyl-C₁-C₆-alkyl oder Hetaryl-C₁-C₆-alkyl mit 5 oder 6 steht, oder
A und D gemeinsam für jeweils gegebenenfalls substituiertes C₃-C₆-Alkandiyl oder C₃-C₆-Alkendiyl stehen, worin gegebenenfalls eine Methylengruppe durch Sauerstoff oder Schwefel ersetzt ist und wobei als Substituenten jeweils in Frage kommen:
Halogen, Hydroxy, Mercapto oder jeweils gegebenenfalls durch Halogen substituiertes C₁-C₁₀-Alkyl, C₁-C₆-Alkoxy, C₁-C₆-Alkylthio, C₃-C₇-Cycloalkyl, Phenyl oder Benzyloxy, oder eine weitere C₃-C₆-Alkandiylgruppierung, C₃-C₆-Alkendiylgruppierung oder eine Butadienylgruppierung, die gegebenenfalls durch C₁-C₆-Alkyl substituiert ist oder in der gegebenenfalls zwei benachbarte Substituenten mit den Kohlenstoffatomen, an die sie gebunden sind, einen weiteren gesättigten oder ungesättigten Cyclus mit 5 oder 6 Ringatomen bilden (im Fall der Verbindung der Formel (I-1) stehen A und D dann gemeinsam mit den Atomen, an die sie gebunden sind beispielsweise für die weiter unten genannten Gruppen AD-1 bis AD-10), der Sauerstoff oder Schwefel enthalten kann, oder worin gegebenenfalls eine der folgenden Gruppen 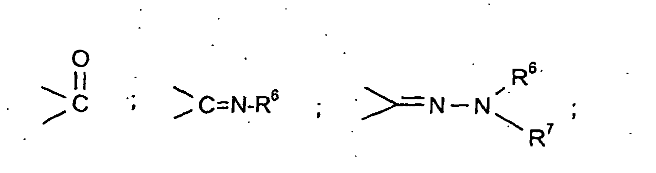 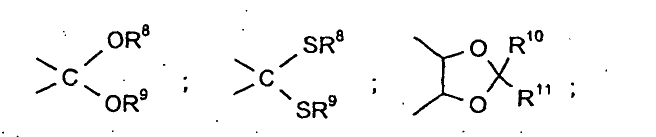 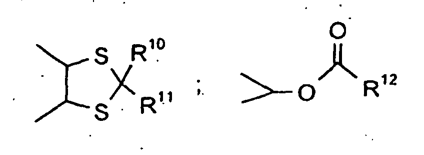 oder 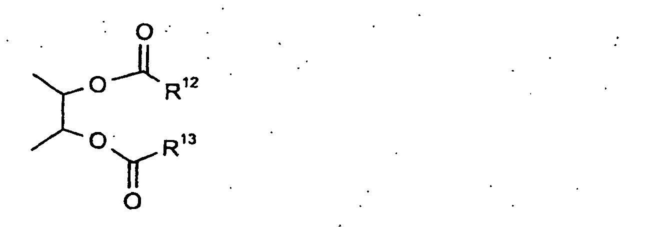 enthalten ist, oder
A und Q¹ gemeinsam für jeweils gegebenenfalls einfach oder zweifach, gleich oder verschieden durch Halogen, Hydroxy, durch jeweils gegebenenfalls einfach bis dreifach, gleich oder verschieden durch Halogen substituiertes C₁-C₁₀-Alkyl, C₁-C₆-Alkoxy, C₁-C₆-Alkylthio, C₃-C₇-Cycloalkyl oder durch jeweils gegebenenfalls einfach bis dreifach, gleich oder verschieden durch Halogen, C₁-C₆-Alkyl oder C₁-C₆-Alkoxy substituiertes Benzyloxy oder Phenyl substituiertes C₃-C₆-Alkandiyl oder C₄-C₆-Alkendiyl stehen, welches außerdem gegebenenfalls eine der nachstehenden Gruppen 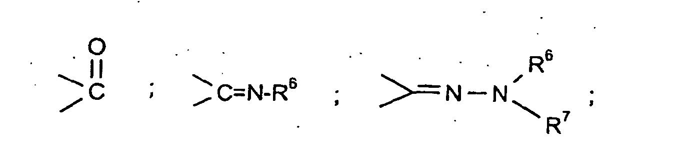 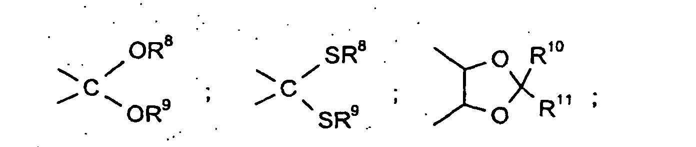 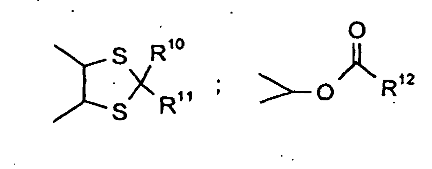 oder 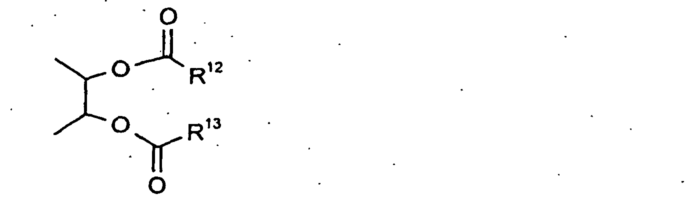 enthält oder durch eine C₁-C₂-Alkandiylgruppe oder durch ein Sauerstoffatom überbrückt ist oder
Q¹ für Wasserstoff oder C₁-C₄-Alkyl steht,
Q², Q⁴, Q⁵ und Q⁶ unabhängig voneinander für Wasserstoff oder C₁-C₄-Alkyl stehen,
Q³ für Wasserstoff, C₁-C₆-Alkyl, C₁-C₆-Alkoxy-C₁-C₂-alkyl, C₁-C₆-Alkylthio-C₁-C₂-alkyl, gegebenenfalls durch C₁-C₄-Alkyl oder C₁-C₄-Alkoxy substituiertes C₃-C₈-Cycloalkyl, worin gegebenenfalls eine Methylengruppe durch Sauerstoff oder Schwefel ersetzt ist oder gegebenenfalls durch Halogen, C₁-C₄-Alkyl, C₁-C₄-Alkoxy, C₁-C₂-Halogenalkyl, C₁-C₂-Halogenalkoxy, Cyano oder Nitro substituiertes Phenyl steht, oder
Q³ und Q⁴ gemeinsam mit dem Kohlenstoffatom, an das sie gebunden sind, für gegebenenfalls durch C₁-C₄-Alkyl, C₁-C₄-Alkoxy oder C₁-C₂-Halogenalkyl substituiertes C₃-C₇-Cycloalkyl stehen, in welchem gegebenenfalls ein Ringglied durch Sauerstoff oder Schwefel ersetzt ist,
L für eine Alkandiylgruppe mit 1 bis 6 Kohlenstoffatomen steht,
M für eine der folgenden Gruppierungen: CN; 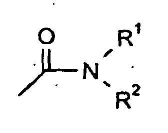 -CO₂R², -OR², -SR², -COR³, 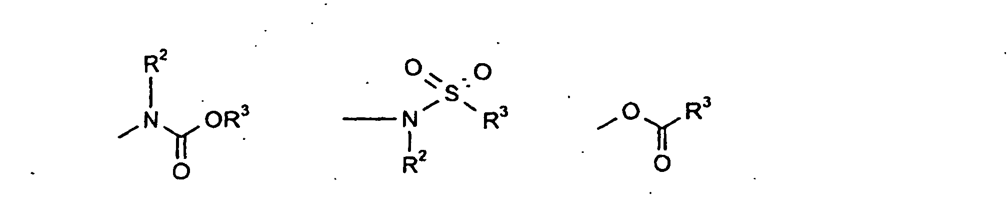 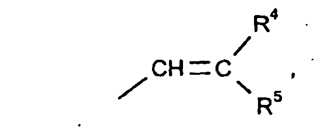 ―C≡C―R⁴ oder 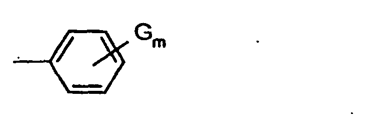
steht,
R¹ für Wasserstoff oder C₁-C₁₂-Alkyl steht,
R² für jeweils gegebenenfalls durch Halogen substituiertes C₁-C₁₂-Alkyl, C₃-C₈-Alkenyl, C₃-C₈-Alkinyl, C₃-C₈-Cycloalkyl, C₁-C₈-Alkoxy-C₂-C₈-alkyl, C₁-C₈-Alkylthio-C₂-C₈-alkyl, für jeweils gegebenenfalls durch Halogen, Nitro, Cyano, C₁-C₆-Alkoxy, C₁-C₆-Halogenalkoxy, C₁-C₆-Alkylthio, C₁-C₆-Halogenakylthio, C₁-C₆-Alkyl oder C₁-C₆-Halogenalkyl substituiertes Phenyl oder Benzyl steht,
R³ für gegebenenfalls durch Halogen substituiertes C₁-C₁₂-Alkyl oder für jeweils durch Halogen, C₁-C₆-Alkyl, C₁-C₆-Halogenalkyl, C₁-C₆-Alkoxy oder C₁-C₆-Halogenalkoxy substituiertes Phenyl oder Benzyl. steht,
R⁴ für Wasserstoff, Halogen, gegebenenfalls durch Halogen substituiertes C₁-C₆-Alkyl oder jeweils durch Halogen, C₁-C₆-Alkyl, C₁-C₆-Alkoxyl, C₁-C₄-Halogenalkyl, C₁-C₄-Halogenalkoxy, Cyano oder Nitro substituiertes Phenyl steht,
R⁵ für Wasserstoff, Halogen oder gegebenenfalls durch Halogen substituiertes C₁-C₆-Alkyl steht,
G für Halogen, C₁-C₆-Alkyl, C₁-C₆-Alkoxy, C₁-C₄-Halgenoalkyl, C₁-C₄-Halogenalkoxy, Cyano oder Nitro steht,
m für eine Zahl 0, 1, 2 oder 3 steht,
R⁶ für Wasserstoff, für jeweils gegebenenfalls durch Halogen substituiertes C₁-C₈-Alkyl oder C₁-C₈-Alkoxy, für gegebenenfalls durch Halogen, C₁-C₄-Alkyl oder C₁-C₄-Alkoxy substituiertes C₃-C₈-Cycloalkyl, in welchem gegebenenfalls eine Methylengruppe durch Sauerstoff oder Schwefel ersetzt ist, oder für jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenyl-C₁-C₄-alkyl oder Pheny-C₁-C₄-alkoxy steht,
R⁷ für Wasserstoff oder C₁-C₈-Alkyl steht, oder
R⁶ und R⁷ gemeinsam für C₄-C₆-Alkandiyl stehen,
R⁸ und R⁹ gleich oder verschieden sind und für C₁-C₆-Alkyl stehen, oder
R⁸ und R⁹ gemeinsam für einen C₂-C₄-Alkandiylrest stehen, der gegebenenfalls durch C₁-C₆-Alkyl, C₁-C₆-Halogenalkyl oder durch gegebenenfalls durch Halogen, C₁-C₆-Alkyl, C₁-C₄-Halogenalkyl, C₁-C₆-Alkoxy, C₁-C₄-Halogenalkoxy, Nitro oder Cyano substituiertes Phenyl substituiert ist,
R¹⁰ und R¹¹ unabhängig voneinander für Wasserstoff, für gegebenenfalls durch Halogen substituiertes C₁-C₈-Alkyl oder für gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl stehen, oder
R¹⁰ und R¹¹ gemeinsam mit dem Kohlenstoffatom, an das sie gebunden sind, für eine Carbonylgruppe oder für gegebenenfalls durch Halogen, C₁-C₄-Alkyl oder C₁-C₄-Alkoxy substituiertes C₅-C₇-Cycloalkyl stehen, in dem gegebenenfalls eine Methylengruppe durch Sauerstoff oder Schwefel ersetzt ist,
R¹² und R¹³ unabhängig voneinander für C₁-C₁₀-Alkyl, C₂-C₁₀-Alkenyl, C₁-C₁₀-Alkoxy, C₁-C₁₀-Alkylamino, C₃-C₁₀-Alkenylamino, Di-(C₁-C₁₀-alkyl)amino oder Di-(C₃-C₁₀-alkenyl)amino stehen.

2. Verbindungen der Formel (I) gemäß Anspruch 1, in welcher
W für Wasserstoff, Fluor, Chlor, Brom, C₁-C₄-Alkyl, C₁-C₄-Alkoxy, C₁-C₄-Halogenalkyl, C₁-C₄-Halogenalkoxy, Nitro oder Cyano steht,
X für Fluor, Chlor, Brom, C₁-C₄-Alkyl, C₁-C₄-Alkoxy, C₃-C₄-Alkenyloxy, C₁-C₄-Halogenalkyl, C₁-C₄-Halogenalkoxy, C₃-C₄-Halogenalkenyloxy, Nitro oder Cyano steht,
Y für einen der Reste 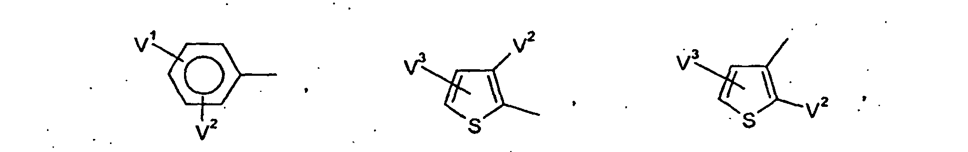 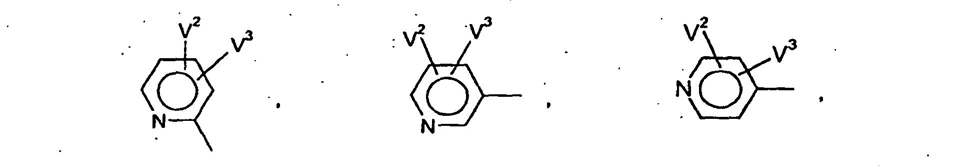 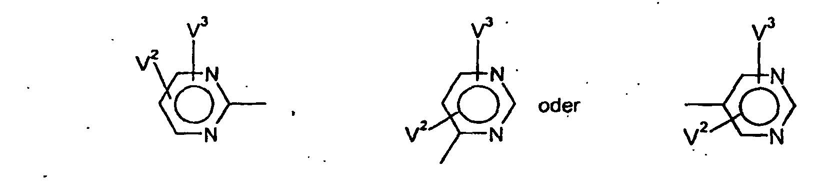 steht,
V¹ für Wasserstoff, Fluor, Chlor, Brom, C₁-C₆-Alkyl, C₁-C₄-Alkoxy, C₁-C₂-Halogenalkyl, C₁-C₂-Halogenalkoxy, Nitro,Cyano oder jeweils gegebenenfalls einfach oder zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl, Phenoxy, Phenoxy-C₁-C₂-alkyl, Phenyl-C₁-C₂-alkoxy, Phenylthio-C₁-C₂-alkyl oder Phenyl-C₁-C₂-alkylthio steht,
V² und V³ unabhängig voneinander für Wasserstoff, Fluor, Chlor, Brom, C₁-C₄-Alkyl, C₁-C₄-Alkoxy, C₁-C₂-Halogenalkyl oder C₁-C₂-Halogenalkoxy stehen,
Z für Wasserstoff, Fluor, Chlor, Brom, C₁-C₄-Alkyl, C₁-C₄-Halogenalkyl, C₁-C₄-Alkoxy oder C₁-C₄-Halogenalkoxy steht,
CKE für eine der Gruppen 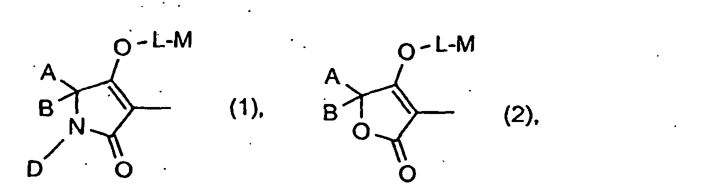 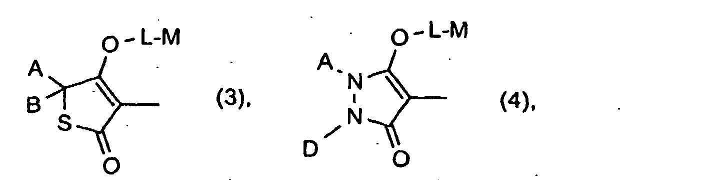 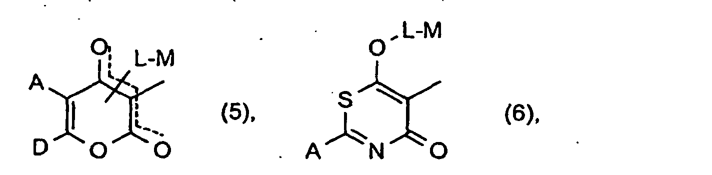 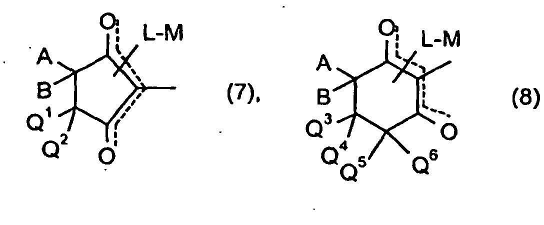 steht,
A für Wasserstoff, jeweils gegebenenfalls durch Fluor oder Chlor substituiertes C₁-C₁₀-Alkyl, C₁-C₈-Alkoxy-C₁-C₆-alkyl, gegebenenfalls durch Fluor, Chlor, C₁-C₄-Alkyl oder C₁-C₄-Alkoxy substituiertes C₃-C₇-Cycloalkyl, in welchem gegebenenfalls ein Ringglied durch Sauerstoff oder Schwefel ersetzt ist oder (jedoch nicht im Fall der Verbindungen der Formeln (I-5), (1-7) und (I-8)) jeweils gegebenenfalls durch Fluor, Chlor, Brom, C₁-C₄-Alkyl, C₁-C₄-Halogenalkyl, C₁-C₄-Alkoxy, Cyano, Nitro oder C₁-C₄-Halogenalkoxy substituiertes Phenyl, Furanyl, Pyridyl, Imidazolyl, Triazolyl, Pyrazolyl, Pyrimidyl, Thiazolyl, Thienyl oder Phenyl-C₁-C₄-alkyl steht,
B für Wasserstoff oder C₁-C₆-Alkyl steht, oder
A, B und das Kohlenstoffatom an das sie gebunden sind für gesättigtes oder ungesättigtes C₅-C₇-Cycloalkyl stehen, worin gegebenenfalls ein Ringglied durch Sauerstoff oder Schwefel ersetzt ist und welches gegebenenfalls einfach durch C₁-C₆-Alkyl, C₅-C₈-Cycloalkyl, C₁-C₃-Halogenalkyl, C₁-C₆-Alkoxy, Fluor, Chlor oder Phenyl substituiert ist oder
A, B und das Kohlenstoffatom, an das sie gebunden sind für C₅-C₆-Cycloalkyl stehen, welches durch eine gegebenenfalls ein oder zwei nicht direkt benachbarte Sauerstoff- oder Schwefelatome enthaltende Alkylendiyl- oder durch eine Alkylendioxyl- oder durch eine Alkylendithiol-Gruppe substituiert ist, die mit dem Kohlenstoffatom, an das sie gebunden ist, einen weiteren fünf- oder sechsgliedrigen Ring bildet oder
A, B und das Kohlenstoffatom, an das sie gebunden sind für C₃-C₆-Cycloalkyl oder C₅-C₆-Cycloalkenyl stehen, in welchen zwei Substituenten gemeinsam mit den Kohlenstoffatomen, an die sie gebunden sind, für jeweils gegebenenfalls durch C₁-C₅-Alkyl, C₁-C₅-Alkoxy, Fluor, Chlor oder Brom substituiertes C₂-C₄-Alkandiyl, C₂-C₄-Alkendiyl, worin gegebenenfalls eine Methylengruppe durch Sauerstoff oder Schwefel ersetzt ist oder Butadiendiyl stehen,
D für Wasserstoff, für jeweils gegebenenfalls durch Fluor oder Chlor substituiertes C₁-C₁₀-Alkyl, C₃-C₆-Alkenyl, C₁-C₈-Alkoxy-C₂-C₆-alkyl oder C₁-C₈-Alkylthio-C₂-C₆-alkyl, für gegebenenfalls durch Fluor, Chlor, C₁-C₄-Alkyl, C₁-C₄-Alkoxy oder C₁-C₂-Halogenalkyl substituiertes C₃-C₇-Cycloalkyl, in welchem gegebenenfalls eine Methylengruppe durch Sauerstoff oder Schwefel ersetzt ist oder (jedoch nicht im Fall der Verbindungen der Formeln (I-1) und (I-4)) für jeweils gegebenenfalls durch Fluor, Chlor, Brom, C₁-C₄-Alkyl, C₁-C₄-Halogenalkyl, C₁-C₄-Alkoxy oder C₁-C₄-Halogenalkoxy substituiertes Phenyl, Furanyl, Imidazolyl, Pyridyl, Thiazolyl, Pyrazolyl, Pyrimidyl, Pyrrolyl, Thienyl, Triazolyl oder Phenyl-C₁-C₄-alkyl steht,oder
A und D gemeinsam für gegebenenfalls substituiertes C₃-C₅-Alkandiyl stehen, in welchem eine Methylengruppe durch eine Carbonylgruppe, Sauerstoff oder Schwefel ersetzt sein kann, wobei als Substituenten Hydroxy, C₁-C₆-Alkyl oder C₁-C₄-Alkoxy in Frage kommen oder
A und D (im Fall der Verbindungen der Formel (I-1)) gemeinsam mit den Atomen, an die sie gebunden sind, für eine der Gruppen AD-1 bis AD-10: 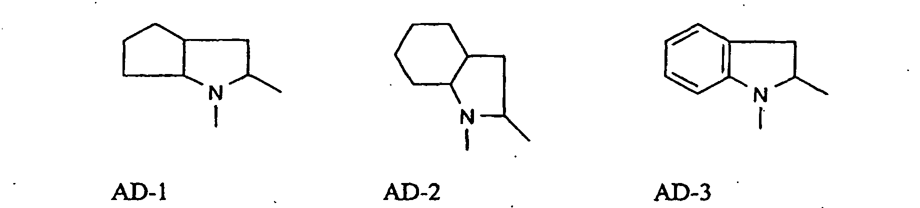 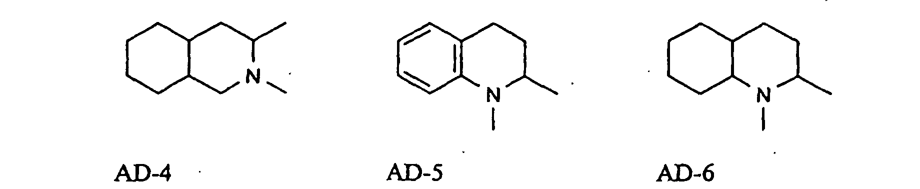 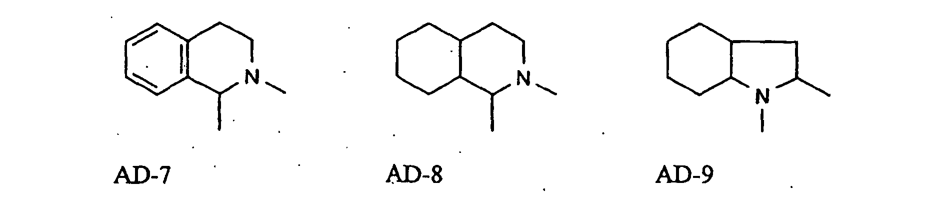 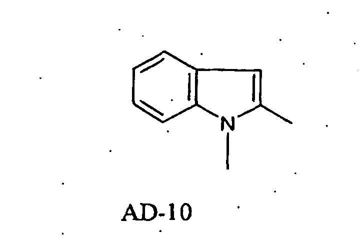 stehen oder
A und Q¹ gemeinsam für jeweils gegebenenfalls einfach oder zweifach, gleich oder verschieden durch Fluor, Chlor, Hydroxy, durch jeweils gegebenenfalls einfach bis dreifach durch Fluor substituiertes C₁-C₈-Alkyl oder C₁-C₄-Alkoxy substituiertes C₃-C₄-Alkandiyl oder C₃-C₄-Alkendiyl stehen, oder
Q¹ für Wasserstoff steht,
Q² für Wasserstoff steht,
Q⁴, Q⁵ und Q⁶ unabhängig voneinander für Wasserstoff oder C₁-C₃-Alkyl stehen,
Q³ für Wasserstoff, C₁-C₄-Alkyl, C₁-C₄-Alkoxy-C₁-C₂-alkyl, C₁-C₄-Alkylthio-C₁-C₂-alkyl oder gegebenenfalls durch Methyl oder Methoxy substituiertes C₃-C₆-Cycloalkyl steht, worin gegebenenfalls eine Methylengruppe durch Sauerstoff oder Schwefel ersetzt ist oder
Q³ und Q⁴ gemeinsam mit dem Kohlenstoff, an das sie gebunden sind, für gegebenenfalls durch C₁-C₄-Alkyl oder C₁-C₄-Alkoxy substituiertes gesättigtes C₅-C₆-Cycloalkyl stehen, in welchem gegebenenfalls ein Ringglied durch Sauerstoff oder Schwefel ersetzt ist,
L für eine Alkandiylgruppe mit 1 bis 4 Kohlenstoffatomen steht,
M für eine der folgenden Gruppierungen: CN; 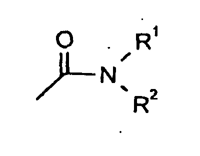 -CO₂R² ; -OR², -SR², -COR³, 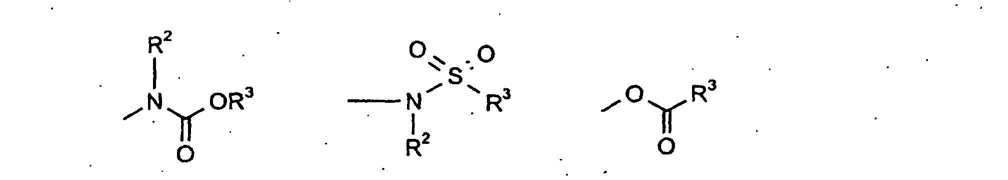 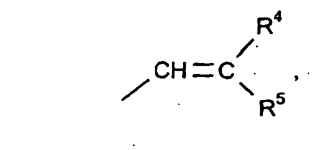 ―C≡C―R⁴ oder 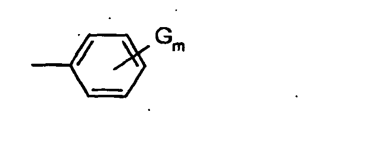
steht,
R¹ für Wasserstoff oder C₁-C₁₀-Alkyl steht,
R² für jeweils gegebenenfalls durch Fluor, Chlor, Brom substituiertes C₁-C₁₀-Alkyl, C₃-C₆-Alkenyl, C₃-C₆-Alkinyl, C₃-C₈-Cycloalkyl, C₁-C₆-Alkoxy-C₂-C₆-alkyl, C₁-C₆-Alkylthio-C₂-C₆-alkyl, für jeweils gegebenenfalls durch Fluor, Chlor, Brom, Nitro, Cyano, C₁-C₄-Alkylthio, C₁-C₄-Alkoxy, C₁-C₄-Halogenalkoxy, C₁-C₄-Halogenalkylthio, C₁-C₄-Alkyl oder C₁-C₄-Halogenalkyl substituiertes Phenyl oder Benzyl steht,
R³ für gegebenenfalls durch Fluor, Chlor, Brom substituiertes C₁-C₁₀-Alkyl oder für jeweils durch Fluor, Chlor, C₁-C₄-Alkyl, C₁-C₄-Halogenalkyl, C₁-C₄-Alkoxy oder C₁-C₄-Halogenalkoxy substituiertes Phenyl oder Benzyl steht,
R⁴ für Wasserstoff, Fluor, Chlor, Brom, gegebenenfalls durch Fluor oder Chlor substituiertes C₁-C₅-Alkyl oder durch Fluor, Chlor, Brom, C₁-C₄-Alkyl, C₁-C₄-Alkoxy, C₁-C₂-Haloalkyl, C₁-C₂-Haloalkoxy, Cyano oder Nitro substituiertes Phenyl steht,
R⁵ für Wasserstoff, Fluor, Chlor, Brom oder gegebenenfalls durch Fluor oder Chlor substituiertes C₁-C₄-Alkyl steht,
G für Fluor, Chlor, Brom, C₁-C₄-Alkyl, C₁-C₄-Alkoxy, C₁-C₂-Halogenalkyl, C₁-C₂-Halogenalkoxy, Cyano oder Nitro steht,
m für eine Zahl 0, 1 oder 2 steht.

3. Verbindungen der Formel (I) gemäß Anspruch 1, in welcher
W für Wasserstoff, Chlor, Fluor, Methyl, Ethyl, Methoxy, Ethoxy steht,
X für Fluor, Chlor, Methyl, Ethyl, n-Propyl, iso-Propyl, Methoxy, Ethoxy, n-Propoxy, iso-Propoxy, Trifluormethyl, Difluormethoxy, Trifluormethoxy, Nitro oder Cyano steht,
Y für einen der Reste 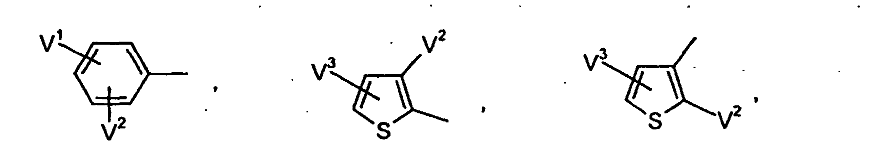 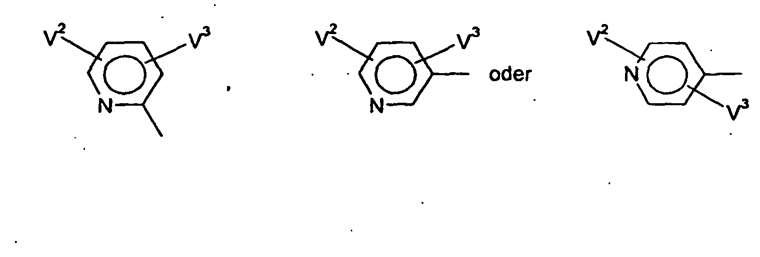 steht,
V¹ für Wasserstoff, Fluor, Chlor, Brom, Methyl, Ethyl, n-Propyl, iso-Propyl, n-Butyl, iso-Butyl, tert.-Butyl, Methoxy, Ethoxy, n-Propoxy, iso-Propoxy, Trifluormethyl, Trifluormethoxy, Nitro, Cyano oder Phenyl steht,
V² und V³ unabhängig voneinander für Wasserstoff, Fluor, Chlor, Methyl, Ethyl, n-Propyl, iso-Propyl, Methoxy, Ethoxy, Trifluormethyl oder Trifluormethoxy stehen,
Z für Wasserstoff, Fluor, Chlor, Methyl oder Methoxy steht,
CKE bevorzugt für eine der Gruppen 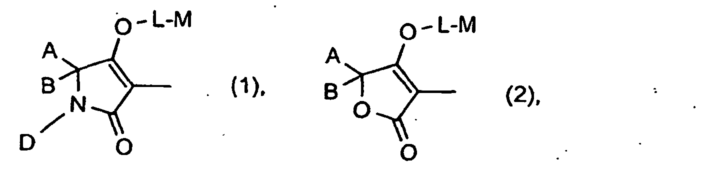 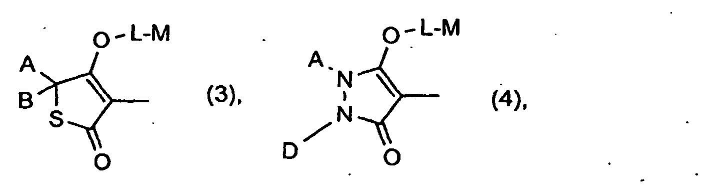 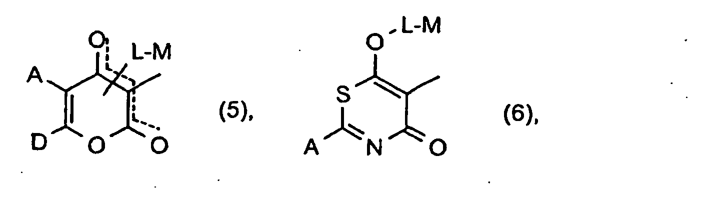 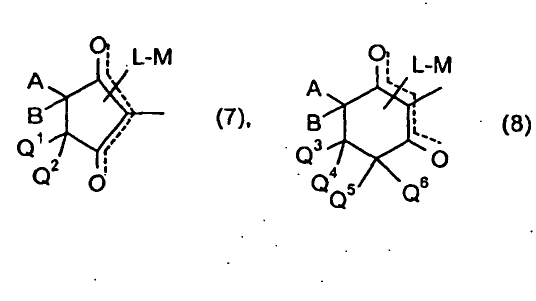 steht,
A für Wasserstoff, jeweils gegebenenfalls durch Fluor substituiertes C₁-C₈-Alkyl oder C₁-C₆-Alkoxy-C₁-C₄-alkyl, gegebenenfalls durch Fluor, Methyl, Ethyl oder Methoxy substituiertes C₃-C₆-Cycloalkyl, in welchem gegebenenfalls ein Ringglied durch Sauerstoff oder Schwefel ersetzt ist oder (jedoch nicht im Fall der Verbindungen der Formeln (I-5), (1-7) und (I-8)) für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl oder Benzyl steht,
B für Wasserstoff oder C₁-C₄-Alkyl steht, oder
A, B und das Kohlenstoffatom an das sie gebunden sind für gesättigtes C₅-C₆-Cycloalkyl stehen, in welchem gegebenenfalls ein Ringglied durch Sauerstoff oder Schwefel ersetzt ist und welches gegebenenfalls einfach durch Methyl, Ethyl, n-Propyl, Isopropyl, Butyl, iso-Butyl, sec.-Butyl, tert.-Butyl, Trifluormethyl, Methoxy, Ethoxy, n-Propoxy, iso-Propoxy, n-Butoxy, iso-Butoxy, sek.-Butoxy, tert.-Butoxy, Fluor oder Chlor substituiert ist oder
A, B und das Kohlenstoffatom, an das sie gebunden sind für C₅-C₆-Cycloalkyl stehen, worin zwei Substituenten gemeinsam mit den Kohlenstoffatomen, an die sie gebunden sind, für C₂-C₄-Alkandiyl oder C₂-C₄-Alkendiyl, worin jeweils gegebenenfalls eine Methylengruppe durch Sauerstoff oder Schwefel ersetzt ist oder Butadiendiyl stehen.
D für Wasserstoff, für jeweils gegebenenfalls durch Fluor oder Chlor substituiertes C₁-C₈-Alkyl, C₃-C₄-Alkenyl, C₁-C₆-Alkoxy-C₂-C₄-alkyl, C₁-C₄-Alkylthio-C₂-C₄-alkyl oder C₃-C₆-Cycloalkyl, in welchem gegebenenfalls eine Methylengruppe durch Sauerstoff oder Schwefel ersetzt ist oder (jedoch nicht im Fall der Verbindungen der Formeln (I-1) und (I-4)) für jeweils gegebenenfalls durch Fluor, Chlor, Methyl, Ethyl, n-Propyl, iso-Propyl, Methoxy, Ethoxy, Trifluormethyl oder Trifluormethoxy substituiertes Phenyl, Furanyl, Pyridyl, Thienyl oder Benzyl steht,
oder
A und D gemeinsam für gegebenenfalls substituiertes C₃-C₄-Alkandiyl stehen, worin gegebenenfalls ein Kohlenstoffatom durch Schwefel ersetzt ist und welches gegebenenfalls durch Hydroxy, Methyl, Ethyl, Methoxy oder Ethoxy substituiert ist oder
A und D (im Fall der Verbindungen der Formel (I-1)) gemeinsam mit den Atomen, an die sie gebunden sind, für eine der folgenden Gruppen AD: 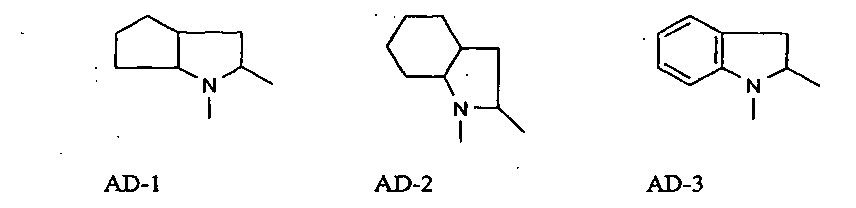 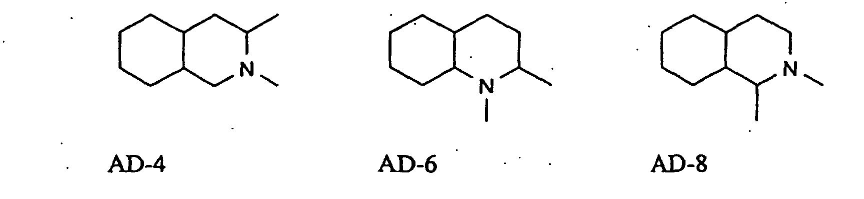 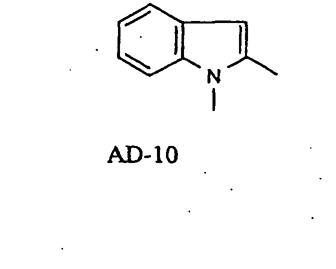 stehen
A und Q¹ gemeinsam für gegebenenfalls einfach oder zweifach durch Fluor, Hydroxy, Methyl oder Methoxy substituiertes C₃-C₄-Alkandiyl oder Butendiyl stehen, oder
Q¹ für Wasserstoff steht,
Q² für Wasserstoff steht,
Q⁴, Q⁵ und Q⁶ unabhängig voneinander für Wasserstoff, Methyl oder Ethyl stehen,
Q³ für Wasserstoff, Methyl, Ethyl oder C₃-C₆-Cycloalkyl, worin gegebenenfalls eine Methylengruppe durch Sauerstoff oder Schwefel ersetzt steht, oder
Q³ und Q⁴ gemeinsam mit dem Kohlenstoff, an den sie gebunden sind, für gegebenenfalls durch Methyl oder Methoxy, substituiertes gesättigtes C₅-C₆-Cycloalkyl stehen, in welchem gegebenenfalls ein Ringglied durch Sauerstoff oder Schwefel ersetzt ist,
L für eine der folgenden Gruppierungen -CH₂-, 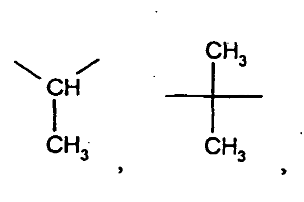 -CH₂-CH₂, 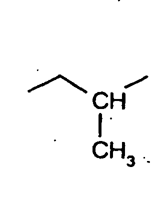 steht,
M für eine der folgenden Gruppierungen CN; 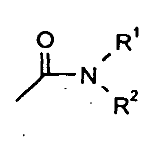 -CO₂R², -OR², -SR², -COR³, 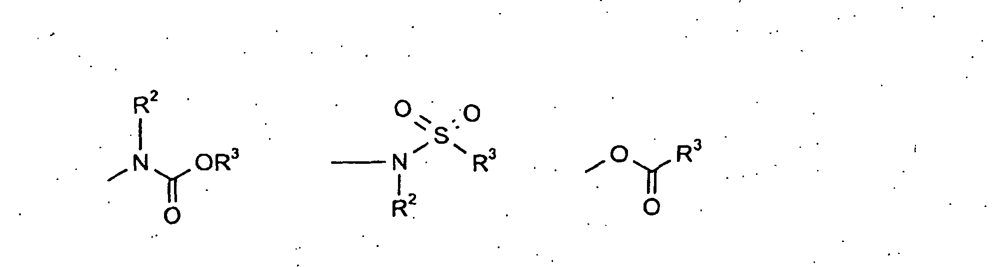 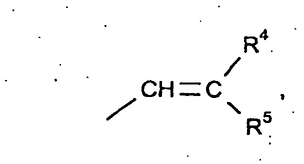 ―C≡C―R⁴ oder 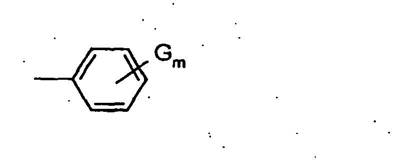
steht,
R¹ für Wasserstoff oder C₁-C₈-Alkyl steht,
R² für jeweils gegebenenfalls durch Fluor, Chlor, Brom substituiertes C₁-C₈-Alkyl, C₃-C₄-Alkenyl, C₃-C₄-Alkinyl, Cyclopropyl, Cyclopentyl, Cyclohexyl, C₁-C₆-Alkoxy-C₂-C₄-alkyl, C₁-C₆-Alkylthio-C₂-C₄-alkyl, für jeweils gegebenenfalls durch Fluor, Chlor, Brom, Nitro, Cyano, Methylthio, Ethylthio, Methoxy, Ethoxy, Trifluormethylthio, Trifluormethoxy, Methyl, Ethyl, Trifluormethyl substituiertes Phenyl oder Benzyl steht,
R³ für jeweils gegebenenfalls durch Fluor, Chlor, Brom substituiertes C₁-C₈-Alkyl oder durch Fluor, Chlor, C₁-C₂-Alkyl, Trifluormethyl, C₁-C₂-Alkoxy oder Trifluormethoxy substituiertes Phenyl oder Benzyl steht,
R⁴ für Wasserstoff, Fluor, Chlor, gegebenenfalls durch Fluor oder Chlor substituiertes C₁-C₄-Alkyl oder durch Fluor, Chlor, Methyl, Ethyl, Methoxy, Ethoxy, Trifluormethyl, Difluormethoxy, Trifluormethoxy, Cyano oder Nitro substituiertes Phenyl steht,
R⁵ für Wasserstoff, Fluor, Chlor oder gegebenenfalls durch Fluor oder Chlor substituiertes Methyl, Ethyl, Propyl oder Isopropyl steht,
G für Fluor, Chlor, Methyl, Ethyl, Propyl, Isopropyl, Methoxy, Ethoxy, Propoxy, iso-Propoxy, Trifluormethyl, Difluormethoxy, Trifluormethoxy, Cyano oder Nitro steht,
m für eine Zahl von 0 bis 2 steht.

4. Verbindungen der Formel (I) gemäß Anspruch 1, in welcher
W für Wasserstoff, Chlor, Fluor, Methyl, Ethyl, Methoxy, Ethoxy steht.
X für Fluor, Chlor, Methyl, Ethyl, n-Propyl, iso-Propyl, Methoxy, Ethoxy, n-Propoxy, iso-Propoxy, Trifluormethyl, Difluormethoxy, Trifluormethoxy, Nitro oder Cyano steht.
Y für einen der Reste 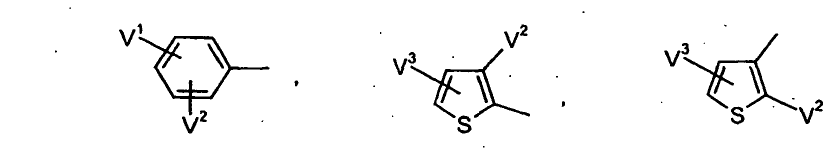 steht.
V¹ für Wasserstoff, Fluor, Chlor, Methyl, Ethyl, n-Propyl, iso-Propyl, n-Butyl, iso-Butyl, tert.-Butyl, Methoxy, Ethoxy, n-Propoxy, iso-Propoxy, Trifluormethyl, Trifluormethoxy, Nitro, Cyano oder Phenyl steht,
V² für Wasserstoff, Fluor, Chlor, Methyl, Methoxy, Trifluormethyl oder Trifluormethoxy steht,
V³ für Wasserstoff, Methyl oder Chlor steht,
Z für Wasserstoff, Fluor, Chlor, Methyl oder Methoxy steht,
CKE für eine der Gruppen 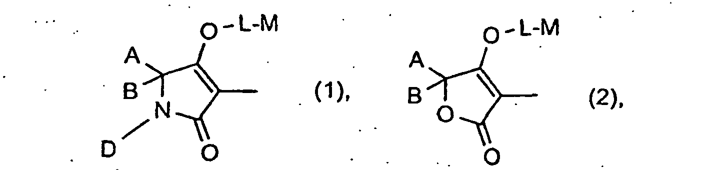 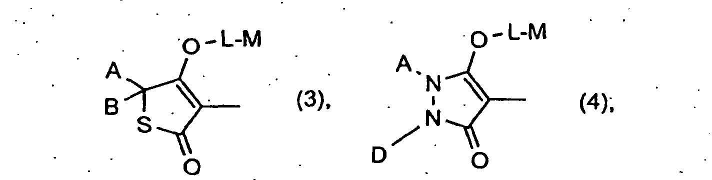 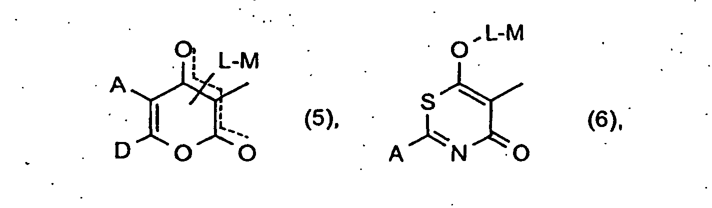 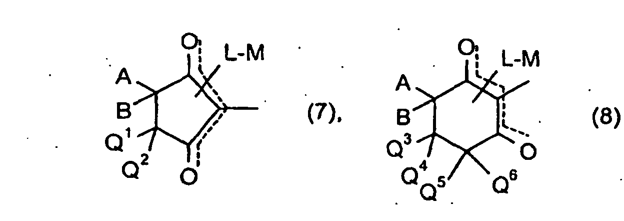 steht,
A für Wasserstoff, jeweils gegebenenfalls durch Fluor substituiertes C₁-C₈-Alkyl oder C₁-C₆-Alkoxy-C₁-C₄-alkyl, gegebenenfalls durch Fluor, Methyl oder Methoxy substituiertes C₃-C₆-Cycloalkyl, in welchem gegebenenfalls ein Ringglied durch Sauerstoff oder Schwefel ersetzt ist oder (jedoch nicht im Fall der Verbindungen der Formeln (I-5), (I-7) und (I-8)) für jeweils gegebenenfalls durch Fluor, Chlor, Brom, Methyl, Methoxy, Trifluormethyl oder Trifluormethoxy, substituiertes Phenyl oder Benzyl steht,
B für Wasserstoff oder C₁-C₄-Alkyl steht, oder
A, B und das Kohlenstoffatom an das sie gebunden sind für gesättigtes C₅-C₆-Cycloalkyl stehen, in welchem gegebenenfalls ein Ringglied durch Sauerstoff oder Schwefel ersetzt ist und welches gegebenenfalls einfach durch Methyl, Ethyl, n-Propyl, Isopropyl, Trifluormethyl, Methoxy, Ethoxy, n-Propoxy, iso-Propoxy, n-Butoxy oder iso-Butoxy substituiert ist oder
A, B und das Kohlenstoffatom, an das sie gebunden sind für C₅-C₆-Cycloalkyl stehen, worin zwei Substituenten gemeinsam mit den Kohlenstoffatomen, an die sie gebunden sind, für C₂-C₄-Alkandiyl oder C₂-C₄-Alkendiyl, worin jeweils gegebenenfalls eine Methylengruppe durch Sauerstoff oder Schwefel ersetzt ist oder Butadiendiyl stehen,
D für Wasserstoff, für jeweils gegebenenfalls durch Fluor oder Chlor substituiertes C₁-C₆-Alkyl, C₃-C₄-Alkenyl, C₁-C₄-Alkoxy-C₂-C₄-alkyl, C₁-C₄-Alkylthio-C₂-C₄-alkyl oder C₃-C₆-Cycloalkyl, in welchem gegebenenfalls eine Methylengruppe durch Sauerstoff oder Schwefel ersetzt ist oder (jedoch nicht im Fall der Verbindungen der Formeln (I-1) und (I-4)), für jeweils gegebenenfalls durch Fluor, Chlor, Methyl, Methoxy, Trifluormethyl oder Trifluormethoxy substituiertes Phenyl, Pyridyl oder Benzyl steht,
oder
A und D gemeinsam für gegebenenfalls substituiertes C₃-C₄-Alkandiyl stehen, worin gegebenenfalls ein Kohlenstoffatom durch Schwefel ersetzt ist und welches gegebenenfalls durch Methyl oder Methoxy substituiert ist,
A und Q¹ gemeinsam für gegebenenfalls einfach oder zweifach durch Methyl oder Methoxy substituiertes C₃-C₄-Alkandiyl oder Butendiyl stehen, oder
Q¹ für Wasserstoff steht,
Q² für Wasserstoff steht,
Q⁴, Q⁵ und Q⁶ unabhängig voneinander für Wasserstoff, Methyl oder Ethyl stehen,
Q³ für Wasserstoff, Methyl, Ethyl oder C₃-C₆-Cycloalkyl steht, worin gegebenenfalls eine Methylengruppe durch Sauerstoff oder Schwefel ersetzt ist,
Q³ und Q⁴ gemeinsam mit dem Kohlenstoff, an den sie gebunden sind, für gegebenenfalls durch Methyl oder Methoxy substituiertes gesättigtes C₅-C₆-Cycloalkyl stehen, in welchem gegebenenfalls ein Ringglied durch Sauerstoff oder Schwefel ersetzt ist,
L für eine der folgenden Gruppierungen -CH₂-, 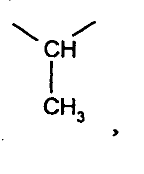 -CH₂-CH₂ steht,
M für eine der folgenden Gruppierungen CN; 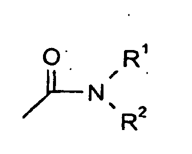 -CO₂R², -OR², -SR², -COR³, 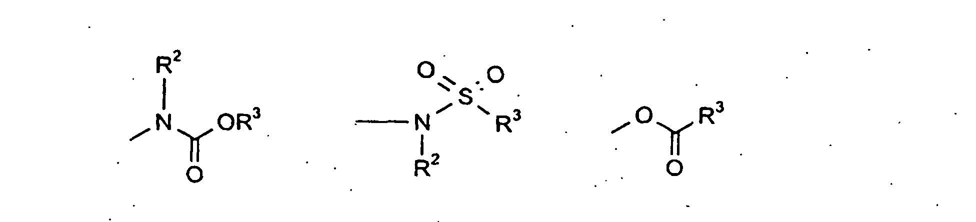 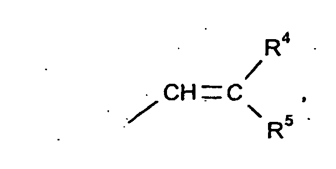 ―C≡C―R⁴ oder 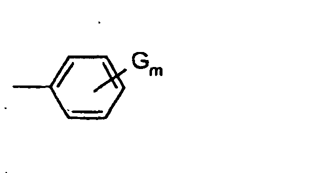
steht,
R¹ für Wasserstoff oder C₁-C₄-Alkyl steht,
R² für jeweils gegebenenfalls durch Fluor substituiertes C₁-C₄-Alkyl, C₃-C₄-Alkenyl, C₃-C₄-Alkinyl, Cyclopropyl, Cyclopentyl, Cyclohexyl, C₁-C₄-Alkoxy-C₂-C₄-alkyl, für jeweils gegebenenfalls durch Fluor, Chlor, Brom, Nitro, Cyano, Methylthio, Ethylthio, Methoxy, Ethoxy, Trifluormethylthio, Trifluormethoxy, Methyl, Ethyl, Trifluormethyl substituiertes Phenyl oder Benzyl steht,
R³ für jeweils gegebenenfalls durch Fluor substituiertes C₁-C₄-Alkyl oder durch Fluor, Chlor, Methyl, Trifluormethyl, Methoxy oder Trifluormethoxy substituiertes Phenyl steht,
R⁴ für Wasserstoff, Fluor, Chlor, gegebenenfalls durch Fluor substituiertes C₁-C₄-Alkyl oder durch Fluor, Chlor, Methyl, Ethyl, Methoxy, Ethoxy, Trifluormethyl, Difluormethoxy, Trifluormethoxy, Cyano oder Nitro substituiertes Phenyl steht,
R⁵ für Wasserstoff, Fluor, Chlor oder gegebenenfalls durch Fluor substituiertes Methyl steht,
G für Fluor, Chlor, Methyl, Methoxy, Trifluormethyl, Difluormethoxy, Trifluormethoxy, Cyano oder Nitro steht,
m für eine Zahl von 0 bis 1 steht.

5. Verfahren zur Herstellung von Verbindungen der Formel (I-1) bis (I-8) gemäß Anspruch 1 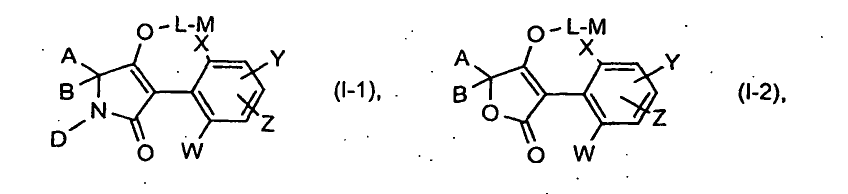 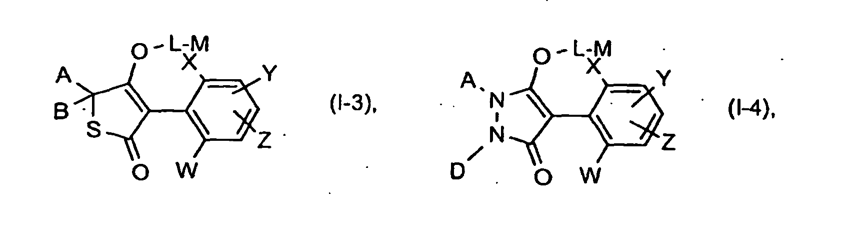 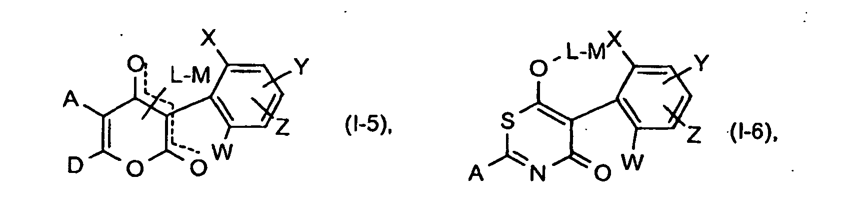 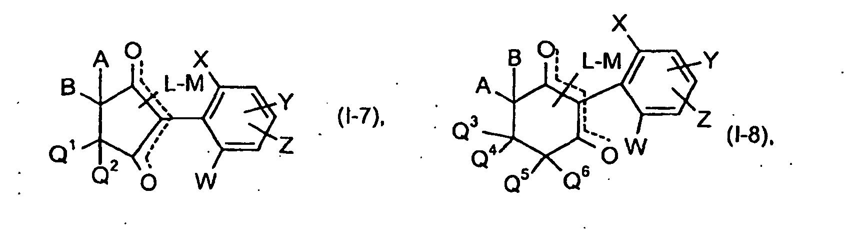 worin
A, B, D, L, M, Q¹, Q², Q³, Q⁴, Q⁵, Q⁶, W, X, Y und Z die oben angegebenen Bedeutungen haben,
**dadurch gekennzeichnet, dass** man Verbindungen der Formel (II-1) bis (II-8) 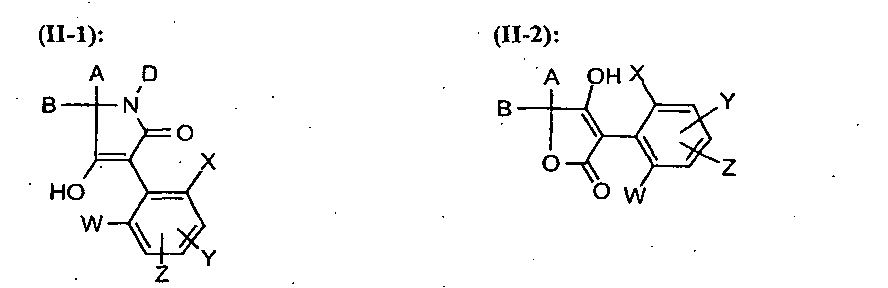 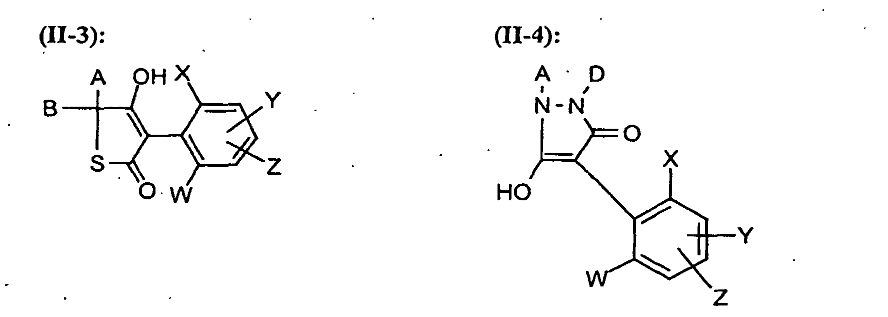 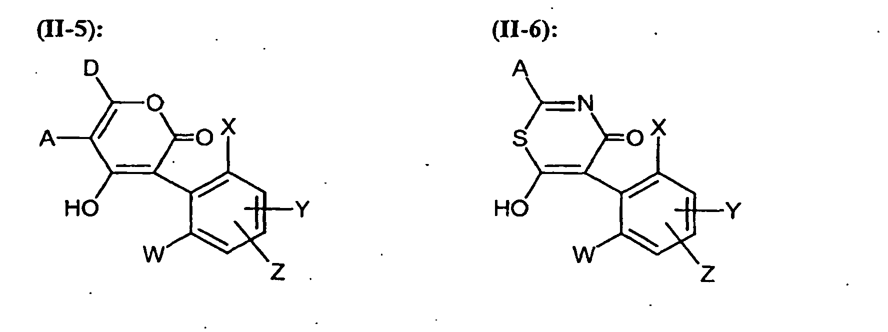 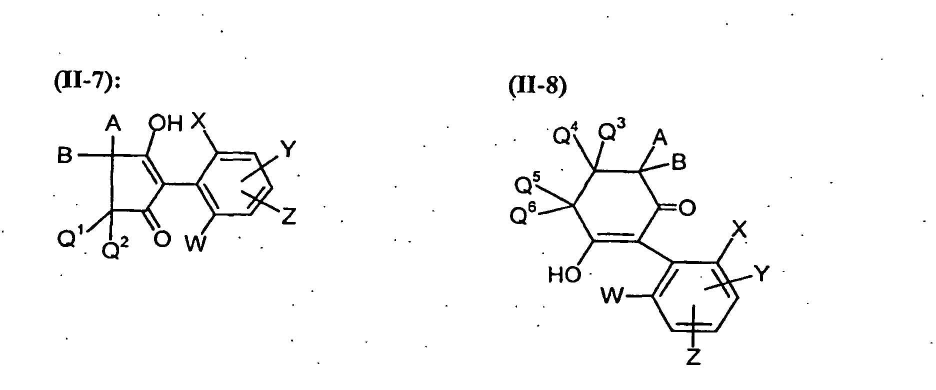 worin
A, B, D, Q¹, Q², Q³, Q⁴, Q⁵, Q⁶, W, X, Y und Z die oben angegebene Bedeutung haben,
mit Verbindungen der Formel (III)
J-L-M (III)
in welcher
L und M die oben angegebene Bedeutung haben und
J für eine Abgangsgruppe, wie Halogen, -O-SO₂-Haloalkyl, -O-SO₂-Alkyl oder -O-SO₂-Aryl steht,
in Gegenwart eines Verdünnungsmittels und in Gegenwart einer Base umsetzt.

6. Schädlingsbekämpfungsmittel und/oder Unkrautbekämpfungsmittel, **gekennzeichnet durch** einen Gehalt an mindestens einer Verbindung der Formel (I) gemäß Anspruch 1.

7. Verwendung von Verbindungen der Formel (I) gemäß Anspruch 1 zur Herstellung von Schädlingsbekämpfungsmitteln zur Bekämpfung von Schädlingen und Unkräutern.

8. Verfahren zur Herstellung von Schädlingsbekämpfungsmitteln zur Bekämpfung von Schädlingen und Unkräutern, **dadurch gekennzeichnet, daß** man Verbindungen der Formel (1) gemäß Anspruch 1 auf die Schädlinge, Pflanzen und/oder ihren Lebensraum einwirken läßt.

9. Verfahren zur Herstellung von Schädlingsbekämpfungsmitteln und/oder Unkrautbekämpfungsmitteln, **dadurch gekennzeichnet, daß** man Verbindungen der Formel (I) gemäß Anspruch 1 mit Streckmitteln und/oder oberflächenaktiven Mitteln vermischt.

10. Verwendung von Verbindungen der Formel (I) gemäß Anspruch 1 zur Herstellung von Schädlingsbekämpfungsmitteln und Herbiziden.

## Claims

1. Compounds of the formula (I) 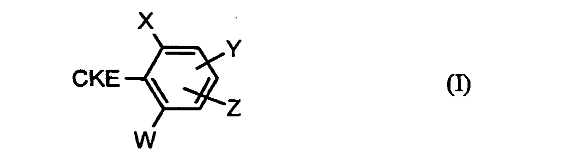 in which
W represents hydrogen, halogen, C₁-C₆-alkyl, C₁-C₆-alkoxy, C₁-C₆-halogenoalkyl, C₁-C₆-halogenoalkoxy, nitro or cyano,
X represents halogen, C₁-C₆-alkyl, C₁-C₆-halogenoalkyl, C₁-C₆-alkoxy, C₃-C₆-alkenyloxy, C₁-C₆-alkylthio, C₁-C₆-alkylsulphinyl, C₁-C₆-alkylsulphonyl, C₁-C₆-halogenoalkoxy, C₃-C₆-halogenoalkenyloxy, nitro, cyano or in each case optionally halogen-, C₁-C₆-alkyl-, C₁-C₆-alkoxy-, C₁-C₄-halogenoalkyl-, C₁-C₄-halogenoalkoxy-, nitro- or cyano-substituted phenyl, phenoxy, phenylthio, benzyloxy or benzylthio,
Y represents one of the radicals 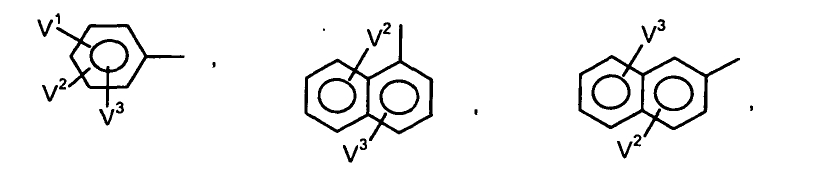 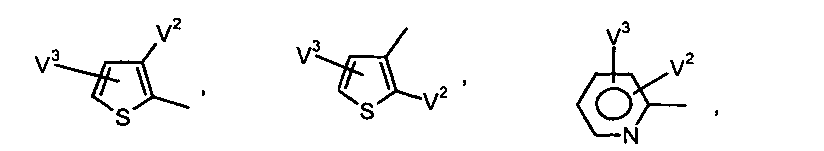 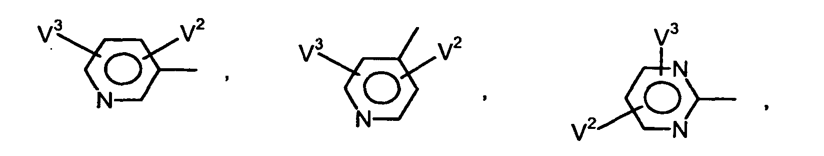 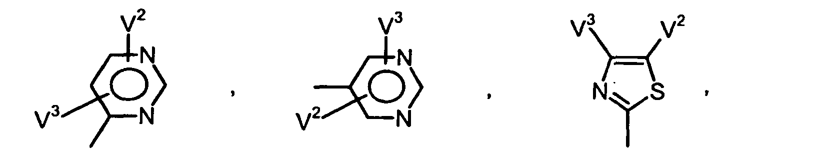 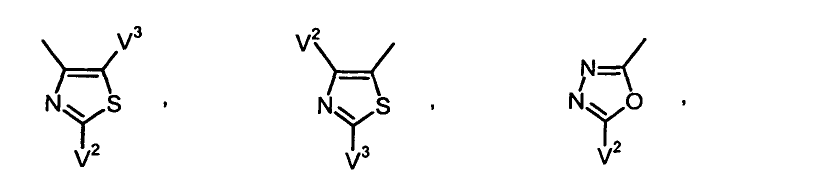 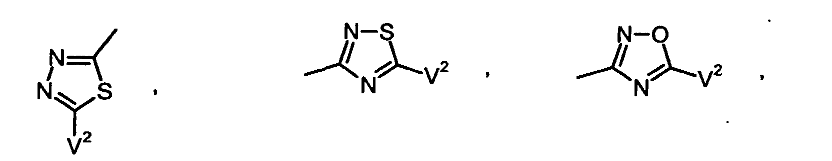 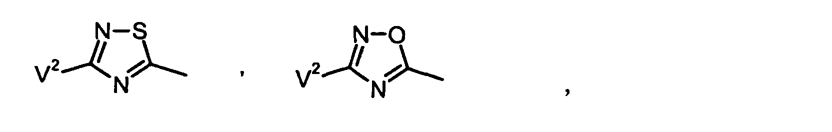
in which
V¹ represents hydrogen, halogen, C₁-C₁₂-alkyl, C₁-C₆-alkoxy, C₁-C₄-halogenoalkyl, C₁-C₄-halogenoalkoxy, nitro, cyano or phenyl, phenoxy, phenoxy-C₁-C₄-alkyl, phenyl-C₁-C₄-alkoxy, phenylthio-C₁-C₄-alkyl or phenyl-C₁-C₄-alkylthio, each of which is optionally mono- or polysubstituted by halogen, C₁-C₆-alkyl, C₁-C₆-alkoxy, C₁-C₄-halogenoalkyl, C₁-C₄-halogenoalkoxy, nitro or cyano,
V² and V³ independently of one another represent hydrogen, halogen, C₁-C₆-alkyl, C₁-C₆-alkoxy, C₁-C₄-halogenoalkyl or C₁-C₄-halogenoalkoxy,
Z represents hydrogen, halogen, C₁-C₆-alkyl, C₁-C₆-halogenoalkyl, C₁-C₆-alkoxy or C₁-C₆-halogenoalkoxy,
CKE represents one of the groups 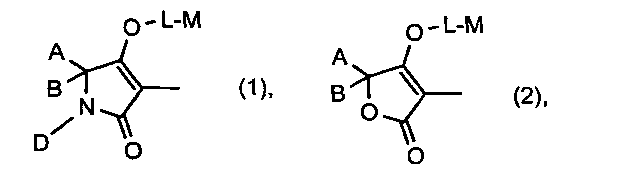 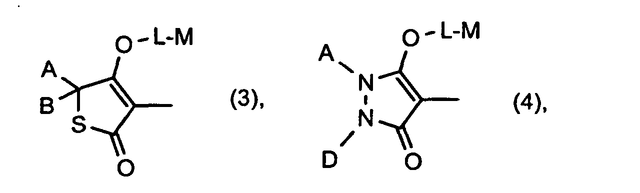 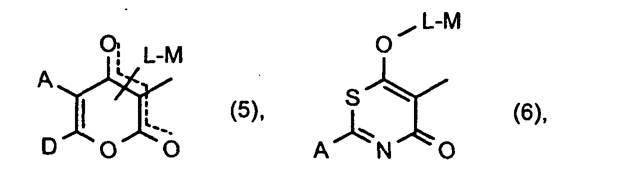 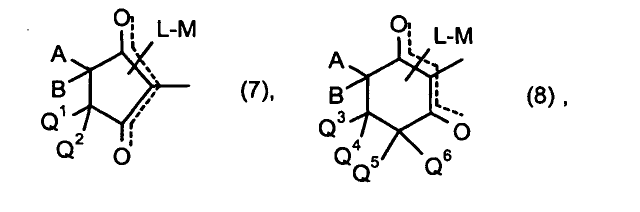 in which
A represents hydrogen or in each case optionally halogen-substituted C₁-C₁₂-alkyl, C₃-C₈-alkenyl, C₁-C₁₀-alkoxy-C₁-C₈-alkyl, di-, tri- or tetra-C₁-C₈-alkoxy-C₁-C₈-alkyl, C₁-C₁₀-alkylthio-C₁-C₆-alkyl, optionally halogen-, C₁-C₆-alkyl- or C₁-C₆-alkoxy-substituted C₃-C₈-cycloalkyl, in which optionally one or two not directly adjacent ring members are replaced by oxygen and/or sulphur, or represents in each case optionally halogen-, C₁-C₆-alkyl-, C₁-C₆-halogenoalkyl-, C₁-C₆-alkoxy-, C₁-C₆-halogenoalkoxy-, cyano- or nitro-substituted C₆- or C₁₀-aryl, hetaryl having 5 or 6 ring atoms or C₆- or C₁₀-aryl-C₁-C₆-alkyl,
B represents hydrogen, C₁-C₁₂-alkyl or C₁-C₈-alkoxy-C₁-C₆-alkyl, or
A, B and the carbon atom to which they are attached represent saturated C₃-C₁₀-cycloalkyl or unsaturated C₅-C₁₀-cycloalkyl in which optionally one ring member is replaced by oxygen or sulphur and which are optionally mono- or disubstituted by C₁-C₈-alkyl, C₃-C₁₀-cycloalkyl, C₁-C₈-halogenoalkyl, C₁-C₈-alkoxy, C₁-C₈-alkylthio, halogen or phenyl, or
A, B and the carbon atom to which they are attached represent C₃-C₆-cycloalkyl which is substituted by an alkylenediyl group which optionally contains one or two not directly adjacent oxygen and/or sulphur atoms or by an alkylenedioxyl or by an alkylenedithiol group which, together with the carbon atom to which they are attached, form a further five- to eight-membered ring, or
A, B and the carbon atom to which they are attached represent C₃-C₈-cycloalkyl or C₅-C₈-cycloalkenyl in which two substituents together with the carbon atoms to which they are attached represent in each case optionally C₁-C₆-alkyl-, C₁-C₆-alkoxy- or halogen-substituted C₂-C₆-alkanediyl, C₂-C₆-alkenediyl or C₄-C₆-alkanedienediyl in which optionally one methylene group is replaced by oxygen or sulphur,
D represents hydrogen, in each case optionally halogen-substituted C₁-C₁₂-alkyl, C₃-C₈-alkenyl, C₃-C₈-alkinyl, C₁-C₁₀-alkoxy-C₂-C₈-alkyl, poly-C₁-C₈-alkoxy-C₂-C₈-alkyl, C₁-C₁₀-alkylthio-C₂-C₈-alkyl, optionally halogen-, C₁-C₄-alkyl-, C₁-C₄-alkoxy- or C₁-C₄-halogenoalkyl-substituted C₃-C₈-cycloalkyl in which optionally one ring member is replaced by oxygen or sulphur or in each case optionally halogen-, C₁-C₆-alkyl-, C₁-C₆-halogenoalkyl-, C₁-C₆-alkoxy-, C₁-C₆-halogenoalkoxy-, cyano- or nitro-substituted phenyl, hetaryl having 5 or 6 ring atoms, phenyl-C ₁-C₆-alkyl or hetaryl-C ₁-C₆-alkyl having 5 or 6 ring atoms, or
A and D together represent in each case optionally substituted C₃-C₆-alkanediyl or C₃-C₆-alkenediyl in which optionally one methylene group is replaced by oxygen or sulphur, possible substituents being in each case:
halogen, hydroxyl, mercapto or in each case optionally halogen-substituted C₁-C₁₀-alkyl, C₁-C₆-alkoxy, C₁-C₆-alkylthio, C₃-C₇-cycloalkyl, phenyl or benzyloxy, or a further C₃-C₆-alkanediyl grouping, C₃-C₆-alkenediyl grouping or a butadienyl grouping, which is optionally substituted by C₁-C₆-alkyl or in which optionally two adjacent substituents together with the carbon atoms to which they are attached form a further saturated or unsaturated cycle having 5 or 6 ring atoms (in the case of the compound of the formula (I-1), A and D.then together with the atoms to which they are attached represent, for example, the groups AD-1 to AD-10 shown further below), which may contain oxygen or sulphur, or which optionally contains one of the groups below 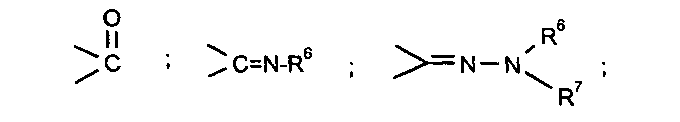 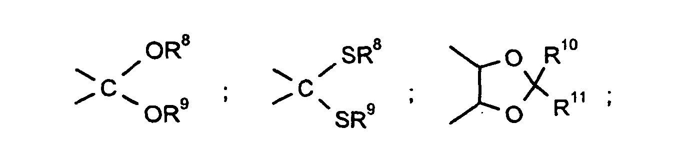 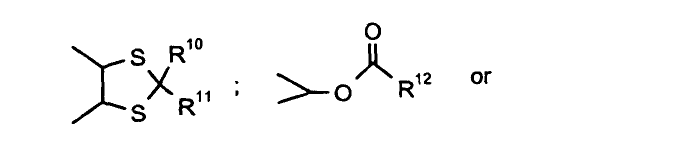 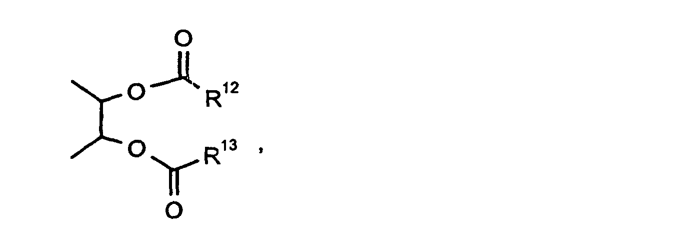 or
A and Q¹ together represent C₃-C₆-alkanediyl or C₄-C₆-alkenediyl, each of which is optionally mono- or disubstituted by identical or different substituents from the group consisting of halogen, hydroxyl, of C₁-C₁₀-alkyl, C₁-C₆-alkoxy, C₁-C₆-alkylthio, C₃-C₇-cycloalkyl, each of which is optionally mono- to tri-substituted by identical or different halogens, and benzyloxy or phenyl, each of which is optionally mono- to tri-substituted by identical or different substituents from the groups consisting of halogen, C₁-C₆-alkyl and C₁-C₆-alkoxy, which C₃-C₆-alkanediyl or C₄-C₆-alkenediyl furthermore optionally contains one of the groups below 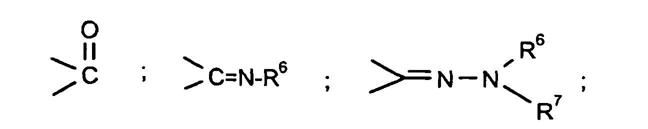 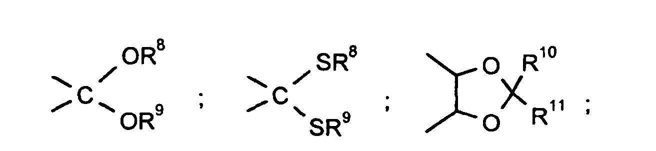 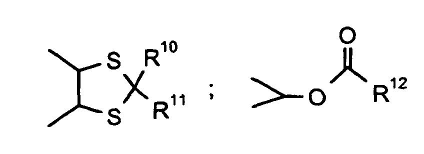 or 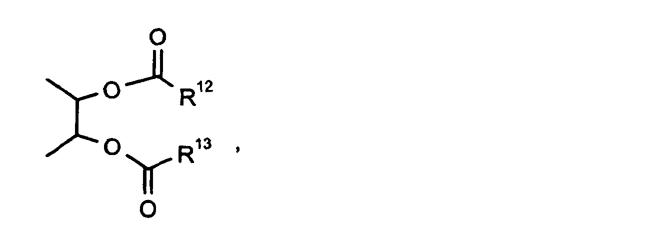 or is bridged by a C₁-C₂-alkanediyl group or by an oxygen atom or
Q¹ represents hydrogen or C₁-C₄-alkyl,
Q², Q⁴, Q⁵ and Q⁶ independently of one another represent hydrogen or C₁-C₄-alkyl,
Q³ represents hydrogen, C₁-C₆-alkyl, C₁-C₆-alkoxy-C₁-C₂-alkyl, C₁-C₆-alkylthio-C₁-C₂-alkyl, optionally C₁-C₄-alkyl- or C₁-C₄-alkoxy-substituted C₃-C₈-cycloalkyl, in which optionally one methylene group is replaced by oxygen or sulphur, or optionally halogen-, C₁-C₄-alkyl-, C₁-C₄-alkoxy-, C₁-C₂-halogenoalkyl-, C₁-C₂-halogenoalkoxy-, cyano- or nitro-substituted phenyl, or
Q³ and Q⁴ together with the carbon atom to which they are attached represent optionally C₁-C₄-alkyl-, C₁-C₄-alkoxy- or C₁-C₂-halogenoalkyl-substituted C₃-C₇-cycloalkyl in which optionally one ring member is replaced by oxygen or sulphur,
L represents an alkanediyl group having 1 to 6 carbon atoms,
M represents one of the groupings below: CN; 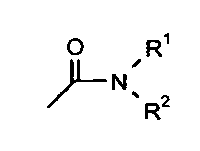 -CO₂R², -OR², -SR², -COR³, 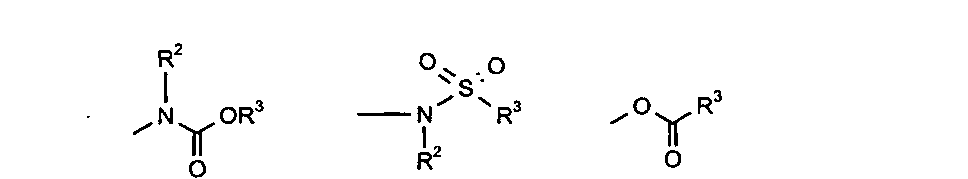 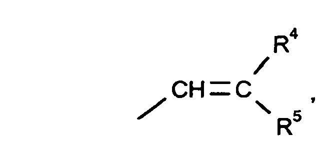 ―C≡C―R⁴ or 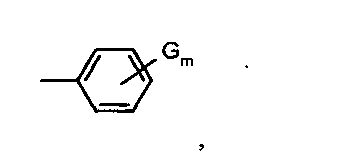
R¹ represents hydrogen or C₁-C₁₂-alkyl,
R² represents in each case optionally halogen-substituted C₁-C₁₂-alkyl, C₃-C₈-alkenyl, C₃-C₈-alkinyl, C₃-C₈-cycloalkyl, C₁-C₈-alkoxy-C₂-C₈-alkyl, C₁-C₈-alkylthio-C₂-C₈-alkyl, represents in each case optionally halogen-, nitro-, cyano-, C₁-C₆-alkoxy-, C₁-C₆-halogenoalkoxy-, C₁-C ₆-alkylthio-, C₁-C₆-halogenoalkylthio-, C₁-C₆-alkyl- or C₁-C₆-halogenoalkyl-substituted phenyl or benzyl,
R³ represents optionally halogen-substituted C₁-C₁₂-alkyl or represents in each case optionally halogen-, C₁-C₆-alkyl-, C₁-C₆-halogenoalkyl-, C ₁-C₆-alkoxy- or C₁-C₆-halogenoalkoxy-substituted phenyl or benzyl,
R⁴ represents hydrogen, halogen, optionally halogen-substituted C₁-C₆-alkyl or in optionally halogen-, C₁-C₆-alkyl-, C₁-C₆-alkoxyl-, C₁-C₄-halogenoalkyl-, C₁-C₄-halogenoalkoxy-, cyano- or nitro-substituted phenyl,
R⁵ represents hydrogen, halogen or optionally halogen-substituted C₁-C₆-alkyl,
G represents halogen, C₁-C₆-alkyl, C₁-C₆-alkoxy, C₁-C₄-halogenoalkyl, C₁-C₄-halogenoalkoxy, cyano or nitro,
m represents a number 0, 1, 2 or 3,
R⁶ represents hydrogen, represents in each case optionally halogen-substituted C₁-C₈-alkyl or C₁-C₈-alkoxy, represents optionally halogen-, C₁-C₄-alkyl- or C₁-C₄-alkoxy-substituted C₃-C₈-cycloalkyl, in which optionally one methylene group is replaced by oxygen or sulphur, or represents in each case optionally halogen-, C₁-C₆-alkyl-, C₁-C₆-alkoxy-, C₁-C₄-halogenoalkyl-, C₁-C₄-halogenoalkoxy-, nitro- or cyano-substituted phenyl, phenyl-C₁-C₄-alkyl or phenyl-C₁-C₄-alkoxy,
R⁷ represents hydrogen or C₁-C₈-alkyl, or
R⁶ and R⁷ together represent C₄-C₆-alkanediyl,
R⁸ and R⁹ are identical or different and represent C₁-C₆-alkyl, or
R⁸ and R⁹ together represent a C₂-C₄-alkanediyl radical which is optionally substituted by C₁-C₆-alkyl, C₁-C₆-halogenoalkyl or by optionally halogen-, C₁-C₆-alkyl-, C₁-C₄-halogenoalkyl-, C₁-C₆-alkoxy-, C₁-C₄-halogenoalkoxy-, nitro- or cyano-substituted phenyl,
R¹⁰ and R¹¹ independently of one another represent hydrogen, represent optionally halogen-substituted C₁-C₈-alkyl or represent optionally halogen-, C₁-C₆-alkyl-, C₁-C₆-alkoxy-, C₁-C₄-halogenoalkyl-, C₁-C₄-halogenoalkoxy-, nitro- or cyano-substituted phenyl, or
R¹⁰ and R¹¹ together with the carbon atom to which they are attached represent a carbonyl group or represent optionally halogen-, C₁-C₄-alkyl- or C₁-C₄-alkoxy-substituted C₅-C₇-cycloakyl in which optionally one methylene group is replaced by oxygen or sulphur,
R¹² and R¹³ independently of one another represent C₁-C₁₀-alkyl, C₂-C₁₀-alkenyl, C₁-C₁₀-alkoxy, C₁-C₁₀-alkylamino, C₃-C₁₀-alkenylamino, di-(C₁-C₁₀-alkyl)amino or di-(C₃-C₁₀-alkenyl)amino.

2. Compounds of the formula (I) according to Claim 1, in which
W represents hydrogen, fluorine, chlorine, bromine, C₁-C₄-alkyl, C₁-C₄-alkoxy, C₁-C₄-halogenoalkyl, C₁-C₄-halogenoalkoxy, nitro or cyano,
X represents fluorine, chlorine, bromine, C₁-C₄-alkyl, C₁-C₄-alkoxy, C₃-C₄-alkenyloxy, C₁-C₄-halogenoalkyl, C₁-C₄-halogenoalkoxy, C₃-C₄-halogenoalkenyloxy, nitro or cyano,
Y represents one of the radicals 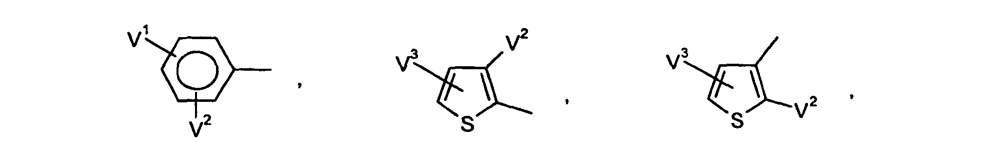 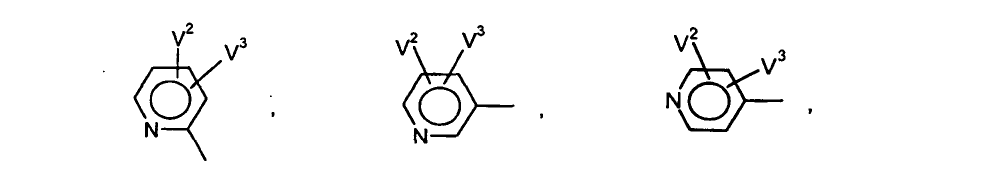 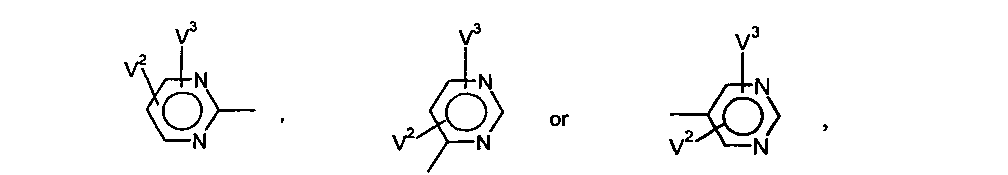 in which
V¹ represents hydrogen, fluorine, chlorine, bromine, C₁-C₆-alkyl, C₁-C₄-alkoxy, C₁-C₂-halogenoalkyl, C₁-C₂-halogenoalkoxy, nitro, cyano or represents phenyl, phenoxy, phenoxy-C₁-C₂-akyl, phenyl-C₁-C₂-alkoxy, phenylthio-C₁-C₂-akyl or phenyl-C₁-C₂-alkylthio, each of which is optionally mono- or disubstituted by fluorine, chlorine, bromine, C₁-C₄-alkyl, C₁-C₄-alkoxy, C₁-C₂-halogenoalkyl, C₁-C₂-halogenoalkoxy, nitro or cyano,
V² and V³ independently of one another represent hydrogen, fluorine, chlorine, bromine, C₁-C₄-alkyl, C₁-C₄-alkoxy, C₁-C₂-halogenoalkyl or C₁-C₂-halogenoalkoxy,
Z represents hydrogen, fluorine, chlorine, bromine, C₁-C₄-alkyl, C₁-C₄-halogenoalkyl, C₁-C₄-alkoxy or C₁-C₄-halogenoalkoxy,
CKE represents one of the groups 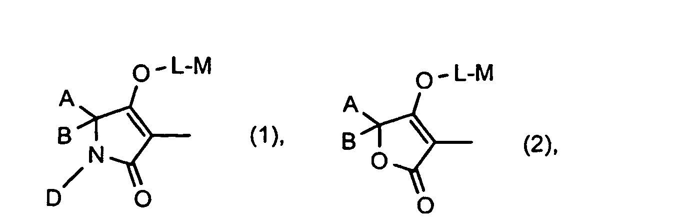 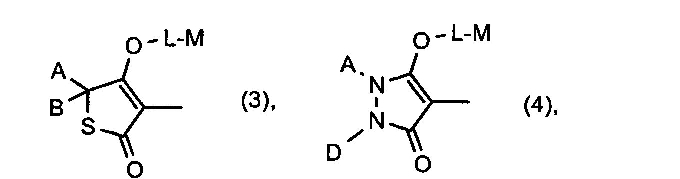 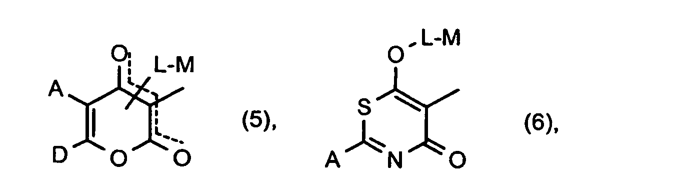 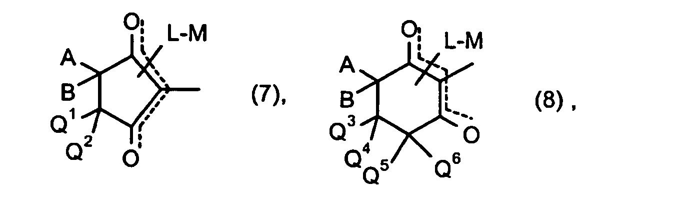
in which
A represents hydrogen, in each case optionally fluorine- or chlorine-substituted C₁-C₁₀-alkyl, C₁-C₈-alkoxy-C₁-C₆-alkyl, optionally fluorine-, chlorine-, C₁-C₄-alkyl- or C₁-C₄-alkoxy-substituted C₃-C₇-cycloalkyl, in which optionally one ring member is replaced by oxygen or sulphur, or (but not in the case of the compounds of the formulae (I-5), (1-7) and (I-8)) in each case optionally fluorine-, chlorine-, bromine-, C₁-C₄-alkyl-, C₁-C₄-halogenoalkyl-, C₁-C₄-alkoxy-, cyano-, nitro- or C₁-C₄-halogenoalkoxy-substituted phenyl, furanyl, pyridyl, imidazolyl, triazolyl, pyrazolyl, pyrimidyl, thiazolyl, thienyl or phenyl-C₁-C₄-alkyl,
B represents hydrogen or C₁-C₆-alkyl, or
A, B and the carbon atom to which they are attached represent saturated or unsaturated C₅-C₇-cycloalkyl in which optionally one ring member is replaced by oxygen or sulphur and which is optionally monosubstituted by C₁-C₆-alkyl, C₅-C₈-cycloalkyl, C₁-C₃-halogenoalkyl, C₁-C₆-alkoxy, fluorine, chlorine or phenyl, or
A, B and the carbon atom to which they are attached represent C₅-C₆-cycloalkyl which is substituted by an alkylenediyl group which optionally contains one or two not directly adjacent oxygen or sulphur atoms or by an alkylenedioxyl or an alkylenedithiol group which, together with the carbon atom to which they are attached, form a further 5- or 6-membered ring, or
A, B and the carbon atom to which they are attached represent C₃-C₆-cycloalkyl or C₅-C₆-cycloalkenyl in which two substituents together with the carbon atoms to which they are attached represent in each case optionally C₁-C₅-alkyl-, C₁-C₅-alkoxy-, fluorine-, chlorine- or bromine-substituted C₂-C₄-alkanediyl, C₂-C₄-alkenediyl, in which optionally one methylene group is replaced by oxygen or sulphur, or butyldienediyl,
D represents hydrogen, represents in each case optionally fluorine- or chlorine-substituted C₁-C₁₀-alkyl, C₃-C₆-alkenyl, C₁-C₈-alkoxy-C₂-C₆-alkyl or C₁-C₈-alkylthio-C₂-C₆-alkyl, represents optionally fluorine-, chlorine-, C₁-C₄-alkyl-, C₁-C₄-alkoxy- or C₁-C₂-halogenoalkyl-substituted C₃-C₇-cycloalkyl, in which optionally one methylene group is replaced by oxygen or sulphur, or (but not in the case of the compounds of the formulae (I-1) and (I-4)) represents in each case optionally fluorine-, chlorine-, bromine-, C₁-C₄-alkyl-, C₁-C₄-halogenoalkyl-, C₁-C₄-alkoxy- or C₁-C₄-halogenoalkoxy-substituted phenyl, furanyl, imidazolyl, pyridyl, thiazolyl, pyrazolyl, pyrimidyl, pyrrolyl, thienyl, triazolyl or phenyl-C₁-C₄-alkyl, or
A and D together represent optionally substituted C₃-C₅-alkanediyl in which one methylene group may be replaced by a carbonyl group, oxygen or sulphur, possible substituents being hydroxyl, C₁-C₆-alkyl and C₁-C₄-alkoxy, or
A and D (in the case of the compounds of the formula (I-1)) together with the atoms to which they are attached represent one of the groups AD-1 to AD-10: 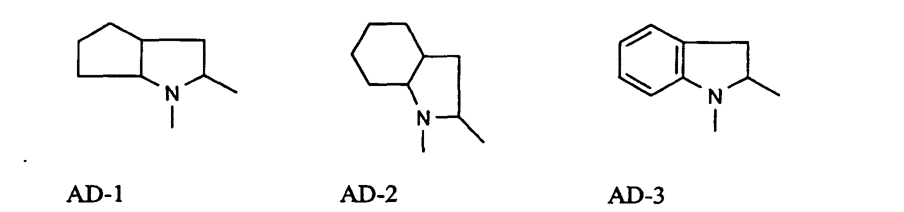 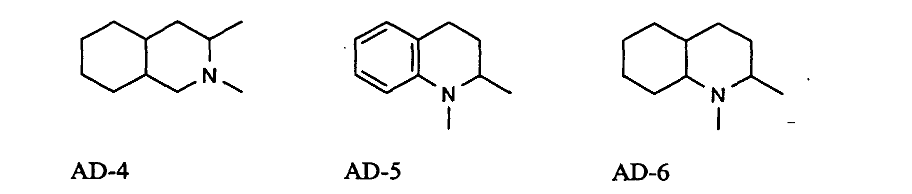 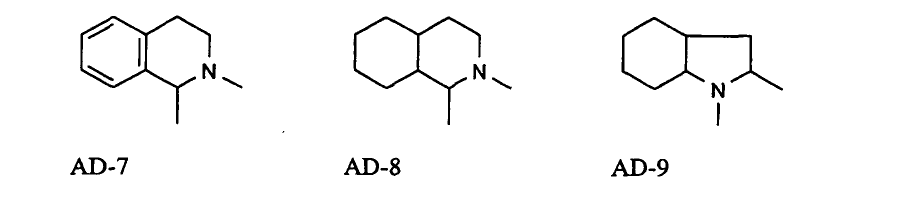 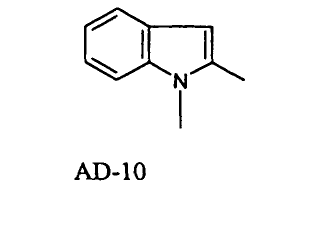 or
A and Q¹ together represent C₃-C₄-alkanediyl or C₃-C₄-alkenediyl, each of which is optionally mono- or disubstituted by identical or different substituents from the group consisting of fluorine, chlorine, hydroxyl and C₁-C₈-alkyl and C₁-C₄-alkoxy, each of which may optionally be mono- to trisubstituted by fluorine, or
Q¹ represents hydrogen,
Q² represents hydrogen,
Q⁴, Q⁵ and Q⁶ independently of one another represent hydrogen or C₁-C₃-alkyl,
Q³ represents hydrogen, C₁-C₄-alkyl, C₁-C₄-alkoxy-C₁-C₂-alkyl, C₁-C₄-alkylthio-C₁-C₂-alkyl or optionally methyl- or methoxy-substituted C₃-C₆-cycloalkyl in which optionally one methylene group is replaced by oxygen or sulphur, or
Q³ and Q⁴ together with the carbon atom to which they are attached represent optionally C₁-C₄-alkyl- or C₁-C₄-alkoxy-substituted saturated C₅-C₆-cycloalkyl in which optionally one ring member is replaced by oxygen or sulphur,
L represents an alkanediyl group having 1 to 4 carbon atoms,
M represents one of the groupings below: CN; 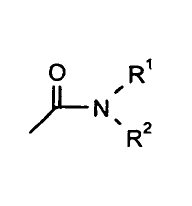 -CO₂R². -OR² , -SR², -COR³, 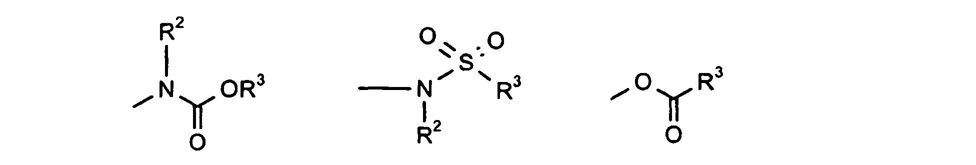 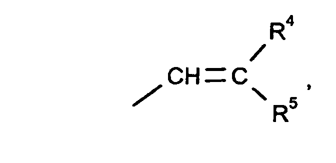 ―C≡C―R⁴ or 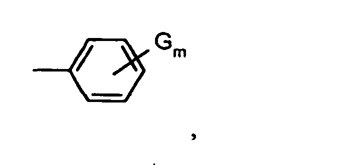
in which
R¹ represents hydrogen or C₁-C₁₀-alkyl,
R² represents in each case optionally fluorine-, chlorine-, bromine-substituted C₁-C₁₀-alkyl, C₃-C₆-alkenyl, C₃-C₆-alkinyl, C₃-C₈-cycloalkyl, C₁-C₆-alkoxy-C₂-C₆-alkyl, C₁-C₆-alkylthio-C₂-C₆-alkyl, represents in each case optionally fluorine-, chlorine-, bromine-, nitro-, cyano-, C₁-C₄-alkylthio-, C₁-C₄-alkoxy-, C₁-C₄-halogenoalkoxy-, C₁-C₄-halogenoalkylthio-, C₁-C₄-alkyl- or C₁-C₄-halogenoalkyl-substituted phenyl or benzyl,
R³ represents optionally fluorine-, chlorine-, bromine-substituted C₁-C₁₀-alkyl or represents in each case optionally fluorine-, chlorine-, C₁-C₄-alkyl-, C₁-C₄-halogenoalkyl-, C₁-C₄-alkoxy- or C₁-C₄-halogenoalkoxy-substituted phenyl or benzyl,
R⁴ represents hydrogen, fluorine, chlorine, bromine, optionally fluorine- or chlorine-substituted C₁-C₅-alkyl or optionally fluorine-, chlorine-, bromine-, C₁-C₄-alkyl-, C₁-C₄-alkoxy-, C₁-C₂-halogenoalkyl-, C₁-C₂-halogenoalkoxy, cyano- or nitro-substituted phenyl,
R⁵ represents hydrogen, fluorine, chlorine, bromine or optionally fluorine- or chlorine-substituted C₁-C₄-alkyl,
G represents fluorine, chlorine, bromine, C₁-C₄-alkyl, C₁-C₄-alkoxy, C₁-C₂-halogenoalkyl, C₁-C₂-halogenoalkoxy, cyano or nitro,
m represents a number 0, 1 or 2.

3. Compounds of the formula (I) according to Claim 1, in which
W represents hydrogen, chlorine, fluorine, methyl, ethyl, methoxy, ethoxy,
X represents fluorine, chlorine, methyl, ethyl, n-propyl, isopropyl, methoxy, ethoxy, n-propoxy, isopropoxy, trifluoromethyl, difluoromethoxy, trifluoromethoxy, nitro or cyano,
Y represents one of the radicals 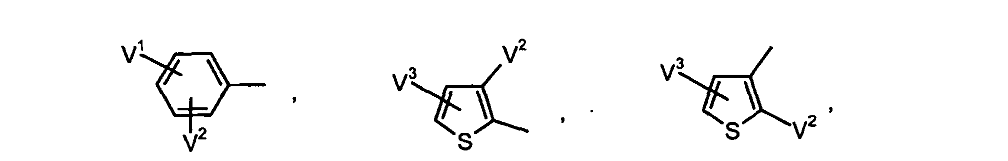 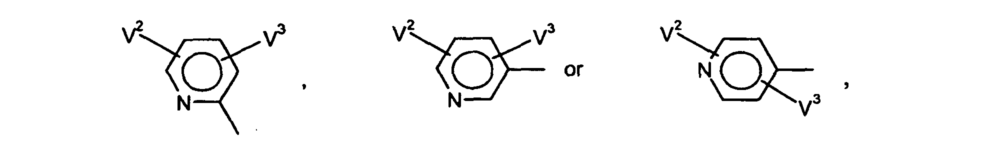 in which
V¹ represents hydrogen, fluorine, chlorine, bromine, methyl, ethyl, n-propyl, isopropyl, n-butyl, isobutyl, tert-butyl, methoxy, ethoxy, n-propoxy, isopropoxy, trifluoromethyl, trifluoromethoxy, nitro, cyano or phenyl,
V² and V³ independently of one another represent hydrogen, fluorine, chlorine, methyl, ethyl, n-propyl, isopropyl, methoxy, ethoxy, trifluoromethyl or trifluoromethoxy,
Z represents hydrogen, fluorine, chlorine, methyl or methoxy,
CKE represents one of the groups 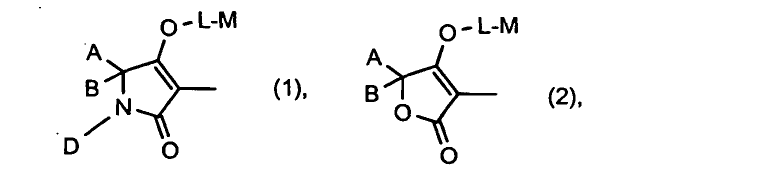 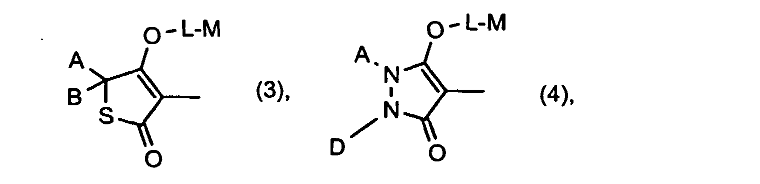 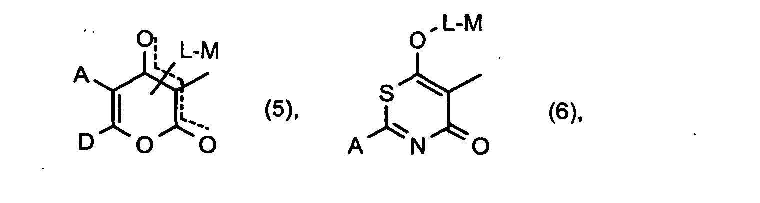 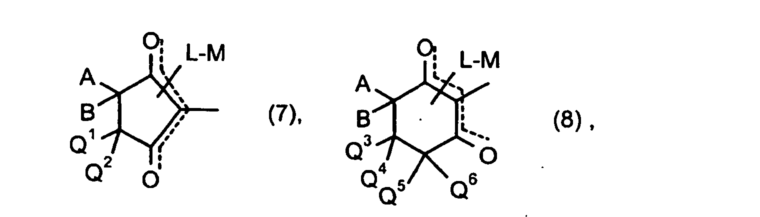 in which
A represents hydrogen, in each case optionally fluorine-substituted C₁-C₈-alkyl or C₁-C₆-alkoxy-C₁-C₄-alkyl, optionally fluorine-, methyl-, ethyl- or methoxy-substituted C₃-C₆-cycloalkyl, in which optionally one ring member is replaced by oxygen or sulphur, or (but not in the case of the compounds of formulae (I-5), (I-7) and (I-8)) represents in each case optionally fluorine-, chlorine-, bromine-, methyl-, ethyl-, n-propyl-, isopropyl-, methoxy-, ethoxy-, trifluoromethyl-, trifluoromethoxy-, cyano- or nitro-substituted phenyl or benzyl,
B represents hydrogen or C₁-C₄-alkyl, or
A, B and the carbon atom to which they are attached represent saturated C₅-C₆-cycloalkyl in which optionally one ring member is replaced by oxygen or sulphur and which is optionally monosubstituted by methyl, ethyl, n-propyl, isopropyl, butyl, isobutyl, sec-butyl, tert-butyl, trifluoromethyl, methoxy, ethoxy, n-propoxy, isopropoxy, n-butoxy, isobutoxy, sec-butoxy, tert-butoxy, fluorine or chlorine, or
A, B and the carbon atom to which they are attached represent C₅-C₆-cycloalkyl in which two substituents together with the carbon atoms to which they are attached represent C₂-C₄-alkanediyl or C₂-C₄-alkenediyl, in which in each case, optionally one methylene group is replaced by oxygen or sulphur, or butadienediyl,
D represents hydrogen, represents in each case optionally fluorine- or chlorine-substituted C₁-C₈-alkyl, C₃-C₄-alkenyl, C₁-C₆-alkoxy-C₂-C₄-alkyl, C₁-C₄-alkylthio-C₂-C₄-alkyl or C₃-C₆-cycloalkyl, in which optionally one methylene group is replaced by oxygen or sulphur, or (but not in the case of the compounds of formulae (I-1) and (I-4)) represents in each case optionally fluorine-, chlorine-, methyl-, ethyl-, n-propyl-, isopropyl-, methoxy-, ethoxy-, trifluoromethyl- or trifluoromethoxy-substituted phenyl, furanyl, pyridyl, thienyl or benzyl,
or
A and D together represent optionally substituted C₃-C₄-alkanediyl in which optionally one carbon atom is replaced by sulphur and which is optionally substituted by hydroxyl, methyl, ethyl, methoxy or ethoxy, or
A and D (in the case of the compounds of the formula (I-1)) together with the atoms to which they are attached represent one of the groups AD below: 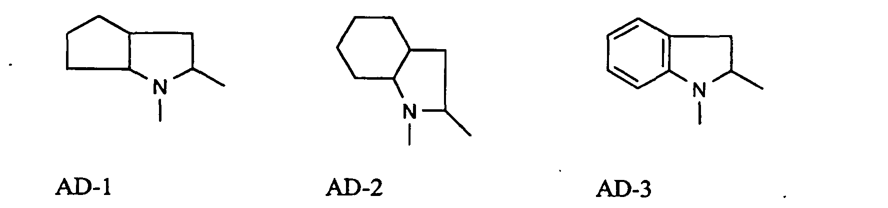 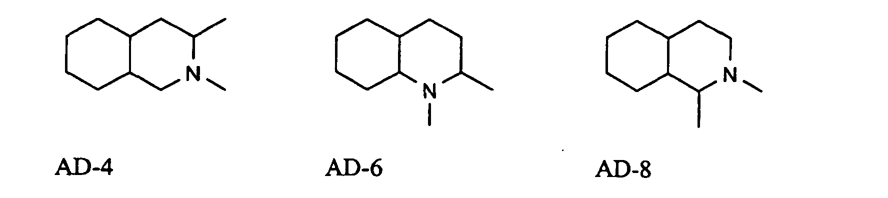 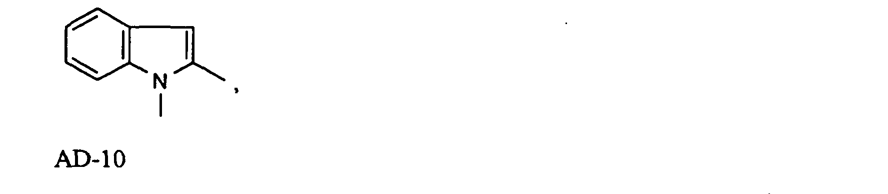
A and Q¹ together represent butenediyl or C₃-C₄-alkanediyl, optionally mono- or disubstituted by fluorine, hydroxyl, methyl or methoxy, or
Q¹ represents hydrogen,
Q² represents hydrogen,
Q⁴, Q⁵ and Q⁶ independently of one another represent hydrogen, methyl or ethyl,
Q³ represents hydrogen, methyl, ethyl or C₃-C₆-cycloalkyl, in which optionally one methylene group is replaced by oxygen or sulphur, or
Q³ and Q⁴ together with the carbon to which they are attached represent optionally methyl- or methoxy-substituted saturated C₅-C₆-cycloalkyl in which optionally one ring member is replaced by oxygen or sulphur,
L represents one of the groupings below -CH₂-, 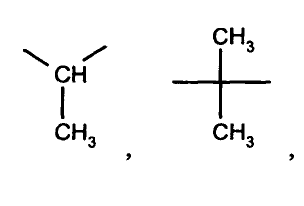 -CH₂-CH₂, 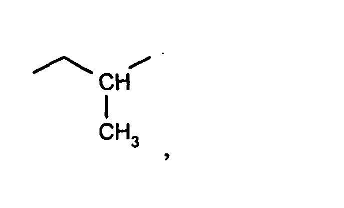
M represents one of the groupings below CN; 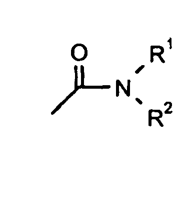 -CO₂R², -OR², -SR², -COR³, 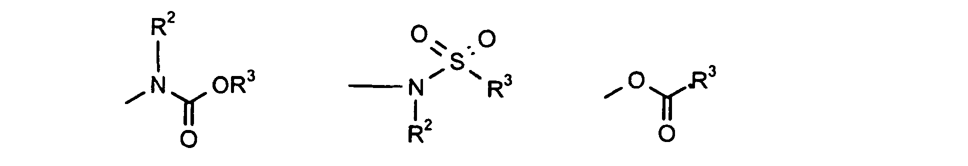 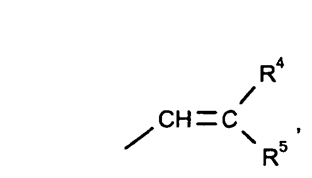 ―C≡C―R⁴ or 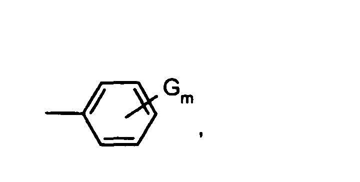 in which
R¹ represents hydrogen or C₁-C₈-alkyl,
R² represents in each case optionally fluorine-, chlorine-, bromine-substituted C₁-C₈-alkyl, C₃-C₄-alkenyl, C₃-C₄-alkinyl, cyclopropyl, cyclopentyl, cyclohexyl, C₁-C₆-alkoxy-C₂-C₄-alkyl, C₁-C₆-alkylthio-C₂-C₄-alkyl, represents in each case optionally fluorine-, chlorine-, bromine-, nitro-, cyano-, methylthio-, ethylthio-, methoxy-, ethoxy-, trifluoromethylthio-, trifluoromethoxy-, methyl-, ethyl-, trifluoromethyl-substituted phenyl or benzyl,
R³ represents optionally fluorine-, chlorine-, bromine-substituted C₁-C₈-alkyl or in each case optionally fluorine-, chlorine-, C₁-C₂-alkyl-, trifluoromethyl-, C₁-C₂-alkoxy- or trifluoromethoxy-substituted phenyl or benzyl,
R⁴ represents hydrogen, fluorine, chlorine, optionally fluorine- or chlorine-substituted C₁-C₄-alkyl or optionally fluorine-, chlorine-, methyl-, ethyl-, methoxy-, ethoxy-, trifluoromethyl-, difluoromethoxy-, trifluoromethoxy-, cyano- or nitro-substituted phenyl,
R⁵ represents hydrogen, fluorine, chlorine or in each case optionally fluorine- or chlorine-substituted methyl, ethyl, propyl or isopropyl,
G represents fluorine, chlorine, methyl, ethyl, propyl, isopropyl, methoxy, ethoxy, propoxy, isopropoxy, trifluoromethyl, difluoromethoxy, trifluoromethoxy, cyano or nitro,
m represents a number from 0 to 2.

4. Compounds of the formula (I) according to Claim 1, in which
W represents hydrogen, chlorine, fluorine, methyl, ethyl, methoxy, ethoxy,
X represents fluorine, chlorine, methyl, ethyl, n-propyl, isopropyl, methoxy, ethoxy, n-propoxy, isopropoxy, trifluoromethyl, difluoromethoxy, trifluoromethoxy, nitro or cyano,
Y represents one of the radicals 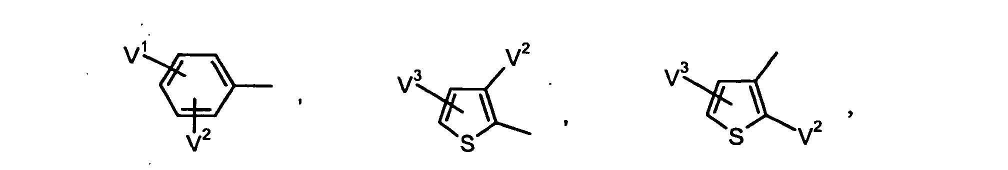 in which
V¹ represents hydrogen, fluorine, chlorine, methyl, ethyl, n-propyl, isopropyl, n-butyl, isobutyl, tert-butyl, methoxy, ethoxy, n-propoxy, isopropoxy, trifluoromethyl, trifluoromethoxy, nitro, cyano or phenyl,
V² represents hydrogen, fluorine, chlorine, methyl, methoxy, trifluoromethyl or trifluoromethoxy,
V³ represents hydrogen, methyl or chlorine,
Z represents hydrogen, fluorine, chlorine, methyl or methoxy,
CKE represents one of the groups 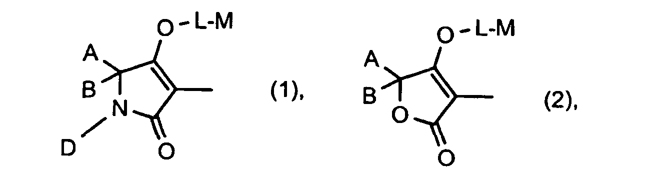 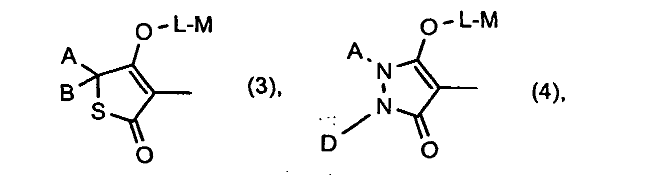 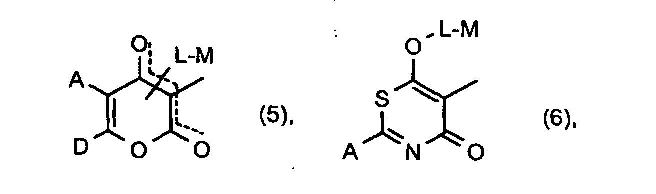 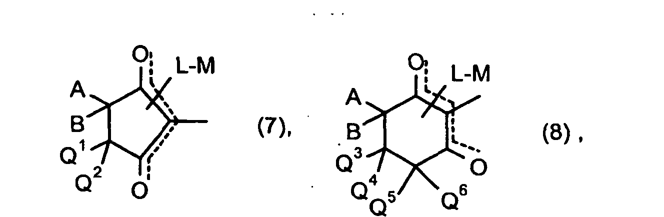 in which
A represents hydrogen, in each case optionally fluorine-substituted C₁-C₈-alkyl or C₁-C₆-alkoxy-C₁-C₄-alkyl, optionally fluorine-, methyl- or methoxy-substituted C₃-C₆-cycloalkyl, in which optionally one ring member is replaced by oxygen or sulphur, or (but not in the case of the compounds of the formulae (1-5), (I-7) and (I-8)) represents in each case optionally fluorine-, chlorine-, bromine-, methyl-, methoxy-, trifluoromethyl- or trifluoromethoxy-substituted phenyl or benzyl,
B represents hydrogen or C ₁-C₄-alkyl, or
A, B and the carbon atom to which they are attached represent saturated C₅-C₆-cycloalkyl in which optionally one ring member is replaced by oxygen or sulphur and which is optionally monosubstituted by methyl, ethyl, n-propyl, isopropyl, trifluoromethyl, methoxy, ethoxy, n-propoxy, isopropoxy, n-butoxy or isobutoxy, or
A, B and the carbon atom to which they are attached represent C₅-C₆-cycloalkyl in which two substituents together with the carbon atoms to which they attached represent C₂-C₄-alkanediyl or C₂-C₄-alkenediyl in which in each case optionally one methylene group is replaced by oxygen or sulphur, or butadienediyl,
D x represents hydrogen, represents in each case optionally fluorine- or chlorine-substituted C₁-C₆-alkyl, C₃-C₄-alkenyl, C₁-C₄-alkoxy-C₂-C₄-alkyl, C₁-C₄-alkylthio-C₂-C₄-alkyl or C₃-C₆-cycloalkyl, in which optionally one methylene group is replaced by oxygen or sulphur, or (but not in the case of the compounds of the formulae (I-1) and (I-4)), represents in each case optionally fluorine-, chlorine-, methyl-, methoxy-, trifluoromethyl- or trifluoromethoxy-substituted phenyl, pyridyl or benzyl,
or
A and D together represent optionally substituted C₃-C₄-alkanediyl in which optionally one carbon atom is replaced by sulphur and which is optionally substituted by methyl or methoxy,
A and Q¹ together represent butenediyl or C₃-C₄-alkanediyl, optionally mono- or disubstituted by methyl or methoxy, or
Q¹ represents hydrogen,
Q² represents hydrogen,
Q⁴, Q⁵ and Q⁶ independently of one another represent hydrogen, methyl or ethyl,
Q³ represents hydrogen, methyl, ethyl or C₃-C₆-cycloalkyl in which optionally one methylene group is replaced by oxygen or sulphur,
Q³ and Q⁴ together with the carbon to which they are attached represent optionally methyl- or methoxy-substituted saturated C₅-C₆-cycloalkyl in which optionally one ring member is replaced by oxygen or sulphur,
L represents one of the groupings below -CH₂-, 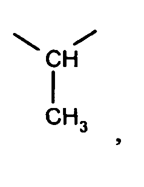 -CH₂-CH₂ ,
M represents one of the groupings below CN; 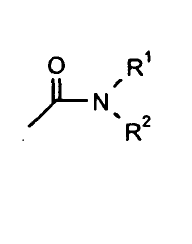 -CO₂R² , -OR², -SR², -CO_{R}³, 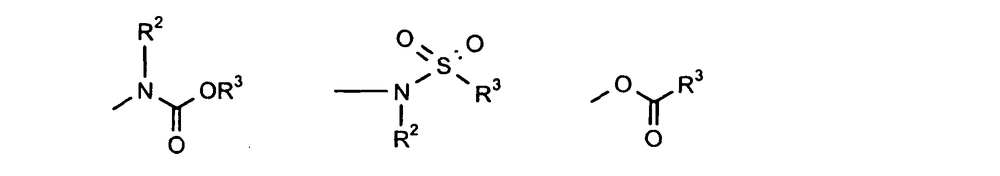 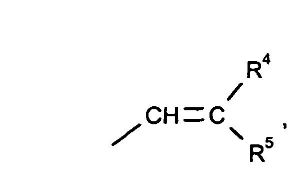 ―C≡C―R⁴ or 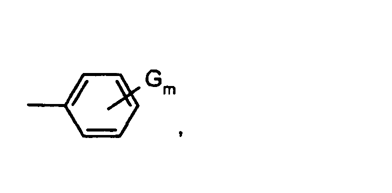
in which
R¹ represents hydrogen or C₁-C₄-alkyl,
R² represents in each case optionally fluorine-substituted C₁-C₄-alkyl, C₃-C₄-alkenyl, C₃-C₄-alkinyl, cyclopropyl, cyclopentyl, cyclohexyl, C₁-C₄-alkoxy-C₂-C₄-alkyl, represents in each case optionally fluorine-, chlorine-, bromine-, nitro-, cyano-, methylthio-, ethylthio-, methoxy-, ethoxy-, trifluoromethylthio, trifluoromethoxy-, methyl-, ethyl-, trifluoromethyl-substituted phenyl or benzyl,
R³ represents optionally fluorine-substituted C₁-C₄-alkyl or optionally fluorine-, chlorine-, methyl-, trifluoromethyl-, methoxy- or trifluoromethoxy-substituted phenyl,
R⁴ represents hydrogen, fluorine, chlorine, optionally fluorine-substituted C₁-C₄-alkyl or optionally fluorine-, chlorine-, methyl-, ethyl-, methoxy-, ethoxy-, trifluoromethyl-, difluoromethoxy-, trifluoromethoxy-, cyano- or nitro-substituted phenyl,
R⁵ represents hydrogen, fluorine, chlorine or optionally fluorine-substituted methyl,
G represents fluorine, chlorine, methyl, methoxy, trifluoromethyl, difluoromethoxy, trifluoromethoxy, cyano or nitro,
m represents a number from 0 to 1.

5. Process for preparing compounds of the formulae (I-1) to (I-8) according to Claim 1 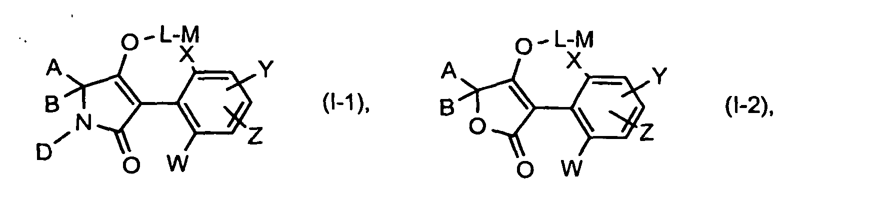 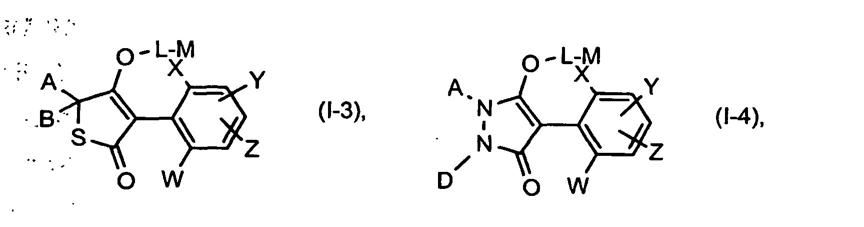 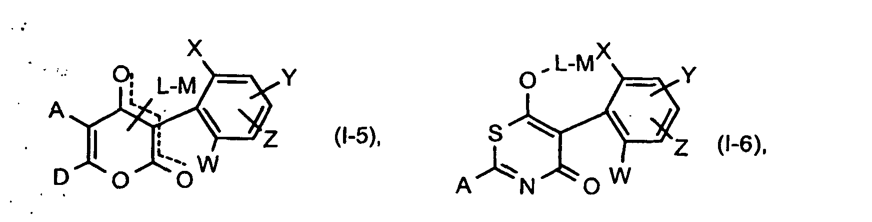 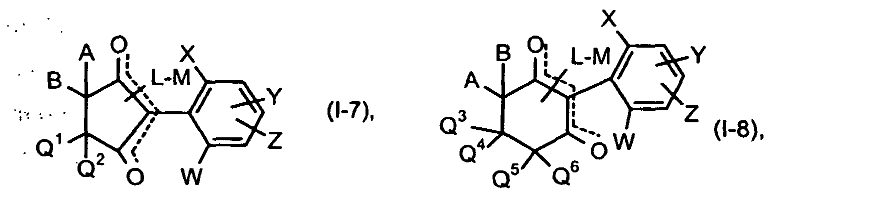 in which
A, B, D, L, M, Q¹, Q², Q³, Q⁴, Q⁵, Q⁶, W, X, Y and Z are as defined above,
**characterized in that** compounds of the formulae (II-1) to (II-8) 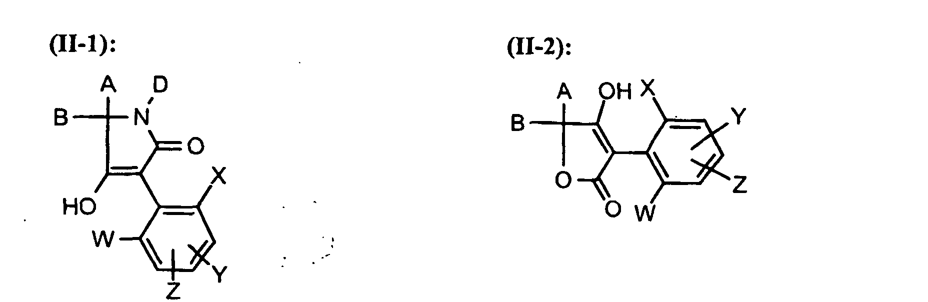 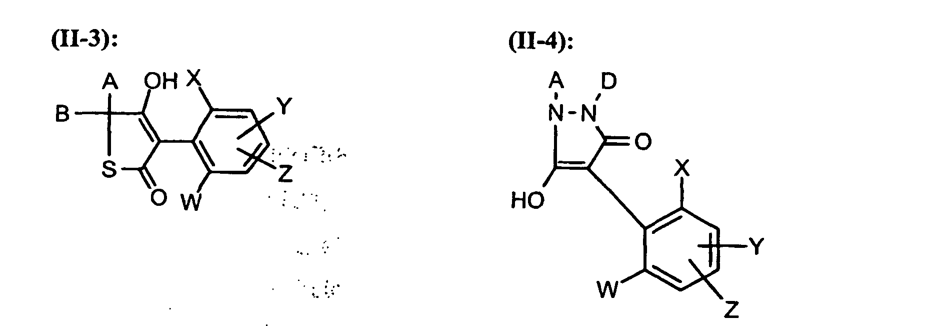 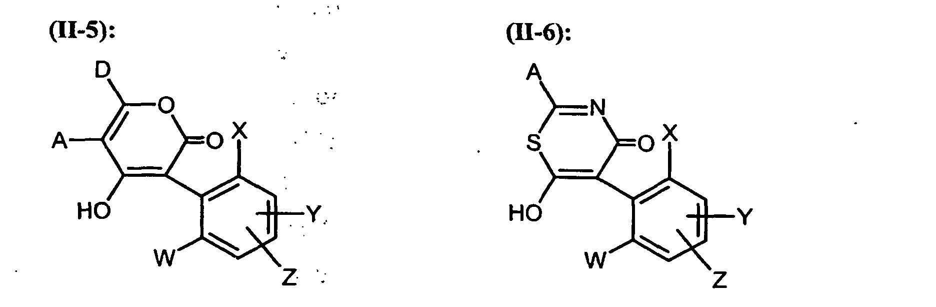 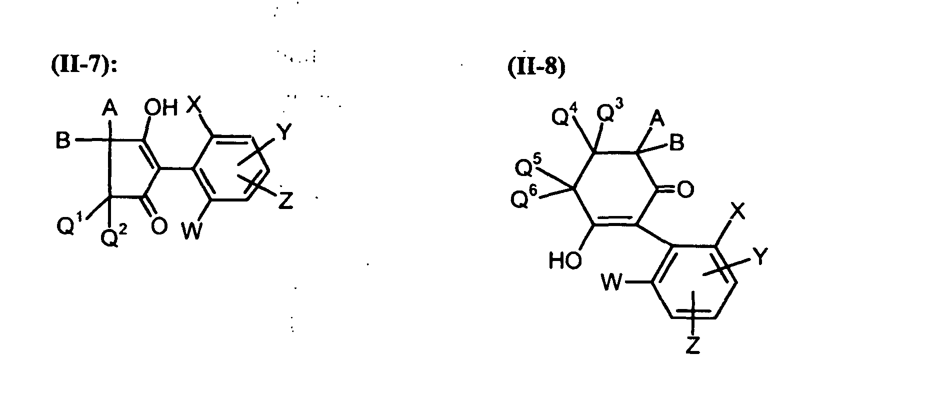 in which
A, B, D, Q¹, Q², Q³, Q⁴, Q⁵, Q⁶, W, X, Y and Z are as defined above,
are reacted with compounds of the formula (III)
J-L-M (III)
in which
L and M are as defined above and
J represents a leaving group, such as halogen, -O-SO₂-halogenoalkyl, -O-SO₂-alkyl or -O-SO₂-aryl,
in the presence of a diluent and in the presence of a base.

6. Pesticides and/or herbicides, **characterized in that** they comprise at least one compound of the formula (I) according to Claim 1.

7. Use of compounds of the formula (I) according to Claim 1 for producing pesticides for controlling pests and weeds.

8. Method for producing pesticides for controlling pests and weeds, **characterized in that** compounds of the formula (I) according to Claim 1 are allowed to act on the pests, plants and/or their habitat.

9. Process for preparing pesticides and/or herbicides, **characterized in that** compounds of the formula (I) according to Claim 1 are mixed with extenders and/or surfactants.

10. Use of compounds of the formula (I) according to Claim 1 for preparing pesticides and herbicides.

## Revendications

1. Composés de formule (I) 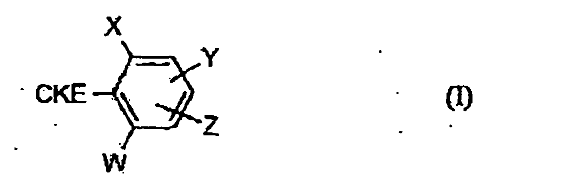 dans laquelle
W représente l'hydrogène, un halogène, un reste alkyle en C₁ à C₆, alkoxy en C₁ à C₆, halogénalkyle en C₁ à C₆, halogénalkoxy en C₁ à C₆, nitro ou cyano,
X représente un halogène, un reste alkyle en C₁ à C₆, halogénalkyle en C₁ à C₆, alkoxy en C₁ à C₆, alcényloxy en C₃ à C₆, alkylthio en C₁ à C₆, alkylsulfinyle en C₁ à C₆, alkylsulfonyle en C₁ à C₆, halogénalkoxy en C₁ à C₆, halogénalcényloxy en C₃ à C₆, nitro, cyano ou un reste phényle, phénoxy, phénylthio, benzyloxy ou benzylthio, chacun éventuellement substitué par un radical halogéno, alkyle en C₁ à C₆, alkoxy en C₁ à C₆, halogénalkyle en C₁ à C₄, halogénalkoxy en C₁ à C₄, nitro ou cyano,
Y représente l'un des restes 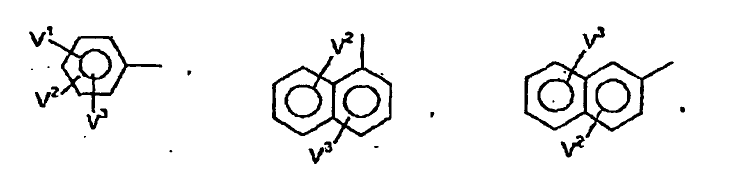 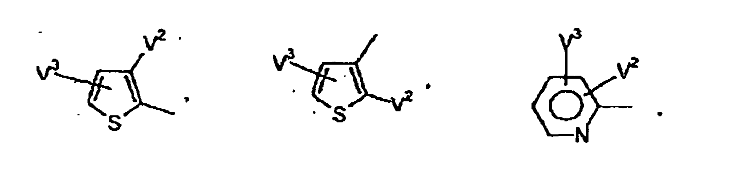 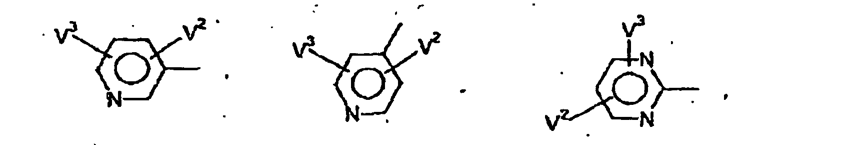 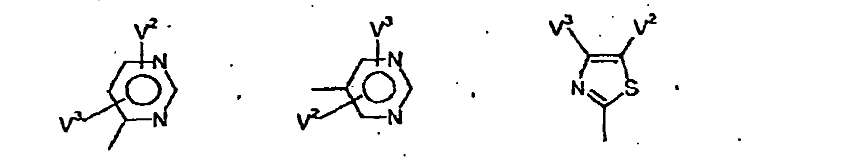 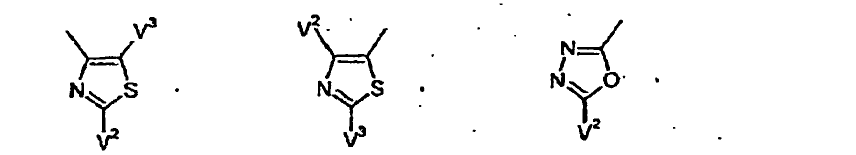 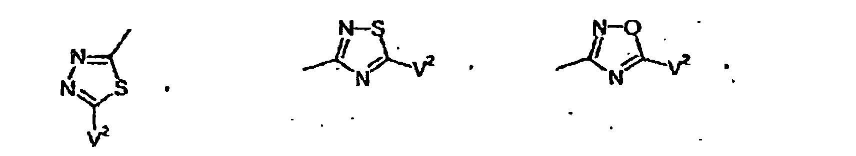 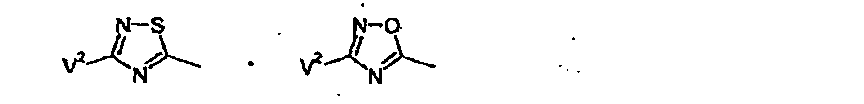
V¹ représente l'hydrogène, un halogène, un reste alkyle en C₁ à C₁₂, alkoxy en C₁ à C₆, halogénalkyle en C₁ à C₄, halogénalkoxy en C₁ à C₄, nitro, cyano ou un reste phényle, phénoxy, phénoxy-(alkyle en C₁ à C₄), phényl(alkoxy en C₁ à C₄), phénylthio- (alkyle en C₁ à C₄) ou phényl-(alkylthio en C₁ à C₄), chacun éventuellement substitué une ou plusieurs fois par un radical halogéno, alkyle en C₁ à C₆, alkoxy en C₁ à C₆, halogénalkyle en C₁ à C₄, halogénalkoxy en C₁ à C₄, nitro ou cyano,
V² et V³ représentent, indépendamment l'un de l'autre, l'hydrogène, un halogène, un reste alkyle en C₁ à C₆, alkoxy en C₁ à C₆, halogénalkyle en C₁ à C₄ ou halogénalkoxy en C₁ à C₄,
Z représente l'hydrogène, un halogène, un reste alkyle en C₁ à C₆, halogénalkyle en C₁ à C₆, alkoxy en C₁ à C₆ ou halogénalkoxy en C₁ à C₆,
CKE désigne l'un des groupes 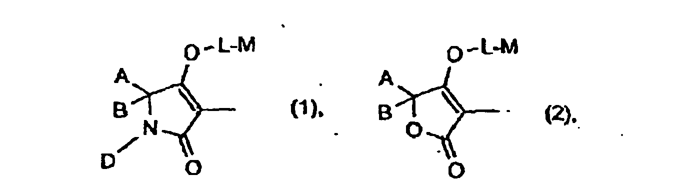 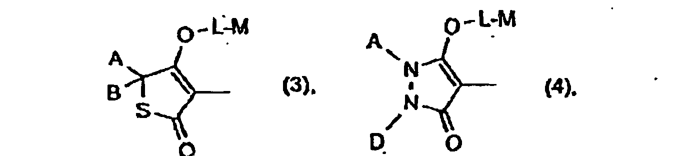 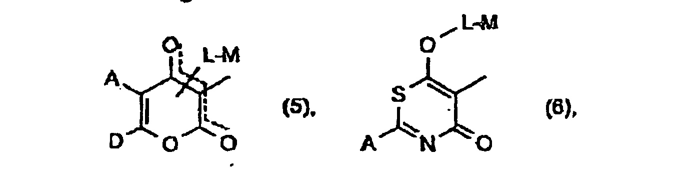 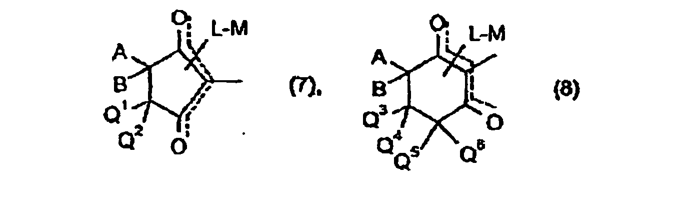
A représente l'hydrogène ou un reste alkyle en C₁ à C₁₂, alcényle en C₃ à C₈, (alkoxy en C₁ à C₁₀) - (alkyle en C₁ à C₈), di-, tri- ou tétra(alkoxy en C₁ à C₈)-(alkyle en C₁ à C₈), (alkylthio en C₁ à C₁₀) - (alkyle en C₁ à C₆), chacun éventuellement substitué par un halogène, un reste cycloalkyle en C₃ à Ce éventuellement substitué par un radical halogéno, alkyle en C₁ à C₆ ou alkoxy en C₁ à C₆ et dans lequel, le cas échéant, un ou deux chaînons du noyau non directement contigus sont remplacés par l'oxygène et/ou le soufre, ou un reste aryle en C₆ ou C₁₀, hétaryle pentagonal ou hexagonal ou (aryle en C₆ ou C₁₀)-(alkyle en C₁ à C₆), chacun éventuellement substitué par un radical halogéno, alkyle en C₁ à C₆, halogénalkyle en C₁ à C₆, alkoxy en C₁ à C₆, halogénalkoxy en C₁ à C₆, cyano ou nitro,
B représente l'hydrogène, un reste alkyle en C₁ à C₁₂ ou (alkoxy en C₁ à C₈)- (alkyle en C₁ à C₆), ou bien
A, B et l'atome de carbone auquel ils sont liés représentent un reste cycloalkyle en C₃ à C₁₀ saturé ou un reste cycloalkyle en C₅ à C₁₀ non saturé, où le cas échéant, un chaînon du noyau est remplacé par de l'oxygène ou du soufre ou qui sont éventuellement substitués une ou deux fois par un radical alkyle en C₁ à C₈, cycloalkyle en C₃ à C₁₀, halogénalkyle en C₁ à C₈, alkoxy en C₁ à Ce, alkylthio en C₁ à C₈, halogéno ou phényle, ou bien
A, B et l'atome de carbone auquel ils sont liés représentent un reste cycloalkyle en C₃ à C₆ qui est substitué par un groupe alkylènediyle comprenant éventuellement un ou deux atomes d'oxygène et/ou de soufre non directement contigus ou par un groupe alkylènedioxyle ou par un groupe alkylènedithioyle, formant avec l'atome de carbone auquel il est lié un autre noyau pentagonal à octogonal, ou bien
A, B et l'atome de carbone auquel ils sont liés représentent un reste cycloalkyle en C₃ à C₈ ou un reste cycloalcényle en C₅ à C₈, dans lesquels deux substituants forment, conjointement avec les atomes de carbone auxquels ils sont liés, un reste alcanediyle en C₂ à C₆, alcènediyle en C₂ à C₆ ou alcanediènediyle en C₄ à C₆, chacun éventuellement substitué par un radical alkyle en C₁ à C₆, alkoxy en C₁ à C₆ ou halogéno, où le cas échéant, un groupe méthylène est remplacé par de l'oxygène ou du soufre,
D représente l'hydrogène, un reste alkyle en C₁ à C₁₂, alcényle en C₃ à Ce, alcynyle en C₃ à C₈, (alkoxy en C₁ à C₁₀) - (alkyle en C₂ à Ce), poly(alkoxy en C₁ à C₈)-(alkyle en C₂ à C₈), (alkylthio en C₁ à C₁₀) - (alkyle en C₂ à C₈), chacun éventuellement substitué par un halogène, un reste cycloalkyle en C₃ à C₈ éventuellement substitué par un radical halogéno, alkyle en C₁ à C₄, alkoxy en C₁ à C₄ ou halogénalkyle en C₁ à C₄, dans lequel, le cas échéant, un chaînon du noyau est remplacé par de l'oxygène ou du soufre, ou bien un reste phényle, hétaryle pentagonal ou hexagonal, phényl-(alkyle en C₁ à C₆) ou hétazyl(alkyle en C₁ à C₆) pentagonal ou hexagonal, chacun éventuellement substitué par un radical halogéno, alkyle en C₁ à C₆, halogénalkyle en C₁ à C₆, alkoxy en C₁ à C₆, halogénalkoxy en C₁ à C₆, cyano ou nitro, ou bien
A et D forment ensemble un reste alcanediyle en C₃ à C₆ ou alcènediyle en C₃ à C₆, chacun éventuellement substitué, où, le cas échéant, un groupe méthylène est remplacé par de l'oxygène ou du soufre et en considérant dans chaque cas les substituants suivants :
halogéno, hydroxy, mercapto ou alkyle en C₁ à C₁₀, alkoxy en C₁ à C₆, alkylthio en C₁ à C₆, cycloalkyle en C₃ à C₇, phényle ou benzyloxy, chacun éventuellement substitué par un halogène, ou un autre groupement alcanediyle en C₃ à C₆, groupement alcènediyle en C₃ à C₆ ou un groupement butadiényle, qui est éventuellement substitué par un radical alkyle en C₁ à C₆ où dans lequel, le cas échéant, deux substituants voisins forment avec les atomes de carbone auxquels ils sont liés un autre cycle saturé ou non saturé pentagonal ou hexagonal (dans le cas du composé de formule (I-1), A et D forment alors, conjointement avec les atomes auxquels ils sont liés, par exemple les groupes AD-1 à AD-10 mentionnés plus loin), qui peut contenir de l'oxygène ou du soufre ou qui contient le cas échéant l'un des groupes suivants 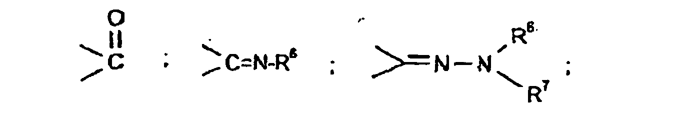 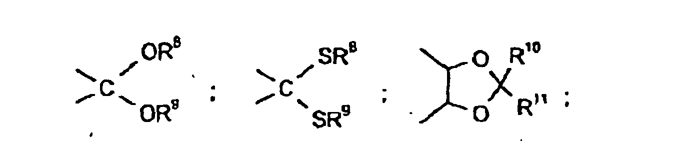 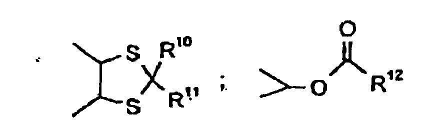 ou 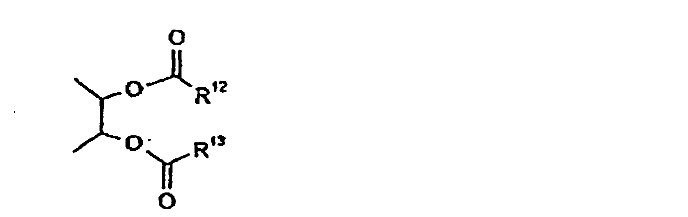
ou bien
A et Q¹ forment ensemble un reste alcanediyle en C₃ à C₆ ou alcènediyle en C₄ à C₆, chacun éventuellement substitué une ou deux fois identiques ou différentes par un radical halogéno, hydroxy, par un radical alkyle en C₁ à C₁₀, alkoxy en C₁ à C₆, alkylthio en C₁ à C₆, cycloalkyle en C₃ à C₇, chacun éventuellement substitué une à trois fois identiques ou différentes par un halogène, ou par un reste benzyloxy ou phényle, chacun éventuellement substitué une à trois fois identiques ou différentes par un radical halogéno, alkyle en C₁ à C₆ ou alkoxy en C₁ à C₆, ce reste contenant en outre le cas échéant l'un des groupes suivants 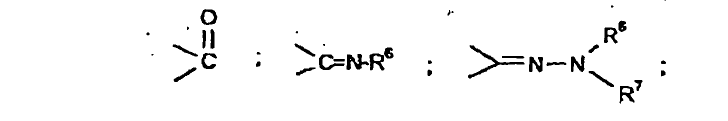 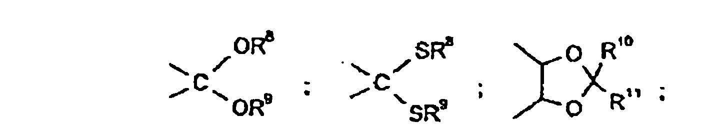 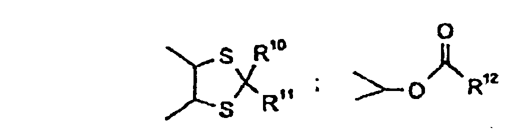 ou 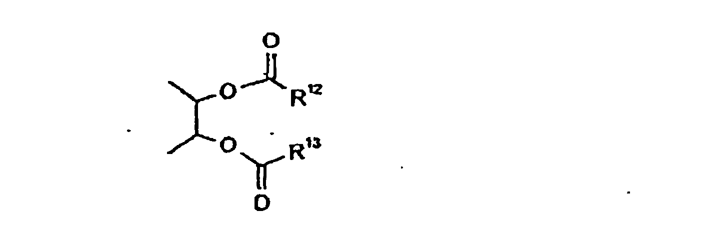 ou étant ponté par un groupe alcanediyle en C₁ ou C₂ ou par un atome d'oxygène, ou bien
Q¹ représente l'hydrogène ou un reste alkyle en C₁ à C₄,
Q², Q⁴, Q⁵ et Q⁶ représentent indépendamment les uns des autres l'hydrogène ou un reste alkyle en C₁ à C₄,
Q³ représente l'hydrogène, un reste alkyle en C₁ à C₆, (alkoxy en C₁ à C₆)-(alkyle en C₁ ou C₂), (alkylthio en C₁ à C₆) - (alkyle en C₁ ou C₂), un reste cycloalkyle en C₃ à C₈ éventuellement substitué par un radical alkyle en C₁ à C₄ ou alkoxy en C₁ à C₄ et dans lequel, le cas échéant, un groupe méthylène est remplacé par de l'oxygène ou du soufre, ou représente un reste phényle éventuellement substitué par un radical halogéno, alkyle en C₁ à C₄, alkoxy en C₁ à C₄, halogénalkyle en C₁ ou C₂, halogénalkoxy en C₁ ou C₂, cyano ou nitro,
Q³ et Q⁴ forment, conjointement avec l'atome de carbone auquel ils sont liés, un reste cycloalkyle en C₃ à C₇ éventuellement substitué par un radical alkyle en C₁ à C₄, alkoxy en C₁ à C₄ ou halogénalkyle en C₁ ou C₂ dans lequel, le cas échéant, un chaînon du noyau est remplacé par de l'oxygène ou du soufre,
L représente un groupe alcanediyle ayant 1 à 6 atomes de carbone,
M représente l'un des groupements suivants : CN; 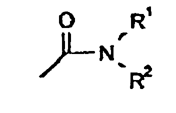 -CO₂R², -OR², -SR², -COR\ 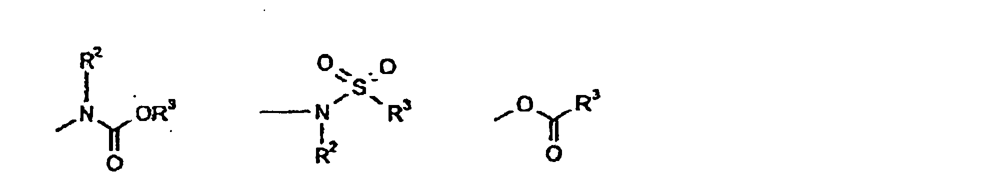 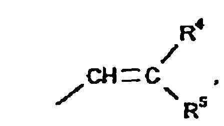 ―C≡C―R⁴ ou 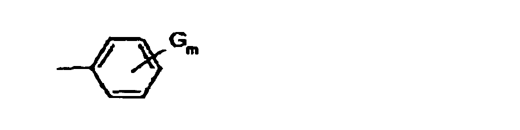
R¹ représente l'hydrogène ou un reste alkyle en C₁ à C₁₂,
R² représente un reste alkyle en C₁ à C₁₂, alcényle en C₃ à C₈, alcynyle en C₃ à C₈, cycloalkyle en C₃ à C₈, (alkoxy en C₁ à C₈) - (alkyle en C₂ à C₈), (alkylthio en C₁ à C₈)-(alkyle en C₂ à C₈), chacun éventuellement substitué par un halogène, un reste phényle ou benzyle, chacun éventuellement substitué par un radical halogéno, nitro, cyano, alkoxy en C₁ à C₆, halogénalkyle en C₁ à C₆, alkylthio en C₁ à C₆, halogénalkylthio en C₁ à C₆, alkyle en C₁ à C₆ ou halogénalkyle en C₁ à C₆,
R³ représente un reste alkyle en C₁ à C₁₂ éventuellement substitué par un halogène ou un reste phényle ou benzyle, chacun éventuellement substitué par un radical halogéno, alkyle en C₁ à C₆, halogénalkyle en C₁ à C₆, alkoxy en C₁ à C₆ ou halogénalkoxy en C₁ à C₆,
R⁴ représente l'hydrogène, un halogène, un reste alkyle en C₁ à C₆ éventuellement substitué par un halogène ou un reste phényle éventuellement substitué par un radical halogéno, alkyle en C₁ à C₆, alkoxyle en C₁ à C₆, halogénalkyle en C₁ à C₄, halogénalkoxy en C₁ à C₄, cyano ou nitro,
R⁵ représente l'hydrogène, un halogène ou un reste alkyle en C₁ à C₆ éventuellement substitué par un halogène,
G représente un halogène, un reste alkyle en C₁ à C₆, alkoxy en C₁ à C₆, halogénalkyle en C₁ à C₄, halogénalkoxy en C₁ à C₄, cyano ou nitro,
m représente le nombre 0, 1, 2 ou 3,
R⁶ représente l'hydrogène, un reste alkyle en C₁ à C₈ ou alkoxy en C₁ à C₈, chacun éventuellement substitué par un halogène, un reste cycloalkyle en C₃ à C₈ éventuellement substitué par un radical halogéno, alkyle en C₁ à C₄ ou alkoxy en C₁ à C₄ et dans lequel, le cas échéant, un groupe méthylène est remplacé par l'oxygène ou le soufre, ou bien un reste phényle, phényl- (alkyle en C₁ à C₄) ou phényl-(alkoxy en C₁ à C₄), chacun éventuellement substitué par un radical halogéno, alkyle en C₁ à C₆, alkoxy en C₁ à C₆, halogénalkyle en C₁ à C₄, halogénalkoxy en C₁ à C₄, nitro ou cyano,
R⁷ représente l'hydrogène ou un reste alkyle en C₁ à C₈, ou bien
R⁶ et R⁷ forment ensemble un reste alcanediyle en C₄ à C₆,
R⁸ et R⁹ sont identiques ou différents et représentent un reste alkyle en C₁ à C₆, ou bien
R⁸ et R⁹ forment ensemble un reste alcanediyle en C₂ à C₄ qui est éventuellement substitué par un radical alkyle en C₁ à C₆, halogénalkyle en C₁ à C₆ ou par un radical phényle éventuellement substitué par un radical halogéno, alkyle en C₁ à C₆, halogénalkyle en C₁ à C₄, alkoxy en C₁ à C₆, halogénalkoxy en C₁ à C₄, nitro ou cyano,
R¹⁰ et R¹¹ représentent indépendamment l'un de l'autre l'hydrogène, un reste alkyle en C₁ à C₆ éventuellement substitué par un halogène ou un reste phényle éventuellement substitué par un radical halogéno, alkyle en C₁ à C₆, alkoxy en C₁ à C₆, halogénalkyle en C₁ à C₄, halogénalkoxy en C₁ à C₄, nitro ou cyano, ou bien
R¹⁰ et R¹¹ forment, conjointement avec l'atome de carbone auquel ils sont liés, un groupe carbonyle ou un reste cycloalkyle en C₅ à C₇ éventuellement substitué par un radical halogéno, alkyle en C₁ à C₄ ou alkoxy en C₁ à C₄ et dans lequel, le cas échéant, un groupe méthylène est remplacé par de l'oxygène ou du soufre,
R¹² et R¹³ représentent indépendamment l'un de l'autre un reste alkyle en C₁ à C₁₀, alcényle en C₂ à C₁₀, alkoxy en C₁ à C₁₀, alkylamino en C₁ à C₁₀, alcénylamino en C₃ à C₁₀, di(alkyle en C₁ à C₁₀) amino ou di (alcényle en C₃ à C₁₀)amino.

2. Composés de formule (I) suivant la revendication 1, formule dans laquelle
W représente l'hydrogène, le fluor, le chlore, le brome, un reste alkyle en C₁ à C₄, alkoxy en C₁ à C₄, halogénalkyle en C₁ à C₄, halogénalkoxy en C₁ à C₄, nitro ou cyano,
X représente le fluor, le chlore, le brome, un reste alkyle en C₁ à C₄, alkoxy en C₁ à C₄, alcényloxy en C₃ ou C₄, halogénalkyle en C₁ à C₄, halogénalkoxy en C₁ à C₄, halogénalcényloxy en C₃ ou C₄, nitro ou cyano,
Y représente l'un des restes 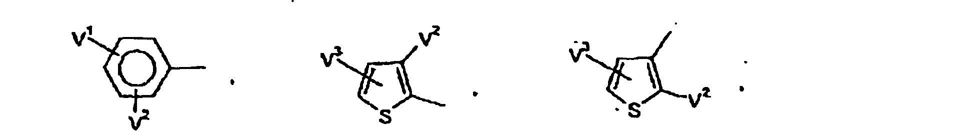 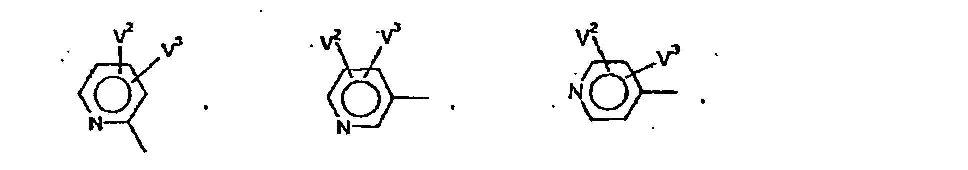 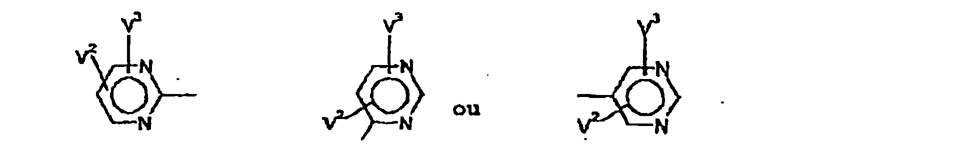
V¹ représente l'hydrogène, le fluor, le chlore, le brome, un reste alkyle en C₁ à C₆, alkoxy en C₁ à C₄, halogénalkyle en C₁ ou C₂, halogénalkoxy en C₁ ou C₂, nitro, cyano ou un reste phényle, phénoxy, phénoxy(alkyle en C₁ ou C₂), phényl- (alkoxy en C₁ ou C₂), phénylthio- (alkyle en C₁ ou C₂) ou phényl-(alkylthio en C₁ ou C₂), chacun éventuellement substitué une ou deux fois par un radical fluoro, chloro, bromo, alkyle en C₁ à C₄, alkoxy en C₁ à C₄, halogénalkyle en C₁ ou C₂, halogénalkoxy en C₁ ou C₂, nitro ou cyano,
V² et V³ représentent, indépendamment l'un de l'autre, l'hydrogène, le fluor, le chlore, le brome, un reste alkyle en C₁ à C₄, alkoxy en C₁ à C₄, halogénalkyle en C₁ ou C₂ ou halogénalkoxy en C₁ ou C₂,
Z représente l'hydrogène, le fluor, le chlore, le brome, un reste alkyle en C₁ à C₄, halogénalkyle en C₁ à C₄, alkoxy en C₁ à C₄ ou halogénalkoxy en C₁ à C₄,
CKE désigne l'un des groupes 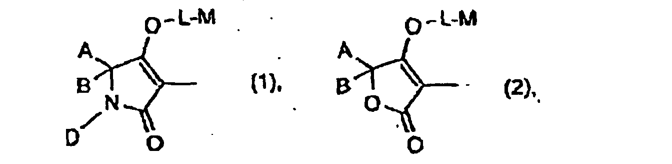 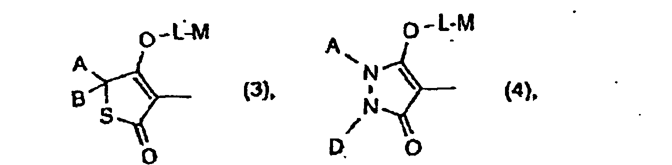 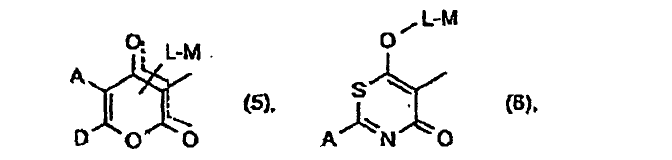 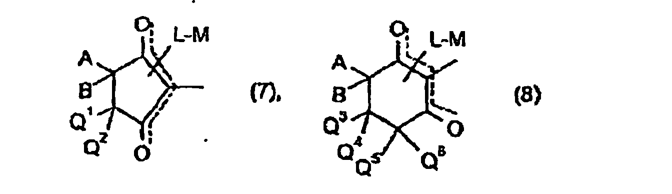
A représente l'hydrogène, un reste alkyle en C₁ à C₁₀, (alkoxy en C₁ à C₈) - (alkyle en C₁ à C₆), chacun éventuellement substitué par du fluor ou du chlore, un reste cycloalkyle en C₃ à C₇ éventuellement substitué par un radical fluoro, chloro, alkyle en C₁ à C₄ ou alkoxy en C₁ à C₄ et dans lequel, le cas échéant, un chaînon du noyau est remplacé par de l'oxygène ou du soufre, ou bien (toutefois non dans le cas des composés de formules (1-5), (I-7 ) et (I-8)), un reste phényle, furannyle, pyridyle, imidazolyle, triazolyle, pyrazolyle, pyrimidyle, thiazolyle, thiényle ou phényl-(alkyle en C₁ à C₄), chacun éventuellement substitué par un radical fluoro, chloro, bromo, alkyle en C₁ à C₄, halogénalkyle en C₁ à C₄, alkoxy en C₁ à C₄, cyano, nitro ou halogénalkoxy en C₁ à C₄,
B représente l'hydrogène ou un reste alkyle en C₁ à C₆, ou bien
A, B et l'atome de carbone auquel ils sont liés forment un reste cycloalkyle en C₅ à C₇ saturé ou non saturé, dans lequel le cas échéant un chaînon du noyau est remplacé par de l'oxygène ou du soufre et qui est éventuellement substitué une fois par un radical alkyle en C₁ à C₆, cycloalkyle en C₅ à C₈, halogénalkyle en C₁ à C₃, alkoxy en C₁ à C₆, fluoro, chloro ou phényle, ou bien
A, B et l'atome de carbone auquel ils sont liés forment un reste cycloalkyle en C₅ ou C₆ qui est substitué par un groupe alcènediyle contenant éventuellement un ou deux atomes d'oxygène ou de soufre non directement contigus ou par un groupe alkylènedioxyle ou par un groupe alkylènedithiol qui forme avec l'atome de carbone auquel il est lié un autre noyau pentagonal ou hexagonal, ou bien
A, B et l'atome de carbone auquel ils sont liés forment un reste cycloalkyle en C₃ à C₆ ou un reste cycloalcényle en C₅ ou C₆, dans lesquels deux substituants forment, conjointement avec les atomes de carbone auxquels ils sont liés, un reste alcanediyle en C₂ à C₄, un reste alcènediyle en C₂ à C₄, chacun éventuellement substitué par un radical alkyle en C₁ à C₅, alkoxy en C₁ à C₅, fluoro, chloro ou bromo, où le cas échéant, un groupe méthylène est remplacé par l'oxygène ou le soufre, ou bien un reste butanediyle,
D représente l'hydrogène, un reste alkyle en C₁ à C₁₀, alcényle en C₃ à C₆, (alkoxy en C₁ à C₈) - (alkyle en C₂ à C₆) ou (alkylthio en C₁ à C₈) - (alkyle en C₂ à C₆), chacun éventuellement substitué par du fluor ou du chlore, un reste cycloalkyle en C₃ à C₇ éventuellement substitué par un radical fluoro, chloro, alkyle en C₁ à C₄, alkoxy en C₁ à C₄ ou halogénalkyle en C₁ ou C₂ et dans lequel, le cas échéant, un groupe méthylène est remplacé par de l'oxygène ou du soufre, ou bien (toutefois non dans le cas des composés de formules (I-1) et (I-4)), un reste phényle, furannyle, imidazolyle, pyridyle, thiazolyle, pyrazolyle, pyrimidyle, pyrrolyle, thiényle, triazolyle ou phényl(alkyle en C₁ à C₄), chacun éventuellement substitué par un radical fluoro, chloro, bromo, alkyle en C₁ à C₄, halogénalkyle en C₁ à C₄, alkoxy en C₁ à C₄ ou halogénalkoxy en C₁ à C₄, ou bien
A et D forment ensemble un reste alcanediyle en C₃ à C₅ éventuellement substitué, dans lequel un groupe méthylène peut être remplacé par un groupe carbonyle, l'oxygène ou le soufre, en considérant comme substituants un radical hydroxy, alkyle en C₁ à C₆ ou alkoxy en C₁ à C₄, ou bien
A et D (dans le cas des composés de formule (I-1)) forment, conjointement avec les atomes auxquels ils sont liés, l'un des groupes AD-1 à AD-10 : 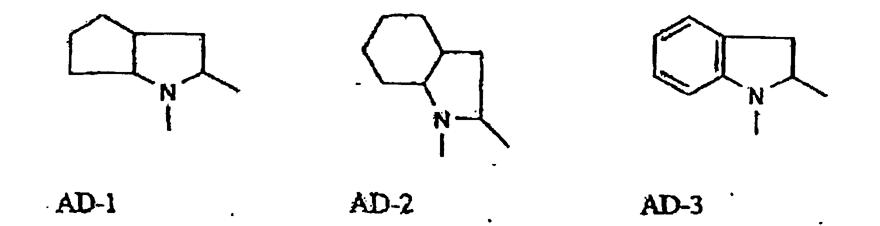 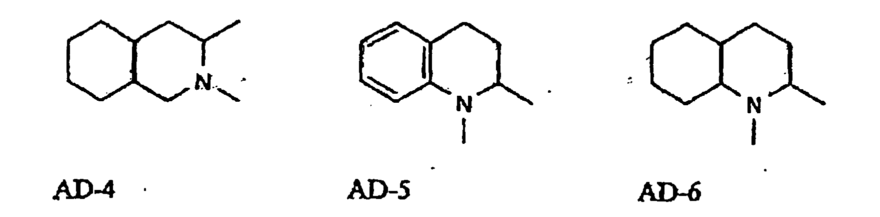 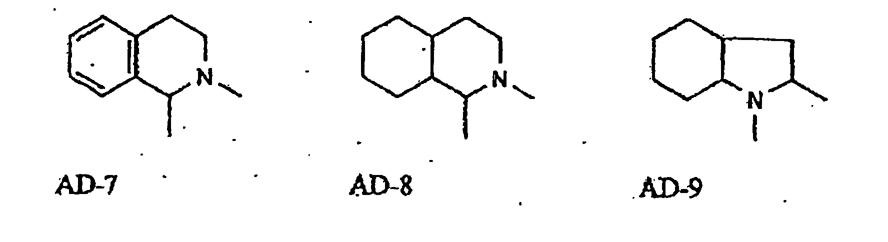 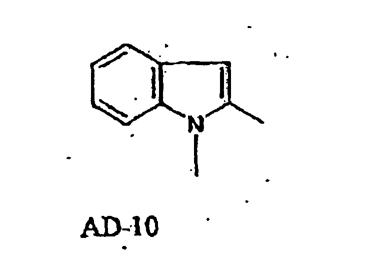 ou bien
A et Q¹ forment ensemble un reste alcanediyle en C₃ ou C₄ ou un reste alcènediyle en C₃ ou C₄, chacun éventuellement substitué une ou deux fois identiques ou différentes par un radical fluoro, chloro, hydroxy, un radical alkyle en C₁ à Ce ou alkoxy en C₁ à C₄, chacun éventuellement substitué une à trois fois par du fluor, ou bien
Q¹ représente l'hydrogène,
Q² représente l'hydrogène,
Q⁴, Q⁵ et Q⁶ représentent indépendamment les uns des autres l'hydrogène ou un reste alkyle en C₁ à C₃,
Q³ représente l'hydrogène, un reste alkyle en C₁ à C₄, (alkoxy en C₁ à C₄)-(alkyle en C₁ ou C₂), (alkylthio en C₁ à C₄)-(alkyle en C₁ ou C₂) ou un reste cycloalkyle en C₃ à C₆ éventuellement substitué par un radical méthyle ou méthoxy et dans lequel, le cas échéant, un groupe méthylène est remplacé par de l'oxygène ou du soufre, ou bien
Q³ et Q⁴ forment, conjointement avec le carbone auquel ils sont liés, un reste cycloalkyle en C₅ ou C₆ éventuellement substitué par un radical alkyle en C₁ à C₄ ou alkoxy en C₁ à C₄ et dans lequel, le cas échéant, un chaînon du noyau est remplacé par de l'oxygène ou du soufre,
L est un groupe alcanediyle ayant 1 à 4 atomes de carbone,
M est l'un des groupements suivants : CN; 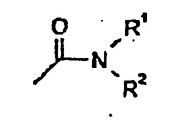 -CO₂R²; -OR², -SR², -COR³. 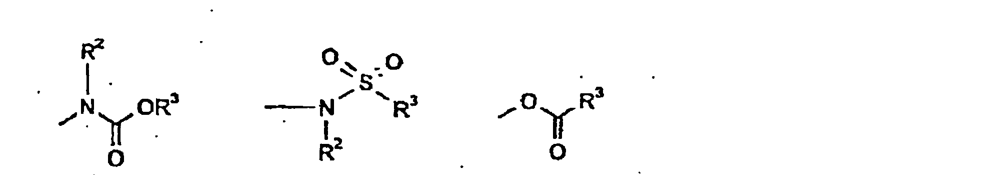 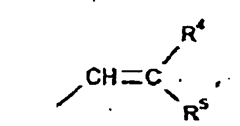 ―C≡C―R⁴ ou 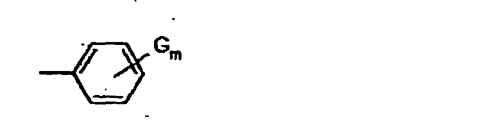
R¹ représente l'hydrogène ou un reste alkyle en C₁ à C₁₀,
R² représente un reste alkyle en C₁ à C₁₀, alcényle en C₃ à C₆, alcynyle en C₃ à C₆, cycloalkyle en C₃ à C₈, (alkoxy en C₁ à C₆) - (alkyle en C₂ à C₆), (alkylthio en C₁ à C₆) - (alkyle en C₂ à C₆), chacun éventuellement substitué par du fluor, du chlore ou du brome, un reste phényle ou un reste benzyle, chacun éventuellement substitué par un radical fluoro, chloro, bromo, nitro, cyano, alkylthio en C₁ à C₄, alkoxy en C₁ à C₄, halogénalkoxy en C₁ à C₄, halogénalkylthio en C₁ à C₄, alkyle en C₁ à C₄ ou halogénalkyle en C₁ à C₄,
R³ représente un reste alkyle en C₁ à C₁₀ éventuellement substitué par du fluor, du chlore ou du brome ou un reste phényle ou benzyle, chacun éventuellement substitué par du fluor, du chlore, un radical alkyle en C₁ à C₄, halogénalkyle en C₁ à C₄, alkoxy en C₁ à C₄ ou halogénalkoxy en C₁ à C₄,
R⁴ représente l'hydrogène, le fluor, le chlore, le brome, un reste alkyle en C₁ à C₅ éventuellement substitué par du fluor ou du chlore ou un reste phényle éventuellement substitué par un radical fluoro, chloro, bromo, alkyle en C₁ à C₄, alkoxy en C₁ à C₄, halogénalkyle en C₁ ou C₂, halogénalkoxy en C₁ ou C₂, cyano ou nitro,
R⁵ représente l'hydrogène, le fluor, le chlore, le brome ou un reste alkyle en C₁ à C₄ éventuellement substitué par du fluor ou du chlore,
G représente le fluor, le chlore, le brome, un reste alkyle en C₁ à C₄, alkoxy en C₁ à C₄, halogénalkyle en C₁ ou C₂, halogénalkoxy en C₁ ou C₂, cyano ou nitro,
m représente le nombre 0, 1 ou 2.

3. Composés de formule (I) suivant la revendication 1, formule dans laquelle
W représente l'hydrogène, le chlore, le fluor, un reste méthyle, éthyle, méthoxy, éthoxy,
X représente le fluor, le chlore, un reste méthyle, éthyle, n-propyle, isopropyle, méthoxy, éthoxy, n-propoxy, isopropoxy, trifluorométhyle, difluorométhoxy, trifluorométhoxy, nitro ou cyano,
Y représente l'un des restes 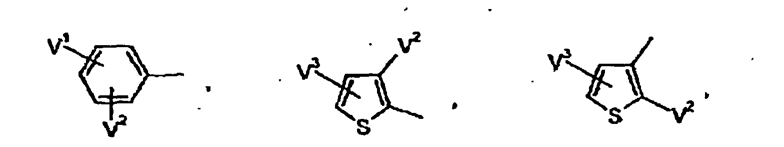 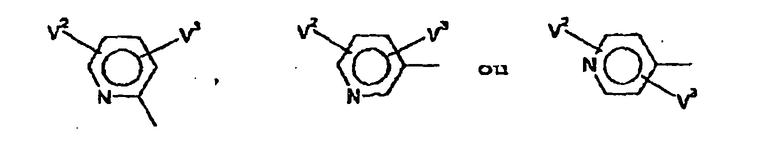
V¹ représente l'hydrogène, le fluor, le chlore, le brome, un reste méthyle, éthyle, n-propyle, isopropyle, n-butyle, isobutyle, tertiobutyle, méthoxy, éthoxy, n-propoxy, isopropoxy, trifluorométhyle, trifluorométhoxy, nitro, cyano ou phényle,
V² et V³ représentent indépendamment l'un de l'autre l'hydrogène, le fluor, le chlore, un reste méthyle, éthyle, n-propyle, isopropyle, méthoxy, éthoxy, trifluorométhyle ou trifluorométhoxy,
Z représente l'hydrogène, le fluor, le chlore, un reste méthyle ou méthoxy,
CKE représente avantageusement l'un des groupes 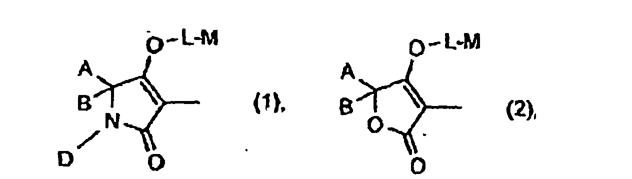 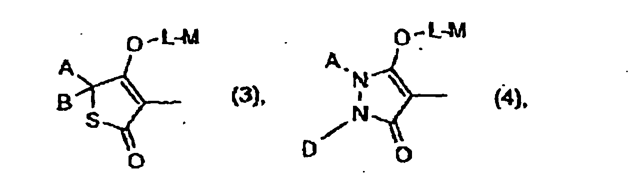 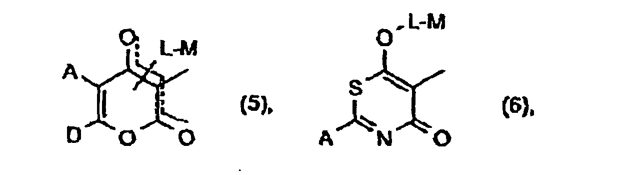 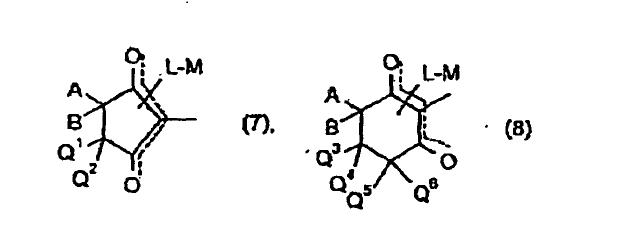
A représente l'hydrogène, un reste alkyle en C₁ à C₈ ou (alkoxy en C₁ à C₆) - (alkyle en C₁ à C₄), chacun éventuellement substitué par du fluor, un reste cycloalkyle en C₃ à C₆ éventuellement substitué par un radical fluoro, méthyle, éthyle ou méthoxy dans lequel, le cas échéant, un chaînon du noyau est remplacé par de l'oxygène ou du soufre ou bien (toutefois non dans le cas des composés des formules (I-5), (I-7) et (I-8)), un reste phényle ou benzyle, chacun éventuellement substitué par un radical fluoro, chloro, bromo, méthyle, éthyle, n-propyle, isopropyle, méthoxy, éthoxy, trifluorométhyle, trifluorométhoxy, cyano ou nitro,
B représente l'hydrogène ou un reste alkyle en C₁ à C₄, ou bien
A, B et l'atome de carbone auquel ils sont liés représentent un reste cycloalkyle en C₅ à C₆ saturé dans lequel, le cas échéant, un chaînon du noyau est remplacé par de l'oxygène ou du soufre et qui est éventuellement substitué par un radical méthyle, éthyle, n-propyle, isopropyle, butyle, isobutyle, sec.-butyle, tertiobutyle, trifluorométhyle, méthoxy, éthoxy, n-propoxy, isopropoxy, n-butoxy, isobutoxy, sec.-butoxy, tertiobutoxy, fluoro ou chloro,
A, B et l'atome de carbone auquel ils sont liés forment un reste cycloalkyle en C₅ ou C₆ dans lequel deux substituants forment, conjointement avec les atomes de carbone auxquels ils sont liés, un reste alcanediyle en C₂ à C₄ ou alcènediyle en C₂ à C₄ où, dans chaque cas, un groupe méthylène est éventuellement remplacé par de l'oxygène ou du soufre, ou bien un reste butadiènediyle,
D représente l'hydrogène, un reste alkyle en C₁ à Ce, alcényle en C₃ ou C₄, (alkoxy en C₁ à C₆)-(alkyle en C₂ à C₄), (alkylthio en C₁ à C₄)-(alkyle en C₂ à C₄) ou cycloalkyle en C₃ à C₆, chacun éventuellement substitué par du fluor ou du chlore et dans lequel, le cas échéant, un groupe méthylène est remplacé par de l'oxygène ou du soufre, ou bien (toutefois non dans le cas des composés de formules (I-1) et (I-4)), un reste phényle, furannyle, pyridyle, thiényle ou benzyle, chacun éventuellement substitué par un radical fluoro, chloro, méthyle, éthyle, n-propyle, isopropyle, méthoxy, éthoxy, trifluorométhyle ou trifluorométhoxy,
ou bien
A et D forment ensemble un reste alcanediyle en C₃ ou C₄ éventuellement substitué, dans lequel, le cas échéant, un atome de carbone est remplacé par du soufre et qui est éventuellement substitué par un radical hydroxy, méthyle, éthyle, méthoxy ou éthoxy, ou bien
A et D (dans le cas des composés de formule (I-1)) forment, conjointement avec les atomes auxquels ils sont liés, l'un des groupes suivants : 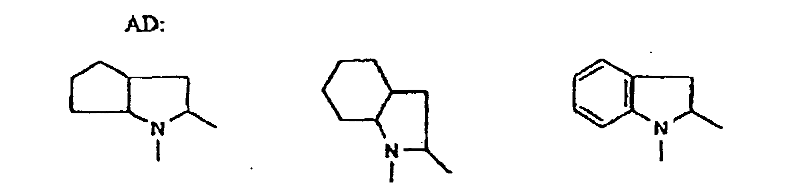 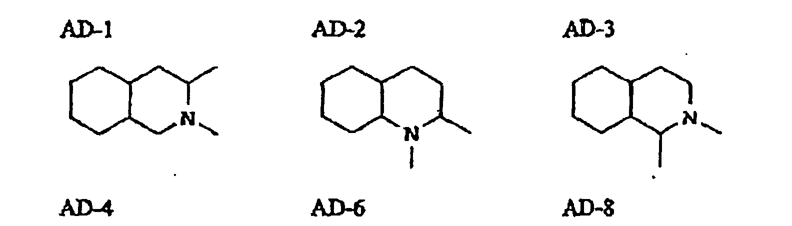 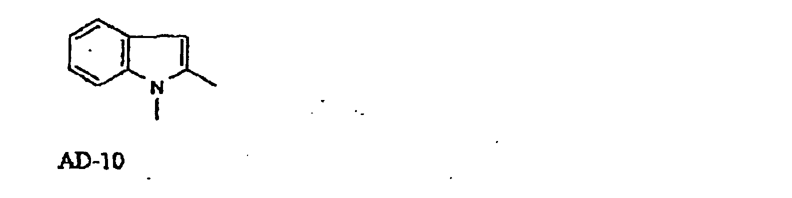
A et Q¹ forment ensemble un reste alcanediyle en C₃ ou C₄ éventuellement substitué une ou deux fois par un radical fluoro, hydroxy, méthyle ou méthoxy, ou un reste butènediyle, ou bien
Q¹ représente l'hydrogène,
Q² représente l'hydrogène,
Q⁴, Q⁵ et Q⁶ représentent indépendamment les uns des autres l'hydrogène, un reste méthyle ou éthyle,
Q³ représente l'hydrogène, un reste méthyle, éthyle ou cycloalkyle en C₃ à C₆ dans lequel, le cas échéant, un groupe méthylène est remplacé par de l'oxygène ou du soufre, ou bien
Q³ et Q⁴ forment, conjointement avec le carbone auquel ils sont liés, un reste cycloalkyle en C₅ ou C₆ saturé éventuellement substitué par un radical méthyle ou méthoxy et dans lequel, le cas échéant, un chaînon du noyau est remplacé par de l'oxygène ou du soufre,
L représente l'un des groupements suivants -CH₂-, 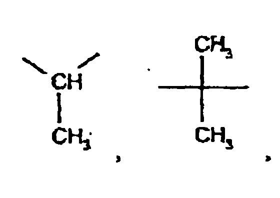 -CH₂-CH₂, 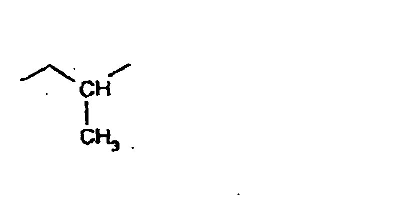
M représente l'un des groupements suivants CN; 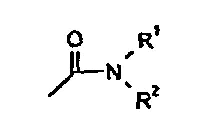 -CO₂R², -OR², -SR², -COR³, 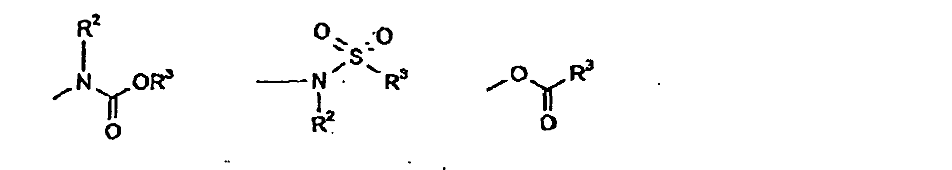 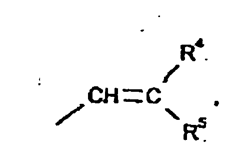 ―C≡C―R⁴ ou 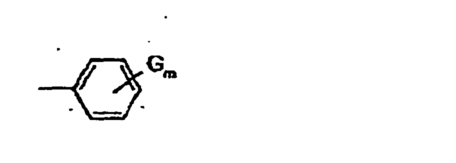
R¹ représente l'hydrogène ou un reste alkyle en C₁ à C₈,
R² représente un reste alkyle en C₁ à C₈, alcényle en C₃ ou C₄, alcynyle en C₃ ou C₄, cyclopropyle, cyclopentyle, cyclohexyle, (alkoxy en C₁ à C₆)-(alkyle en C₂ à C₄), (alkylthio en C₁ à C₆) - (alkyle en C₂ à C₄), chacun éventuellement substitué par du fluor, du chlore ou du brome, un reste phényle ou benzyle, chacun éventuellement substitué par un radical fluoro, chloro, bromo, nitro, cyano, méthylthio, éthylthio, méthoxy, éthoxy, trifluorométhylthio, trifluorométhoxy, méthyle, éthyle, trifluorométhyle,
R³ représente un reste alkyle en C₁ à C₈ éventuellement substitué par du fluor, du chlore, du brome, ou un reste phényle substitué par un radical fluoro, chloro, alkyle en C₁ ou C₂, trifluorométhyle, alkoxy en C₁ ou C₂ ou trifluorométhoxy, ou un reste benzyle,
R⁴ représente l'hydrogène, le fluor, le chlore, un reste alkyle en C₁ à C₄ éventuellement substitué par du fluor ou du chlore ou un reste phényle éventuellement substitué par un radical fluoro, chloro, méthyle, éthyle, méthoxy, éthoxy, trifluorméthyle, difluorométhoxy, trifluorométhoxy, cyano ou nitro,
R⁵ représente l'hydrogène, le fluor, le chlore ou un reste méthyle éventuellement substitué par du fluor ou du chlore, un reste éthyle, propyle ou isopropyle,
G représente le fluor, le chlore, un reste méthyle, éthyle, propyle, isopropyle, méthoxy, éthoxy, propoxy, isopropoxy, trifluorométhyle, difluorométhoxy, trifluorométhoxy, cyano ou nitro,
m représente un nombre de 0 à 2.

4. Composés de formule (I) suivant la revendication 1, formule dans laquelle
W représente l'hydrogène, le chlore, le fluor, un reste méthyle, éthyle, méthoxy, éthoxy,
X représente le fluor, le chlore, un reste méthyle, éthyle, n-propyle, isopropyle, méthoxy, éthoxy, n-propoxy, isopropoxy, trifluorométhyle, difluorométhoxy, trifluorométhoxy, nitro ou cyano,
Y représente l'un des restes 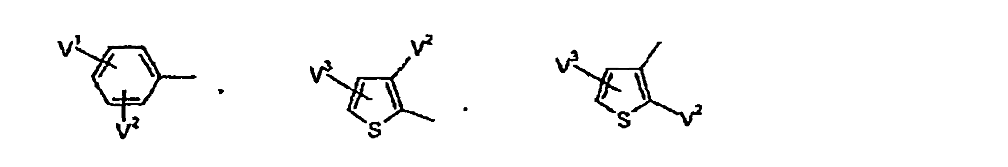
V¹ représente l'hydrogène, le fluor, le chlore, un reste méthyle, éthyle, n-propyle, isopropyle, n-butyle, isobutyle, tertiobutyle, méthoxy, éthoxy, n-propoxy, isopropoxy, trifluorométhyle, trifluorométhoxy, nitro, cyano ou phényle,
V² représente l'hydrogène, le fluor, le chlore, un reste méthyle, méthoxy, trifluorométhyle ou trifluorométhoxy,
V³ représente l'hydrogène, un reste méthyle ou le chlore,
Z représente l'hydrogène, le fluor, le chlore, un reste méthyle ou méthoxy,
CKE représente l'un des groupes 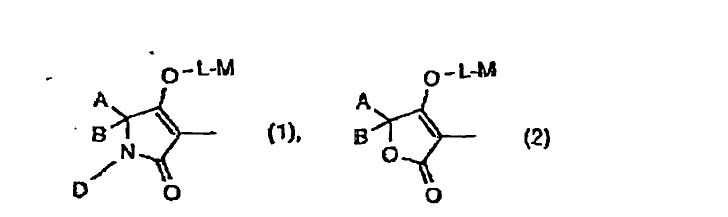 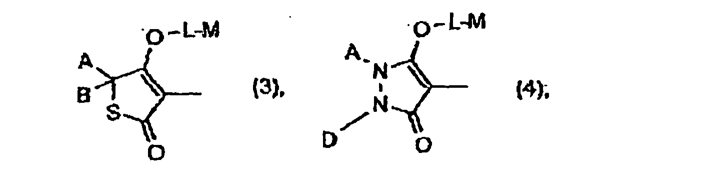 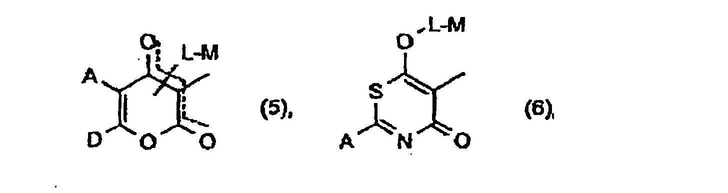 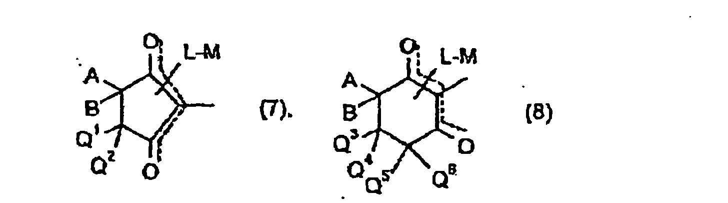
A représente l'hydrogène, un reste alkyle en C₁ à C₈ ou (alkoxy en C₁ à C₆) - (alkyle en C₁ à C₄), chacun éventuellement substitué par du fluor, un reste cycloalkyle en C₃ à C₆ éventuellement substitué par un radical fluoro, méthyle, ou méthoxy dans lequel, le cas échéant, un chaînon du noyau est remplacé par de l'oxygène ou du soufre ou bien (toutefois non dans le cas des composés des formules (1-5), (I-7) et (I-8)), un reste phényle ou benzyle, chacun éventuellement substitué par un radical fluoro, chloro, bromo, méthyle, méthoxy, trifluorométhyle ou trifluorométhoxy,
B représente l'hydrogène ou un reste alkyle en C₁ à C₄, ou bien
A, B et l'atome de carbone auquel ils sont liés représentent un reste cycloalkyle saturé en C₅ à C₆ dans lequel, le cas échéant, un chaînon du noyau est remplacé par de l'oxygène ou du soufre et qui est éventuellement substitué une fois par un radical méthyle, éthyle, n-propyle, isopropyle, trifluorométhyle, méthoxy, éthoxy, n-propoxy, isopropoxy, n-butoxy ou isobutoxy, ou bien
A, B et l'atome de carbone auquel ils sont liés forment un reste cycloalkyle en C₅ ou C₆ dans lequel deux substituants forment, conjointement avec les atomes de carbone auxquels ils sont liés, un reste alcanediyle en C₂ à C₄ ou un reste alcènediyle en C₂ à C₄, dans chacun desquels, le cas échéant, un groupe méthylène est remplacé par de l'oxygène ou du soufre, ou bien un reste butadiènediyle,
D représente l'hydrogène, un reste, chacun éventuellement substitué par du fluor ou du chlore, alkyle en C₁ à C₆, alcényle en C₃ ou C₄, (alkoxy en C₁ à C₄) - (alkyle en C₂ à C₄), (alkylthio en C₁ à C₄)-(alkyle en C₂ à C₄) ou cycloalkyle en C₃ à C₆ dans lequel, le cas échéant, un groupe méthylène est remplacé par de l'oxygène ou du soufre, ou bien (toutefois non dans le cas des composés de formules (I-1) et (I-4)), un reste phényle, pyridyle ou benzyle, chacun éventuellement substitué par un radical fluoro, chloro, méthyle, méthoxy, trifluorométhyle ou trifluorométhoxy,
ou bien
A et D forment ensemble un reste alcanediyle en C₃ ou C₄ éventuellement substitué dans lequel, le cas échéant, un atome de carbone est remplacé par du soufre et qui est éventuellement substitué par un radical méthyle, ou méthoxy,
A et Q¹ forment ensemble un reste alcanediyle en C₃ ou C₆ éventuellement substitué une ou deux fois par un radical méthyle ou méthoxy, ou un reste butènediyle, ou bien
Q¹ représente l'hydrogène,
Q² représente l'hydrogène,
Q⁴, Q⁵ et Q⁶ représentent indépendamment les uns des autres l'hydrogène, un reste méthyle ou éthyle,
Q³ représente l'hydrogène, un reste méthyle, éthyle ou cycloalkyle en C₃ à C₆ dans lequel, le cas échéant, un groupe méthylène est remplacé par de l'oxygène ou du soufre,
Q³ et Q⁴ forment, conjointement avec le carbone auquel ils sont liés, un reste cycloalkyle en C₅ ou C₆ éventuellement substitué par un radical méthyle ou méthoxy et dans lequel, le cas échéant, un chaînon du noyau est remplacé par de l'oxygène ou du soufre,
L représente l'un des groupements suivants 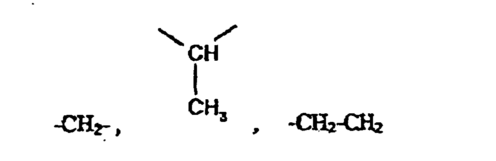
M représente l'un des groupements suivants CN; 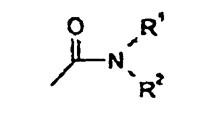 -CO₂R², -OR², -SR², -COR³. 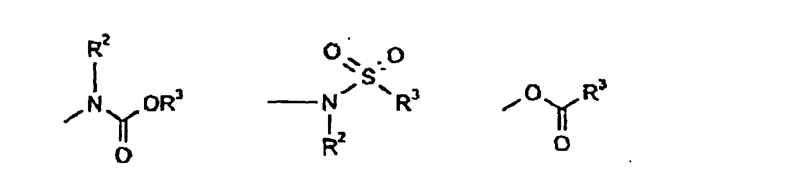 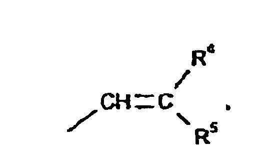 ―C≡C―R⁴ ou 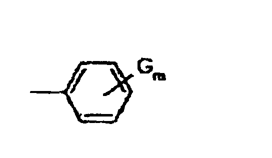
R¹ représente l'hydrogène ou un reste alkyle en C₁ à C₄,
R² représente un reste alkyle en C₂ à C₄, alcényle en C₃ ou C₄, alcynyle en C₃ ou C₄, cyclopropyle, cyclopentyle, cyclohexyle, (alkoxy en C₁ à C₄)-(alkyle en C₂ à C₄), chacun éventuellement substitué par du fluor, un reste phényle ou benzyle, chacun éventuellement substitué par un radical fluoro, chloro, bromo, nitro, cyano, méthylthio, éthylthio, méthoxy, éthoxy, trifluorométhylthio, trifluorométhoxy, méthyle, éthyle, trifluorométhyle,
R³ représente un reste alkyle en C₁ à C₄ éventuellement substitué par du fluor ou un reste phényle substitué par un radical fluoro, chloro, méthyle, trifluorométhyle, méthoxy ou trifluorométhoxy,
R⁴ représente l'hydrogène, le fluor, le chlore, un reste alkyle en C₁ à C₄ éventuellement substitué par du fluor, ou un reste phényle éventuellement substitué par un radical fluoro, chloro, méthyle, éthyle, méthoxy, éthoxy, trifluorométhyle, difluorométhoxy, trifluorométhoxy, cyano ou nitro,
R⁵ représente l'hydrogène, le fluor, le chlore ou un reste méthyle éventuellement substitué par du fluor,
G représente le fluor, le chlore, un reste méthyle, méthoxy, trifluorométhyle, difluorométhoxy, trifluorométhoxy, cyano ou nitro,
m représente un nombre de 0 à 1.

5. Procédé de production de composés de formule (I-1) à (1-8) suivant la revendication 1, 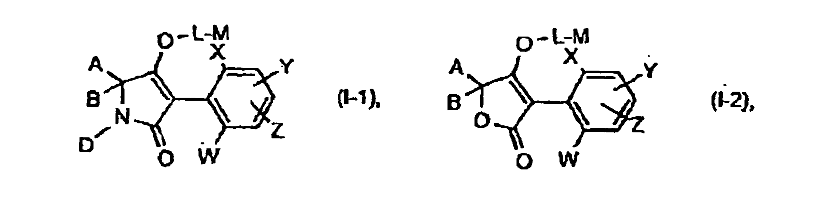 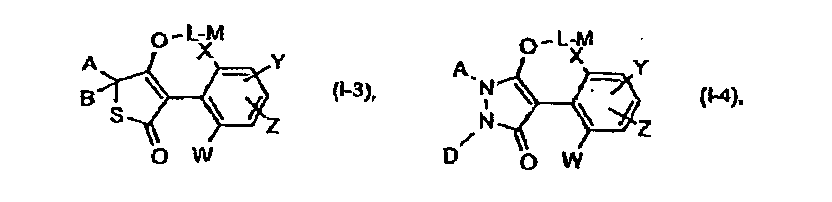 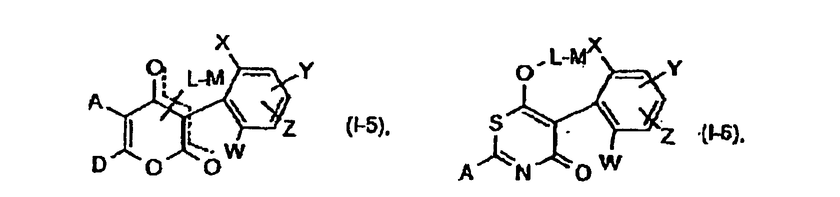 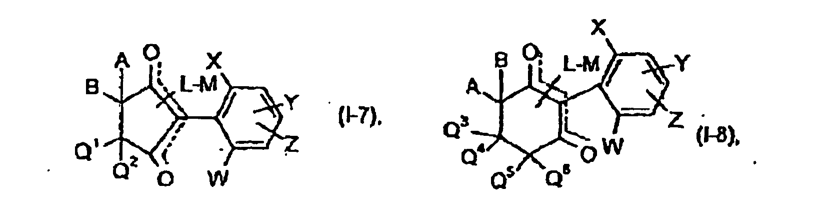 où
A, B, D, L, M, Q¹, Q², Q³, Q⁴, Q⁵, Q⁶, W, X, Y et Z ont les définitions indiquées ci-dessus,
**caractérisé en ce qu'**on fait réagir des composés de formule (II-1) à (II-8) 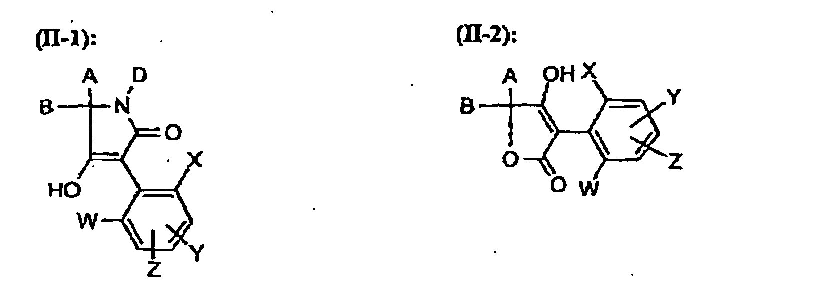 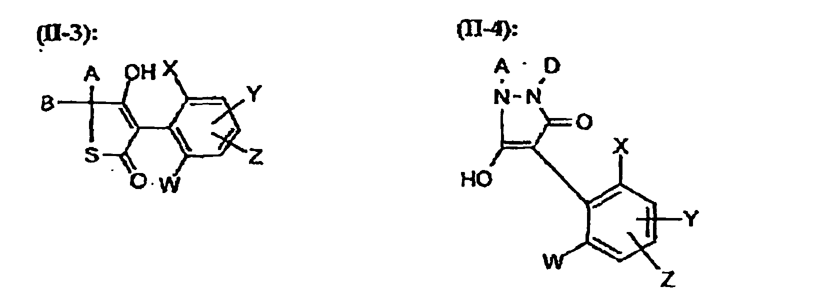 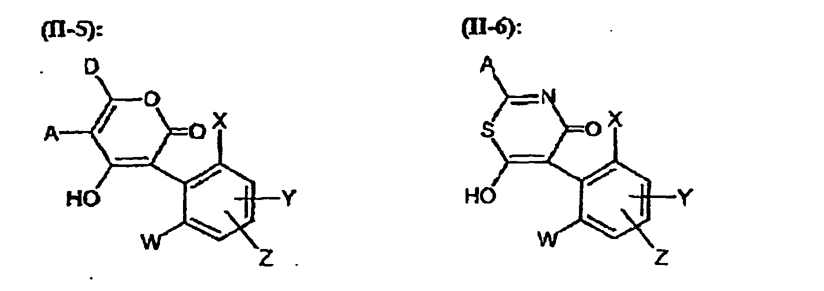 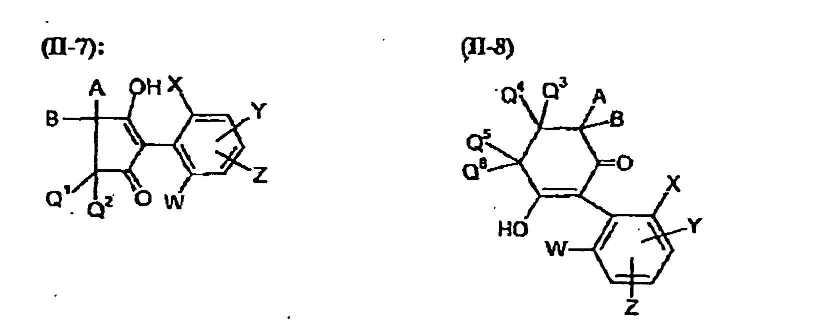 où
A, B, D, Q¹, Q², Q³, Q⁴, Q⁵, Q⁶, W, X, Y et Z ont la définition indiquée ci-dessus,
avec des composés de formulé (III)
J-L-M (III)
dans laquelle
L et M ont la définition indiquée ci-dessus, et
J représente un groupe partant tel qu'halogéno, -O-SO₂-halogénalkyle, -O-SO₂-alkyle ou -O-SO₂-aryle,
en présence d'un diluant et en présence d'une base.

6. Composition pesticide et/ou composition herbicide, **caractérisées par** une teneur en au moins un composé de formule (I) suivant la revendication 1.

7. Utilisation de composés de formule (I) suivant la revendication 1, pour la préparation de compositions pesticides destinées à combattre des parasites et des mauvaises herbes.

8. Procédé de préparation de compositions pesticides destinées à combattre des parasites et des mauvaises herbes, **caractérisé en ce qu'**on fait agir des composés de formule (I) suivant la revendication 1 sur les parasites, les plantes et/ou leur milieu.

9. Procédé de production de compositions pesticides et/ou de compositions herbicides, **caractérisé en ce qu'**on mélange des composés de formule (I) suivant la revendication 1 avec des diluants et/ou des agents tensioactifs.

10. Utilisation de composés de formule (I) suivant la revendication 1, pour la préparation de compositions pesticides et d'herbicides.
